# EUROPEAN PATENT SPECIFICATION

(11) **EP 4 291 157 B1**
(45) Date of publication and mention of the grant of the patent: **19.03.2025**
(21) Application number: 22703680.3
(22) Date of filing: 09.02.2022
(51) Int. Cl.: A61K 8/49, A61K 8/60, A61Q 13/00, C11D 3/50

(54) **FRAGRANCE DELIVERY COMPOSITIONS**
DUFTSTOFFABGABEZUSAMMENSETZUNGEN
COMPOSITIONS POUR DIFFUSION DE PARFUM

(30) Priority: 15.02.2021 EP 21157146
(43) Date of publication of application: 20.12.2023
(73) Proprietor: Taminco BV, 9000 Gent (BE)
(72) Inventor: APHALO, Federico, 9040 Gent (BE); VAN DER BURGH, Stefan, 4007nb Tiel (NL)
(74) Representative: Gevers Patents
(86) International application number: PCT/EP2022/053180
(87) International publication number: WO 2022/171707

(56) References cited:
- WO-A1-2020/058193
- JP-A- H0 261 174
- JP-A- H1 143 863

## Description

### FIELD OF THE INVENTION

The present invention relates to fragrance delivery compositions suitable for use in personal care and home care formulations, said fragrance delivery compositions possessing an enhanced fragrance longevity and thereby providing a prolonged fragrance release over an extended period of time.

### BACKGROUND OF THE INVENTION

Fragrance delivery compositions are ubiquitous, especially with reference to their use in personal care and home care formulations wherein fragrances provide an overall pleasing odor to these formulations. However, as a consequence of rapid evaporation of fragrances in personal care and home care formulations in general, their fragrance profile, in terms of fragrance character and fragrance intensity, as perceived by the user and others in the user's direct environment, decreases rapidly with time. Consequently, in the fragrance industry, there is a constant need to find new technologies for prolonging the perception of fragrances as a function of time. Such a need is particularly pertinent when dealing with fragrance formulations rich in highly volatile components which tend to evaporate rapidly, as for example in case of perfumes and deodorants. Additionally, the perceived extent of this decrease is further enhanced by the rapid evaporation of the organic solvent present in many consumer products, such as high amounts of ethanol in personal care products (e.g. colognes, body splashes, etc.). It is thus desirable to have a fragrance delivery composition which retains a significant portion of its initial fragrance character over time, thereby resulting in said composition having improved longevity of the fragrance character.

As a direct consequence of the foregoing, maintaining fragrance intensity over time is key for a commercial fragrance consumer product, and therefore this has been a widely investigated field of research in fragrance design for the past years and more.

In the prior art, many approaches and various materials are described to solve the above-mentioned problem, in essence to make the fragrance profile last longer. Generally, these strategies center around the use of carrier and/or encapsulation materials to improve deposition of the fragrance delivery composition, around the proper selection of specific fragrance components, in essence fragrance components having a low(er) volatility, or around controlling the rate of release of the fragrance components, for example by using nonodorous fragrance fixatives or fragrance modulators.

Fragrance fixatives impart an increased delay of release to the overall fragrance and slow down and match the evaporation of the individual fragrance components such that the overall fragrance is released over an extended period of time while the fragrance ideally remains practically the same. The effect of most of the known fragrance fixatives is based on a reduced volatility of the fragrance components by the reduction in vapor pressure of said fragrance components, for example by dipole formation, hydrogen bridge bonding, adsorption effects or the formation of azeotropic mixtures. The use of nonodorous fragrance fixatives is known and described in the prior art. Fragrance fixatives may be used on the one hand in terms of fragrance efficiency, in essence the fragrance fixative replaces part of the fragrance components in a fragrance formulation to maintain the overall fragrance performance of said fragrance formulation, or on the other hand in terms of fragrance longevity, in essence the fragrance fixative is added on top of the fragrance formulation while increasing the fragrance longevity of said fragrance formulation. Certain fragrance fixatives have been proposed in the art for extending the fragrance longevity or retention of specific fragrance components in fragrance delivery compositions. In this view, every improvement in fragrance retention is considered to be highly desirable in as much as a personal care or a home care formulation's pleasing scent plays an important role in influencing consumers' acceptance and overall (commercial) preferences.

For instance, WO 2020/058193 A1 describes the use of certain carbohydrate esters, e.g. sucrose carbohydrate esters, as fragrance fixatives in aqueous fabric softener compositions for achieving improved fragrance longevity, in particular having a prolonged fragrance release from dry laundered fabric over an extended period of time.

Furthermore, JP H02 61174 A and JP H11 43863 A respectively disclose a softening agent for clothes and a liquid fabric softener composition for household washing, wherein both the softening agent and the liquid fabric softener composition comprise sucrose octaacetate as a sucrose carbohydrate ester in the presence of various fragrance components or perfume ingredients.

It is known in the art that sucrose acetate isobutyrate is a weighting agent in beverages. Beverage weighting agents are added to flavor oils to increase stability of the emulsion. Flavor oils have a low density, and the high density weighting agents increase the overall oil phase density, near the aqueous phase density, to inhibit gravitational separation. Moreover, it is described that terpenoid molecules, such as for instance citronellal and citral, demonstrated a decreased flavor component volatility when effectively combined with sucrose acetate isobutyrate (Masters of Science Thesis by Kelsey M. Kanyuck, North Carolina State University, abstract of the respective Thesis, page 5 8; Tables 3.2. and 3.3. on pages 75 - 76).

In addition, lactone-containing fragrance components or perfume ingredients are known to possess fragrance fixative properties themselves. For instance, γ-decalactone and coumarin, coumarin being one of the most important and widely used aroma chemicals having a hay like bittersweet odour, have been recognised in literature for their fragrance fixatives properties. However, the vast majority of these fragrance components are considerably expensive thereby inevitably resulting in highly priced personal and home care formulations, and often these fragrance components themselves, when produced synthetically, require a multi-step synthesis starting from commercially available substrates. Additionally, according to studies performed by the Health & Consumer Protection Directorate-General of the European Commission and many others, coumarin has frequently been shown to be able to elicit skin allergenic contact reactions in consumers. Hence, the International Fragrance Association (IFRA) guidelines give concentration limits based on induction experiments leading to a restricted use of coumarin in personal and home care formulations such as for example deodorants, hand creams, and perfumes.

In view of all the above, there remains a continuous and a further need for improved fragrance delivery compositions that are more cost efficient, more environmentally friendly, and less harmful, while maintaining fragrance longevity of said fragrance delivery compositions, in particular said fragrance delivery compositions having a maintained prolonged fragrance release over an extended period of time.

### SUMMARY OF THE INVENTION

The inventors have now surprisingly found that it is possible to provide an improved fragrance delivery composition fulfilling the above-mentioned needs.

Thus, there is now provided a fragrance delivery composition [composition (F), herein after] comprising, relative to the total weight of the composition (F):
a) from 10.00 to 70.00 weight percentage [wt. %, herein after] of at least one fragrance fixative [fixative (Fix), herein after], wherein said fixative (Fix) has a general Formula (I_{F}) 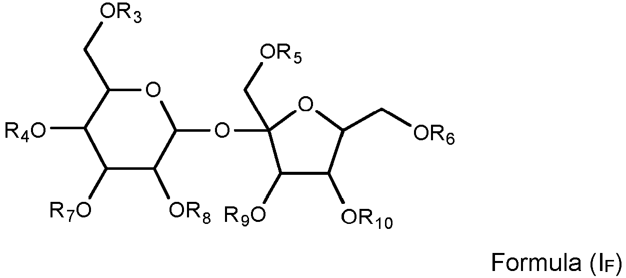 wherein
   - each of R₃, R₄, R₅, R₆, R₇, R₈, R₉, and R₁₀, equal to or different from each other and at each occurrence, are COR₁, wherein each of R₁ is independently selected from methyl, or isopropyl;
   with the proviso that at least one R₁ is isopropyl
b) from 2.50 to 50.00 wt. % of at least one fragrance co-fixative [co-fixative (c-F), herein after], wherein said co-fixative (c-F) is a co-fixative chosen among those of Formula (II_{A}) [co-fixative (c-F) of class (I), herein after], Formula (II_{B}) [co-fixative (c-F) of class (II), herein after], Formula (IIc) [co-fixative (c-F) of class (III), herein after], Formula (II_{D}) [co-fixative (c-F) of class (IV), herein after], Formula (II_{E}) [co-fixative (c-F) of class (V), herein after], Formula (II_{F}) [co-fixative (c-F) of class (VI), herein after], Formula (II_{G}) [co-fixative (c-F) of class (VII), herein after], or Formula (II_{H}) [co-fixative (c-F) of class (VIII), herein after] 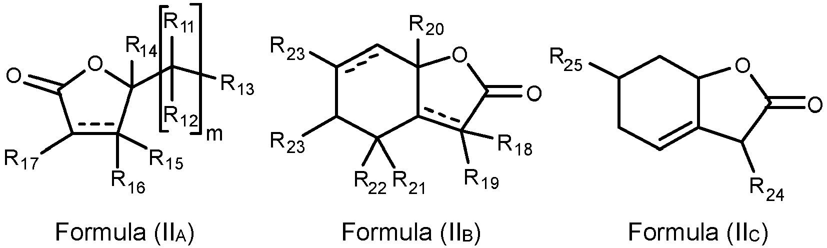 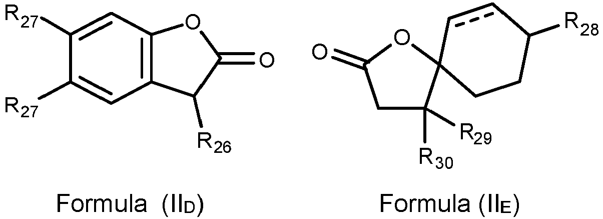 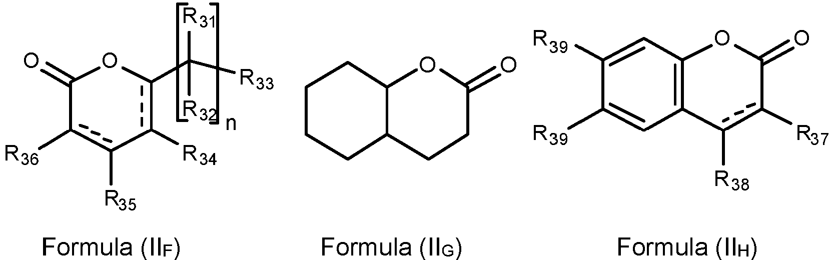 wherein the dash bond represents an optional double bound; provided that when there is a double bond in Formula (II_{A}), R₁₆ is not present; provided that when there is a double bond in the fused 5-membered lactone ring in Formula (II_{B}), R₁₉ is not present;
   and wherein
   - each of m, and n is an integer selected from 0, 1, 2, 3, 4, 5, 6, 7, 8, 9, 10, 11, or 12;
   - each of R₁₁, R₁₂, R₃₁, and R₃₂, equal to or different from each other and at each occurrence, is independently selected from hydrogen, C₁₋₈ alkyl, C₂₋₈ alkenyl, or OH; wherein R₁₁ and R₁₂ can together form =O;
   - each of R₁₃, R₁₅, R₁₆, R₁₈, R₁₉, R₂₀, R₂₁, R₂₂, R₂₄, R₂₆, R₂₇, R₂₉, R₃₀, R₃₃, R₃₅, R₃₆, R₃₇, R₃₈, and R₃₉, equal to or different from each other and at each occurrence, is independently selected from hydrogen, or methyl; wherein R₁₈ and R₁₉ can together form =CH₂ provided that there is no double bond in the fused 5-membered lactone ring of Formula (II_{B}); wherein R₃₇ and R₃₈ can together form a C₃ cycloalkyl provided that there is no double bond in the fused 6-membered lactone ring of Formula (II_{H});
   - each of R₁₄, and R₃₄, equal to or different from each other and at each occurrence, is independently selected from hydrogen, C₁₋₈ alkyl, or C₂₋₈ alkenyl;
   - each of R₁₇ is independently selected from hydrogen, methyl, or OH;
   - each of R₂₃, and R₂₅, equal to or different from each other and at each occurrence, is independently selected from hydrogen, C₁₋₄ alkyl, or C₂₋₄ alkenyl;
   - each of R₂₈ is independently selected from hydrogen, or C₁₋₄ alkyl;
c) at least 1.00 wt. % of at least one fragrance component, wherein said fragrance component is different to the co-fixative (c-F).

In another aspect, the present invention further provides a method for the manufacturing of the composition (F), as detailed above.

In another aspect, the present invention further provides a diluted composition (F) [composition (F_{D}), herein after] comprising the composition (F), as detailed above, and a diluent [diluent (dil), herein after].

In another aspect, the present invention further provides personal and home care formulations comprising the composition (F), as detailed above, or the composition (F_{D}).

In another aspect, the present invention further provides the use of the composition (F), as detailed above, or the composition (F_{D}), for the manufacturing of personal and home care formulations.

### DETAILED DESCRIPTION

### Composition (F)

Within the context of the present invention, the term "comprising" should not be interpreted as being restricted to the means listed thereafter; it does not exclude other elements or steps. It needs to be interpreted as specifying the presence of the stated features, integers, steps or components as referred to, but does not preclude the presence or addition of one or more other features, integers, steps or components, or groups thereof. Thus, the scope of the expression "a composition comprising components A and B" should not be limited to compositions consisting only of components A and B. It means that with respect to the present invention, the only relevant components of the composition are A and B. Accordingly, the terms "comprising" and "including" encompass the more restrictive terms "consisting essentially of" and "consisting of".

As used herein, the terms "optional" or "optionally" means that a subsequently described event or circumstance can or cannot occur, and that the description includes instances where said event or circumstance occurs and instances where it does not.

The inventors have surprisingly found that by using at least one fragrance fixative [fixative (Fix), herein after], as detailed above, in combination with at least one fragrance co-fixative [co-fixative (c-F), herein after], as detailed above, and at least one fragrance component, wherein said fragrance component is different to the co-fixative (c-F), as detailed above, the co-fixative (c-F) can be partially replaced in the fragrance delivery composition [composition (F), herein after] by the fixative (Fix), thereby successfully resulting in an improved composition (F) that is more cost efficient, more environmentally friendly, and less harmful, while maintaining fragrance longevity of said composition (F) and thus keeping its overall performance, in particular the at least one fragrance component is released over an extended period of time. Moreover, the inventors have thus found that said composition (F) has a maintained prolonged fragrance release of a range of fragrance components over an extended period of time, as demonstrated in the working examples.

Within the context of the present invention, the expressions "at least one fragrance fixative [fixative (Fix), herein after]", "at least one fragrance co-fixative [co-fixative (c-F), herein after]", and "at least one fragrance component" are intended to denote one or more than one fixative (Fix), one or more than one co-fixative (c-F), and one or more than one fragrance component, respectively. Mixtures of fixatives (Fix), mixtures of co-fixatives (c-F), and mixtures of fragrance components can also be used for the purpose of the invention, respectively.

In the rest of the text, the expressions "fixative (Fix)", "co-fixative (c-F)", and "fragrance component" are understood, for the purposes of the present invention, both in the plural and the singular form, that is to say the composition (F) of the present invention may comprise one or more than one fixative (Fix), one or more than one co-fixative (c-F), and one or more than one fragrance component.

In the context of the present invention, the term "fixative (Fix)" is intended to denote substances with impart an increased delay of release to the overall fragrance and match the evaporation of the individual fragrance components such that the fragrance is released over an extended period of time while the fragrance profile remains the same.

Within the context of the present invention, the expression "from 10.00 to 70.00 weight percentage [wt. %, herein after] of at least one fragrance fixative [fixative (Fix), herein after]" refers either to the amount of fixative (Fix), when the composition (F) contains only one fixative (Fix), or to the sum of the amounts of fixative (Fix), when the composition (F) contains more than one fixative (Fix). This being said, it means that it is necessary that, when more than one fixative (Fix) is present, then it is the sum of the amounts of each of said fixative (Fix) that ranges from 10.00 to 70.00 wt. %, relative to the total weight of the composition (F).

As used herein the term "alkyl" and "alkenyl" have the broadest meaning generally understood in the art, and may include a moiety which is linear, branched, or a combination thereof.

The term "alkyl", alone or in combination, means a straight or branched alkane-derived radical, for example, C_{F-G} alkyl defines a straight or branched alkyl radical having from F to G carbon atoms, e.g. C₁₋₄ alkyl defines a straight or branched alkyl radical having from 1 to 4 carbon atoms such as for example methyl, ethyl, 1-propyl, 2-propyl (isopropyl), 1-butyl, 2-butyl, 2-methyl-2-propyl (tert-butyl), 2-methyl-1-propyl (isobutyl).

The term "alkenyl", alone or in combination, means a straight or branched hydrocarbon containing at least one carbon to carbon double bond, for example C_{H-I} alkenyl defines a straight or branched alkenyl radical having from H to I carbon atoms, e.g. C₂₋₄ alkenyl defines a straight or branched alkenyl radical having from 2 to 4 carbon atoms. Examples of C₂₋₄ alkenyl groups include ethenyl, propenyl, isopropenyl, butenyl, and the like.

The term "cycloalkyl", alone or in combination, means a cyclic alkane-derived radical, for example, C_{L-M} cycloalkyl defines a cyclic alkyl radical having from L to M carbon atoms, e.g. C₃₋₆ cycloalkyl defines a cyclic alkyl radical having from 3 to 6 carbon atoms such as for example cyclopropyl, cyclobutyl, cyclopentyl, cyclohexyl and the like.

According to the present invention, the composition (F) comprises a fixative (Fix), wherein said fixative (Fix) has a general Formula (I_{F}) 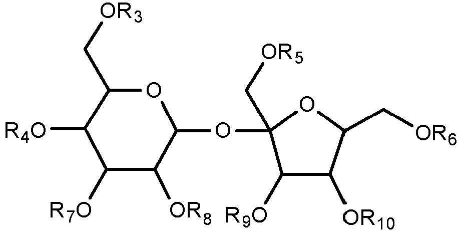 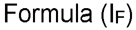 wherein
- each of R₃, R₄, R₅, R₆, R₇, R₈, R₉, and R₁₀, equal to or different from each other and at each occurrence, are COR₁, wherein each of R₁ is independently selected from methyl, or isopropyl;
with the proviso that at least one R₁ is isopropyl.

Without being bound to this theory, the inventors believe that a high number of ester groups may protect the glycosidic bond in the fixative (Fix) according to Formula (I_{F}) and may thereby provide a higher long-term stability of the respective fixative (Fix), as detailed above.

It is understood that the fixative (Fix), as detailed above, may comprise mixtures of fixatives (Fix) according to general Formula (I_{F}), as detailed above, which comprise different substituents. Specifically, the fully esterified fixatives (Fix) as mentioned in the embodiment right above, may be a mixture of fixatives (Fix) according to general Formula (I_{F}), as detailed above, which mixture for example comprises fixatives (Fix) having eight isobutyrate groups as well as fixatives (Fix) having two acetate groups and six isobutyrate groups and possibly other homologues having a different ratio of ester group substituents, respectively.

Fixatives (Fix) according to general Formula (I_{F}) of the present invention may be commercially available or may be chemically synthetized. In general, the alcoholic precursor of the fixative (Fix) can be esterified by methods known in the art. Said synthesis of suitable fixatives (Fix) according to general Formula (I_{F}), as detailed above, may be carried out using conventional methods known to the skilled in the art, such as notably described in U.S. Pat. No 3,096,324 and 6,977,275.

The fixative (Fix) according to general Formula (I_{F}), as detailed above, has several centers of chirality and may exist as stereochemically isomeric forms. The term "stereochemically isomeric forms" as used herein defines all the possible compounds made up of the same atoms bonded by the same sequence of bonds but having different three-dimensional structures. In this view, unless otherwise mentioned or indicated, each of the centers of chirality in the fixative (Fix) according to general Formula (I_{F}), as detailed above, may adopt an (*R*)- or (*S*)-configuration.

Unless otherwise mentioned or indicated, the chemical designation of the
fixative (Fix) according to Formula (I_{F}), as specified herein, encompasses the mixture of all possible stereochemically isomeric forms, which said compound may possess. Said mixture may contain all diastereomers and/or enantiomers of the basis molecular structure of said compound. All stereochemically isomeric forms of the compounds of the present invention both in pure form or mixed with each other are intended to be embraced within the scope of the present invention.

Pure stereoisomeric forms of the fixative (Fix) according to Formula (I_{F}), as specified herein, and intermediates as mentioned herein are defined as isomers substantially free of other enantiomeric or diastereomeric forms of the same basic molecular structure of said compounds. In particular, the term "stereoisomerically pure" concerns compounds or intermediates having a stereoisomeric excess of at least 80% (in essence minimum 90% of one isomer and maximum 10% of the other possible isomers) up to a stereoisomeric excess of 100% (i.e. 100% of one isomer and none of the other), more in particular, compounds or intermediates having a stereoisomeric excess of 90% up to 100%, even more in particular having a stereoisomeric excess of 94% up to 100% and most in particular having a stereoisomeric excess of 97% up to 100%. The terms "enantiomerically pure" and "diastereomerically pure" should be understood in a similar way, but then having regard to the enantiomeric excess, and the diastereomeric excess, respectively, of the mixture in question.

Pure stereoisomeric forms of the compounds and intermediates of this invention may be obtained by the application procedures known in the art. For instance, enantiomers may be separated by chromatographic techniques using chiral stationary phases. Said pure stereochemically isomeric forms may also be derived from the corresponding pure stereochemically isomeric forms of the appropriate starting materials, provided that the reaction occurs stereospecifically. Desirably, if a specific stereoisomer is desired, said compound will be synthesized by stereospecific methods of preparation. These methods will advantageously employ enantiomerically pure starting materials.

Preferred fixatives (Fix) according to general Formula (I_{F}), for use in the composition (F) according to the present invention, are those of Formula (I_{F1}) [fixative (Fix1), herein after] 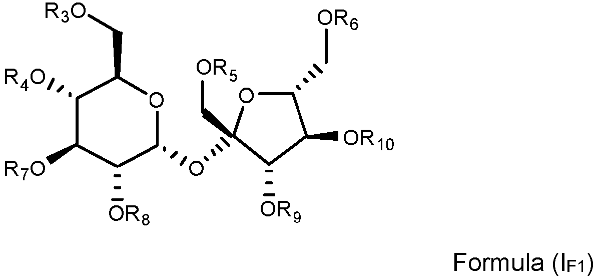 wherein
- each of R₃, R₄, R₅, R₆, R₇, R₈, R₉, and R₁₀, equal to or different from each other and at each occurrence, are COR₁, wherein each of R₁ is independently selected from methyl, or isopropyl;
with the proviso that at least one R₁ is isopropyl.

Non-limiting examples of suitable fixatives (Fix1) of Formula (I_{F1}) include sucrose octoisobutyrate, sucrose heptaisobutyrate acetate, sucrose hexaeisobutyrate diacetate, sucrose pentaisobutyrate triacetate, sucrose tetraisobutyrate tetraacetate, sucrose triisobutyrate pentaacetate.

A non-limitative example of a commercially available fixative (Fix1) of Formula (I_{F1}) suitable for use in the composition (F) according to the present invention is sucrose acetate isobutyrate, SAIB-100 available from Eastman Chemical Company which can be prepared using known techniques by reacting sucrose with acetic and isobutyric anhydrides followed by extensive purification using high vacuum distillation. The approximate ratio of acetate: isobutyrate ester groups in SAIB-100 is 2:6. SAIB-100 is odourless.

It is further understood that all definitions and preferences as described for fixative (Fix) according to Formula (I_{F}) above equally apply for this embodiment and all further embodiments, as described below.

As said above, the amount of the fixative (Fix) according to general Formula (I_{F}), as detailed above, is from 10.00 to 70.00 wt. %, relative to the total weight of the composition (F).

Advantageously, the amount of the fixative (Fix) according to general Formula (I_{F}), as detailed above, relative to the total weight of the composition (F), is equal to or greater than 12.00 wt. %, or equal to or greater than 14.00 wt. %, or equal to or greater than 16.00 wt. %, or equal to or greater than 18.00 wt. %, or equal to or greater than 20.00 wt. %.

It is further understood that the upper limit of the amount of the fixative (Fix) according to general Formula (I_{F}), as detailed above, relative to the total weight of the composition (F), is equal to or less than 65.00 wt. %, or equal to or less than 60.00 wt. %, or equal to or less than 55.00 wt. %, or equal to or less than 50.00 wt. %, or equal to or less than 45.00 wt. %, or equal to or less than 40.00 wt. %, or equal to or less than 35.00 wt. %, or equal to or less than 30.00 wt. %.

In a preferred embodiment of the composition (F) of the present invention, the fixative (Fix) according to general Formula (I_{F}), as detailed above, relative to the total weight of the composition (F), is present in an amount from 12.00 to 65.00 wt. %, or in an amount from 14.00 to 60.00 wt. %, or in an amount from 16.00 to 55.00 wt. %, or in an amount from 16.00 to 50.00 wt. %, or in an amount from 18.00 to 45.00 wt. %, or in an amount from 18.00 to 40.00 wt. %, or in an amount from 20.00 to 35.00 wt. %, or in an amount from 20.00 to 30.00 wt. %.

Within the context of the present invention, the term "co-fixative (c-F)" is intended to denote a substance that is added to the composition (F) in addition to the fixative (Fix), as detailed above, said substance thereby strengthening the fragrance fixative properties of said fixative (Fix), thereupon resulting in an improved fragrance longevity of a range of fragrance components so as to achieve an improved or prolonged perception of the fragrance profile by the consumer.

The co-fixatives (c-F), as detailed above, are also known to possess fragrance fixative properties in itself. However, the vast majority of these co-fixatives (c-F) are considerably expensive thereby inevitably resulting in highly priced personal and home care formulations, and often these fragrance components themselves, when produced synthetically, require a multi-step synthesis starting from commercially available substrates. Additionally, some of these co-fixatives (c-F) have even been shown to be able to elicit skin allergenic contact reactions in consumers. Therefore, in these cases their use in personal and home care formulations is restricted to certain concentration limits.

The inventors have now surprisingly found that those lactone compounds which are of Formula (II_{A}), Formula (II_{B}), Formula (II_{C}), Formula (II_{D}), Formula (II_{E}), Formula (II_{F}), Formula (II_{G}) or Formula (II_{H}), as detailed above, possess the property of strengthening the fragrance fixative properties of said fixative (Fix), thereupon resulting in an improved fragrance longevity of a range of fragrance components so as to achieve an improved or prolonged perception of the fragrance profile by the consumer.

The inventors have also surprisingly found that those co-fixatives (c-F), being lactone-containing compounds, which are of Formula (II_{A}), Formula (II_{B}), Formula (II_{C}), Formula (II_{D}), Formula (II_{E}), Formula (II_{F}), Formula (II_{G}) or Formula (II_{H}), as detailed above, can be partially replaced in the composition (F) by the fixative (Fix), as detailed above, said fixative (Fix) first of all not adding in itself to the character of the fragrance profile as perceived by the consumer and secondly, thereby successfully resulting in an improved composition (F) that is more cost efficient, more environmentally friendly, and less harmful, while maintaining fragrance longevity of said composition (F) and thus keeping the overall performance of the composition (F). In particular the at least one fragrance component as comprised within said composition (F) is released over an extended period of time so as to achieve a maintained prolonged perception of the fragrance profile by the consumer.

According to the present invention, the composition (F) further comprises a co-fixative (c-F), wherein said co-fixative (c-F) is a co-fixative chosen among those of Formula (II_{A}) [co-fixative (c-F) of class (I), herein after], Formula (II_{B}) [co-fixative (c-F) of class (II), herein after], Formula (II_{C}) [co-fixative (c-F) of class (III), herein after], Formula (II_{D}) [co-fixative (c-F) of class (IV), herein after], Formula (II_{E}) [co-fixative (c-F) of class (V), herein after], Formula (II_{F}) [co-fixative (c-F) of class (VI), herein after], Formula (II_{G}) [co-fixative (c-F) of class (VII), herein after], or Formula (II_{H}) [co-fixative (c-F) of class (VIII), herein after] 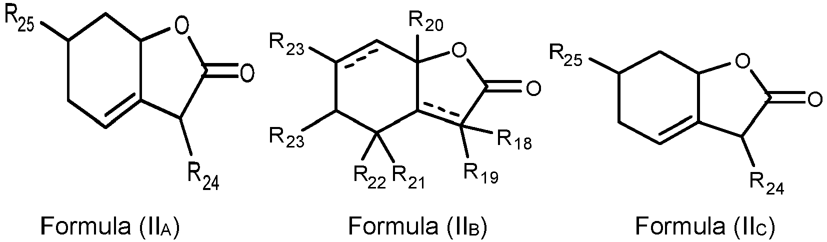 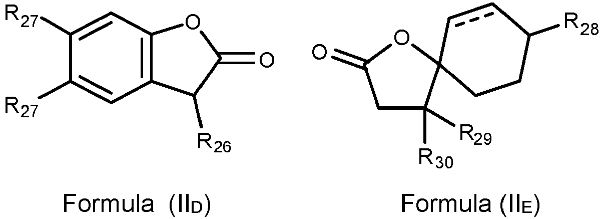 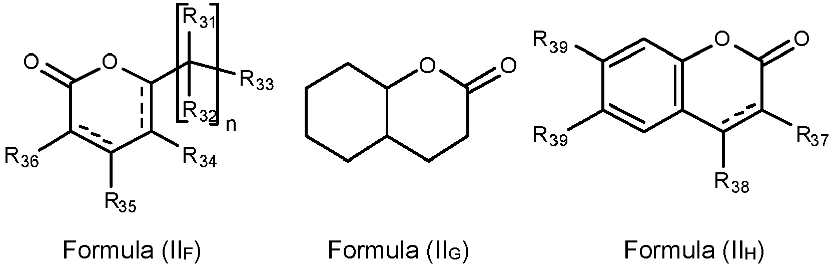 wherein the dash bond represents an optional double bound; provided that when there is a double bond in Formula (II_{A}), R₁₆ is not present; provided that when there is a double bond in the fused 5-membered lactone ring in Formula (II_{B}), R₁₉ is not present;
and wherein
- each of m, and n is an integer selected from 0, 1, 2, 3, 4, 5, 6, 7, 8, 9, 10, 11, or 12;
- each of R₁₁, R₁₂, R₃₁, and R₃₂, equal to or different from each other and at each occurrence, is independently selected from hydrogen, C₁₋₈ alkyl, C₂₋₈ alkenyl, or OH; wherein R₁₁ and R₁₂ can together form =O;
- each of R₁₃, R₁₅, R₁₆, R₁₈, R₁₉, R₂₀, R₂₁, R₂₂, R₂₄, R₂₆, R₂₇, R₂₉, R₃₀, R₃₃, R₃₅, R₃₆, R₃₇, R₃₈, and R₃₉, equal to or different from each other and at each occurrence, is independently selected from hydrogen, or methyl; wherein R₁₈ and R₁₉ can together form =CH₂ provided that there is no double bond in the fused 5-membered lactone ring of Formula (II_{B}); wherein R₃₇ and R₃₈ can together form a C₃ cycloalkyl provided that there is no double bond in the fused 6-membered lactone ring of Formula (II_{H});
- each of R₁₄, and R₃₄, equal to or different from each other and at each occurrence, is independently selected from hydrogen, C₁₋₈ alkyl, or C₂₋₈ alkenyl;
- each of R₁₇ is independently selected from hydrogen, methyl, or OH;
- each of R₂₃, and R₂₅, equal to or different from each other and at each occurrence, is independently selected from hydrogen, C₁₋₄ alkyl, or C₂₋₄ alkenyl;
- each of R₂₈ is independently selected from hydrogen, or C₁₋₄ alkyl.

According to one embodiment of the present invention, the co-fixative (c-F) is a co-fixative (c-F) of Formula (II_{A}) [co-fixative (c-F) of class (I), herein after], wherein said co-fixative (c-F) of Formula (II_{A}) is a co-fixative (c-F) chosen among those of Formula (II_{A1}) [co-fixative (c-F) of class (I₁), herein after], or Formula (II_{A2}) [co-fixative (c-F) of class (I₂), herein after] 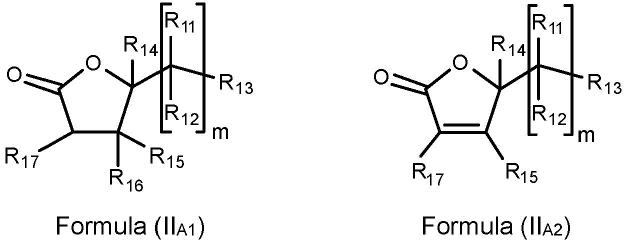 wherein m, R₁₁, R₁₂, R₁₃, R₁₄, R₁₅, R₁₆, and R₁₇ have the same meaning as defined above for Formula (II_{A}).

According to a preferred embodiment of the present invention, the co-fixative (c-F) of class (I₁) is a co-fixative (c-F) of Formula (II_{A1-1}) [co-fixative (c-F) of class (I₁₋₁), herein after] 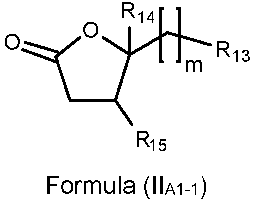 wherein
- each of m is an integer selected from 0, 1, 2, 3, 4, 5, 6, 7, 8, 9, 10, 11, or 12;
- each of R₁₃ is independently selected from hydrogen, or methyl;
- each of R₁₄ is independently selected from hydrogen, or C₁₋₈ alkyl;
- each of R₁₅ is independently selected from hydrogen, or methyl;

In a preferred embodiment of the composition (F) according to the present invention, each of m is an integer selected from 0, 1, 2, 3, 4, 5, 6, 7, 8, 9, or 10, or each of m is an integer selected from 0, 1, 2, 3, 4, 5, 6, 7, or 8, or each of m is an integer selected from 0, 1, 2, 3, 4, 5, or 6, or each of m is an integer selected from 1, 2, 3, 4, 5, or 6. Desirably, each of m is an integer selected from 2, 3, 4, 5, or 6, more desirably, each of m is an integer selected from 3, 4, 5, or 6.

In a preferred embodiment of the composition (F) according to the present invention, each of R₁₃ is methyl.

In a preferred embodiment of the composition (F) according to the present invention, each of R₁₄ is independently selected from hydrogen, or C₁₋₆ alkyl, or each of R₁₄ is independently selected from hydrogen, or C₁₋₄ alkyl, or each of R₁₄ is independently selected from hydrogen, methyl, ethyl, propyl, or isopropyl, or each of R₁₄ is independently selected from hydrogen, methyl, or ethyl. Desirably, each of R₁₄ is independently selected from hydrogen, or methyl, more desirably, each of R₁₄ is hydrogen.

In a preferred embodiment of the composition (F) according to the present invention, each of R₁₅ is hydrogen.

Preferred co-fixatives (c-F) of class (I₁₋₁) are selected from those of Formula (II_{A1-1a}) to (II_{A1-1p}) herein below

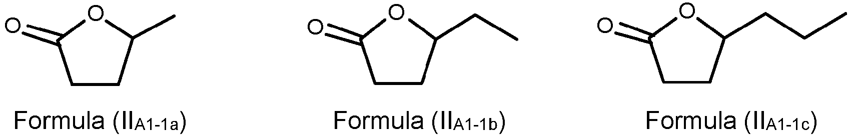

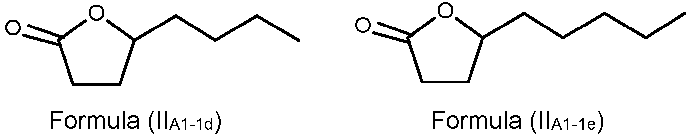

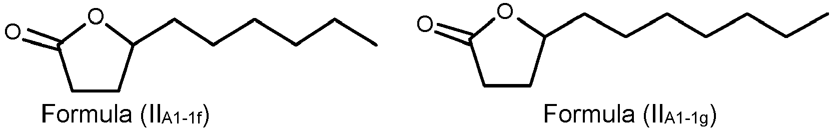

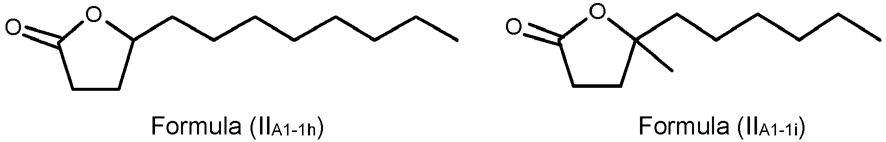

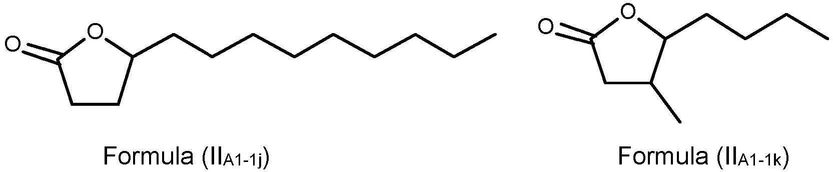

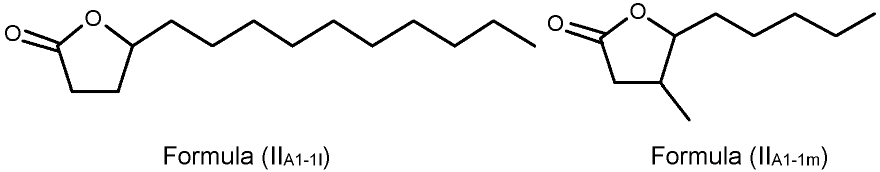

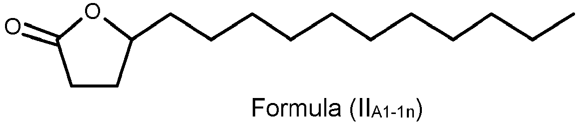

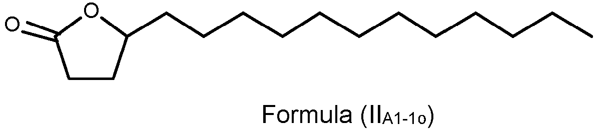

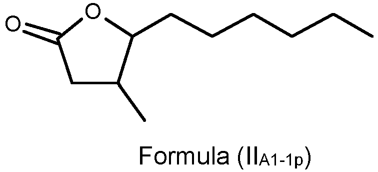

Most preferred co-fixative (c-F) of class (I₁₋₁) is the one of Formula (II_{A1-1f}) herein below

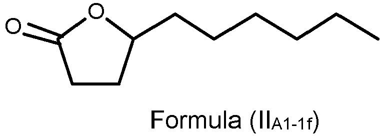

According to one embodiment of the present invention, the co-fixative (c-F) of class (I₁) is a co-fixative (c-F) of Formula (II_{A1-2}) [co-fixative (c-F) of class (I₁₋₂), herein after] 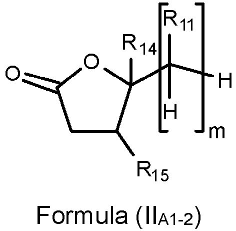 wherein
- each of m is an integer selected from 1, 2, 3, 4, 5, 6, 7, 8, 9, 10, 11, or 12;
- each of R₁₁ is independently selected from hydrogen, or C₂₋₈ alkenyl;
- each of R₁₄ is independently selected from hydrogen, or C₁₋₈ alkyl;
- each of R₁₅ is independently selected from hydrogen, or methyl;

In a preferred embodiment of the composition (F) according to the present invention, each of m is an integer selected from 1, 2, 3, 4, 5, 6, 7, 8, 9, or 10, or each of m is an integer selected from 1, 2, 3, 4, 5, 6, 7, or 8, or each of m is an integer selected from 1, 2, 3, 4, 5, or 6, or each of m is an integer selected from 1, 2, 3, 4, or 5, or each of m is an integer selected from 1, 2, 3, or 4, or each of m is an integer selected from 1, 2, or 3. Desirably, each of m is an integer selected from 1, or 2.

In a preferred embodiment of the composition (F) according to the present invention, each of R₁₁ is independently selected from hydrogen, or C₂₋₇ alkenyl, or each of R₁₁ is independently selected from hydrogen, or C₂₋₆ alkenyl. Desirably, each of R₁₁ is independently selected from hydrogen, or C₂₋₄ alkenyl.

In a preferred embodiment of the composition (F) according to the present invention, each of R₁₄ is independently selected from hydrogen, or C₁₋₆ alkyl, or each of R₁₄ is independently selected from hydrogen, or C₁₋₄ alkyl, or each of R₁₄ is independently selected from hydrogen, methyl, ethyl, propyl, or isopropyl, or each of R₁₄ is independently selected from hydrogen, methyl, or ethyl. Desirably, each of R₁₄ is independently selected from hydrogen, or methyl.

In a preferred embodiment of the composition (F) according to the present invention, each of R₁₅ is hydrogen.

Preferred co-fixatives (c-F) of class (I₁₋₂) are selected from those of Formula (II_{A1-2a}) to (II_{A1-2e}) herein below

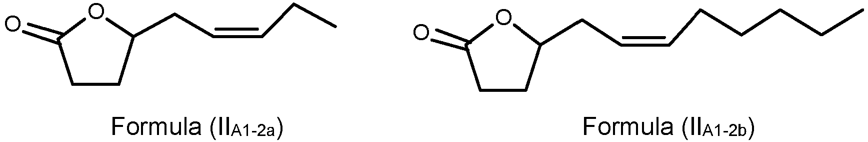

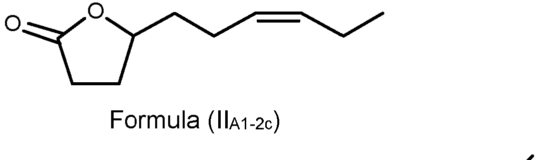

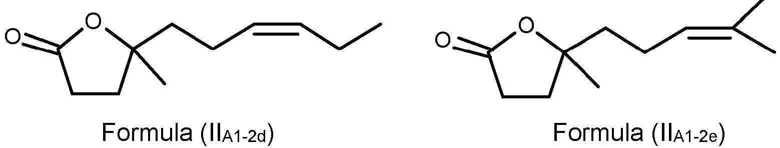

According to one embodiment of the present invention, the co-fixative (c-F) of class (I₁) is a co-fixative (c-F) of Formula (II_{A1-3}) [co-fixative (c-F) of class (I₁₋₃), herein after] 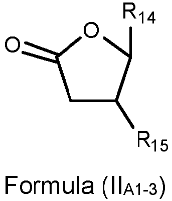 wherein
- each of R₁₄ is C₂₋₈ alkenyl;
- each of R₁₅ is independently selected from hydrogen, or methyl;

In a preferred embodiment of the composition (F) according to the present invention, each of R₁₄ is C₂₋₇ alkenyl, or each of R₁₄ is C₂₋₆ alkenyl, or each of R₁₄ is C₂₋₅ alkenyl. Desirably, each of R₁₄ is C₂₋₄ alkenyl.

In a preferred embodiment of the composition (F) according to the present invention, each of R₁₅ is hydrogen.

Preferred co-fixatives (c-F) of class (I₁₋₃) are selected from those of Formula (II_{A1-3a}) to (II_{A1-3b}) herein below

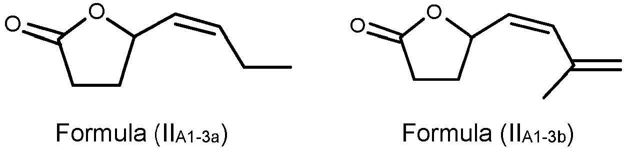

According to one embodiment of the present invention, the co-fixative (c-F) of class (I₁) is a co-fixative (c-F) of Formula (II_{A1-4}) [co-fixative (c-F) of class (I₁₋₄), herein after] 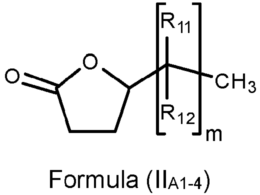 wherein
- each of m is an integer selected from 1, 2, 3, 4, 5, 6, 7, 8, 9, 10, 11, or 12;
- each of R₁₁ is independently selected from hydrogen, or OH;
- each of R₁₂ is H;
and wherein R₁₁ and R₁₂ can together form =O;

In a preferred embodiment of the composition (F) according to the present invention, each of m is selected from 1, 2, 3, 4, 5, 6, 7, 8, 9, or 10, or each of m is selected from 1, 2, 3, 4, 5, 6, 7, or 8, or each of m is selected from 1, 2, 3, 4, 5, or 6, or each of m is selected from 1, 2, 3, or 4. Desirably, each of m is selected from 1, or, 2, more desirably, each of m is 1.

Preferred co-fixatives (c-F) of class (I₁₋₄) are selected from those of Formula (II_{A1-4a}) to (II_{A1-4b}) herein below

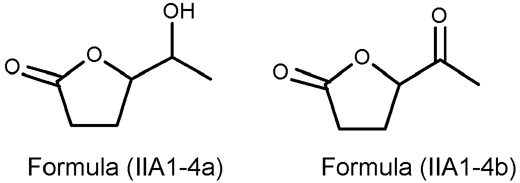

According to one embodiment of the present invention, the co-fixative (c-F) of class (I₁) is a co-fixative (c-F) of Formula (II_{A1-5}) [co-fixative (c-F) of class (I₁₋₅), herein after] 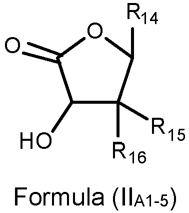 wherein
- each of R₁₄ is independently selected from hydrogen, C₁₋₈ alkyl;
- each of R₁₅ is independently selected from hydrogen, or methyl;
- each of R₁₆ is independently selected from hydrogen, or methyl;

In a preferred embodiment of the composition (F) according to the present invention, each of R₁₄ is independently selected from hydrogen, C₁₋₆ alkyl, or each of R₁₄ is independently selected from hydrogen, C₁₋₄ alkyl, or each of R₁₄ is independently selected from hydrogen, methyl, ethyl, propyl, or isopropyl, or each of R₁₄ is independently selected from hydrogen, methyl, or ethyl. Desirably, each of R₁₄ is independently selected from hydrogen, or methyl, more desirably, each of R₁₄ is hydrogen.

In a preferred embodiment of the composition (F) according to the present invention, each of R₁₅ is methyl.

In a preferred embodiment of the composition (F) according to the present invention, each of R₁₆ is methyl.

A preferred co-fixative (c-F) of class (I₁₋₅) is of Formula (II_{A1-5a}) herein below

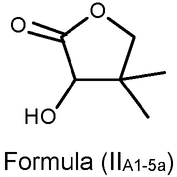

According to one embodiment of the present invention, the co-fixative (c-F) class (I₂) is a co-fixative (c-F) of Formula (II_{A2-1}) [co-fixative (c-F) of class (I₂₋₁), herein after] 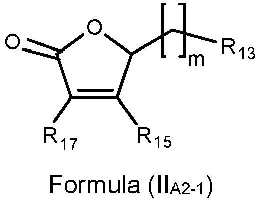 wherein
- each of m is an integer selected from 0, 1, 2, 3, 4, 5, 6, 7, 8, 9, 10, 11, or 12;
- each of R₁₃ is independently selected from hydrogen, or methyl;
- each of R₁₅ is independently selected from hydrogen, or methyl;
- each of R₁₇ is independently selected from hydrogen, or methyl;

In a preferred embodiment of the composition (F) according to the present invention, each of m is an integer selected from 0, 1, 2, 3, 4, 5, 6, 7, 8, 9, or 10, or each of m is an integer selected from 0, 1, 2, 3, 4, 5, 6, 7, or 8, or each of m is an integer selected from 0, 1, 2, 3, 4, 5, or 6, or each of m is an integer selected from 1, 2, 3, 4, 5, or 6. Desirably, each of m is an integer selected from 2, 3, 4, 5, or 6, more desirably, each of m is an integer selected from 3, 4, 5, or 6.

In a preferred embodiment of the composition (F) according to the present invention, each of R₁₃ is methyl.

Preferred co-fixatives (c-F) of class (I₂₋₁) are selected from those of Formula (II_{A2-1a}) to (II_{A2-1b}) herein below

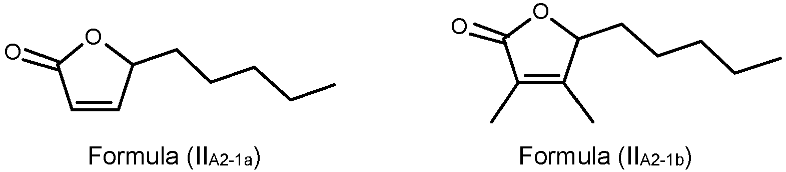

According to one embodiment of the present invention, the co-fixative (c-F) class (I₂) is a co-fixative (c-F) of Formula (II_{A2-2}) [co-fixative (c-F) of class (I₂₋₂), herein after] 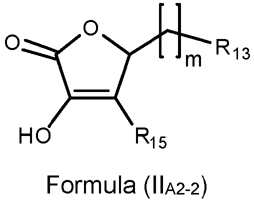 wherein
- each of m is an integer selected from 0, 1, 2, 3, 4, 5, 6, 7, 8, 9, 10, 11, or 12;
- each of R₁₃ is independently selected from hydrogen, or methyl;
- each of R₁₅ is independently selected from hydrogen, or methyl;

In a preferred embodiment of the composition (F) according to the present invention, each of m is an integer selected from 0, 1, 2, 3, 4, 5, 6, 7, 8, 9, or 10, or each of m is an integer selected from 0, 1, 2, 3, 4, 5, 6, 7, or 8, or each of m is an integer selected from 0, 1, 2, 3, 4, 5, or 6, or each of m is an integer selected from 0, 1, 2, 3, or 4, or each of m is an integer selected from 0, 1, or 2. Desirably, each of m is an integer selected from 0, or 1.

In a preferred embodiment of the composition (F) according to the present invention, each of R₁₃ is methyl.

In a preferred embodiment of the composition (F) according to the present invention, each of R₁₅ is methyl.

Preferred co-fixatives (c-F) of class (I₂₋₂) are selected from those of Formula (II_{A2-2a}) to (II_{A2-2b}) herein below

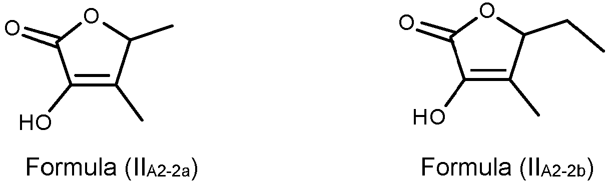

According to one embodiment of the present invention, the co-fixative (c-F) is a co-fixative (c-F) of Formula (II_{B}) [co-fixative (c-F) of class (II), herein after], wherein said co-fixative (c-F) of Formula (II_{B}) is a co-fixative (c-F) chosen among those of Formula (II_{B1}) [co-fixative (c-F) of class (II₁), herein after], Formula (II_{B2}) [co-fixative (c-F) of class (II₂), herein after], or Formula (II_{B3}) [co-fixative (c-F) of class (II₃), herein after] 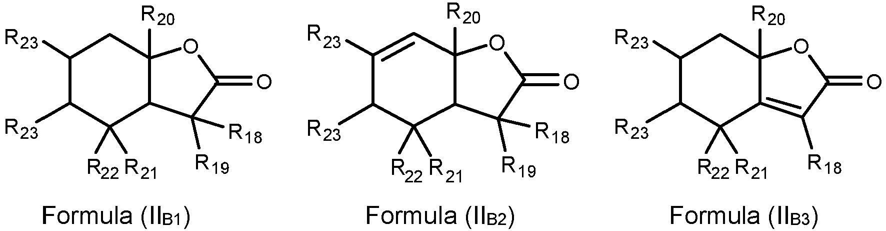 wherein R₁₈, R₁₉, R₂₀, R₂₁, R₂₂, and R₂₃ have the same meaning as defined above for Formula (II_{B}).

Preferred co-fixatives (c-F) of class (II₁) are selected from those of Formula (II_{B1-1}) to (II_{B1-3}) herein below

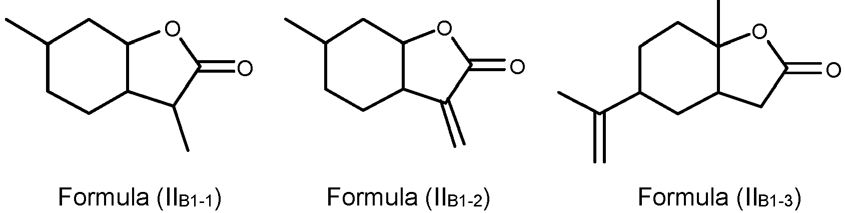

A preferred co-fixative (c-F) of class (II₂) is of Formula (II_{B2-1}) herein below

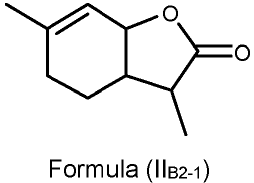

Preferred co-fixatives (c-F) of class (II₃) are selected from those of Formula (II_{B3-1}) to (II_{B3-2}) herein below

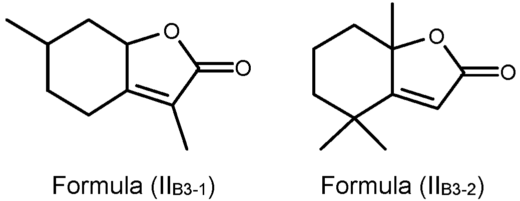

According to one embodiment of the present invention, the co-fixative (c-F) is a co-fixative (c-F) of Formula (II_{C}) [co-fixative (c-F) of class (III), herein after] 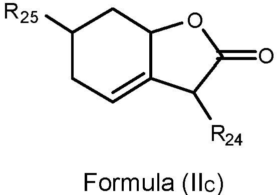 wherein R₂₄, and R₂₅ are as defined above.

A preferred co-fixative (c-F) of class (III) is of Formula (II_{C-1}) herein below

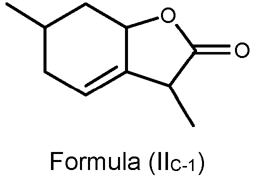

According to one embodiment of the present invention, the co-fixative (c-F) is a co-fixative (c-F) of Formula (II_{D}) [co-fixative (c-F) of class (IV), herein after] 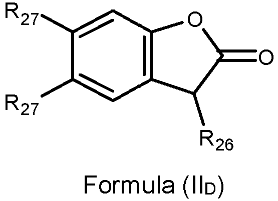 wherein R₂₆, and R₂₇ are as defined above.

A preferred co-fixative (c-F) of class (IV) is of Formula (II_{D-1}) herein below

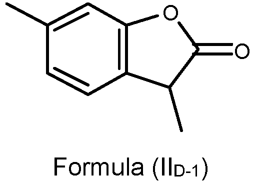

According to one embodiment of the present invention, the co-fixative (c-F) is a co-fixative (c-F) of Formula (II_{E}) [co-fixative (c-F) of class (V), herein after], wherein said co-fixative (c-F) of Formula (II_{E}) is a co-fixative (c-F) chosen among those of Formula (II_{E1}) [co-fixative (c-F) of class (V₁), herein after], or Formula (II_{E2}) [co-fixative (c-F) of class (V₂), herein after] 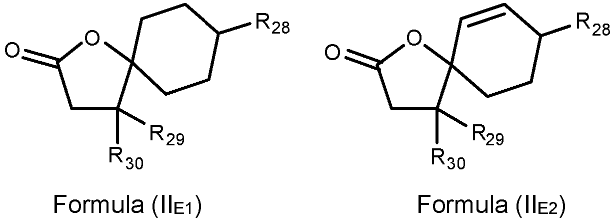 wherein R₂₈, R₂₉, and R₃₀ have the same meaning as defined above for Formula (II_{E}).

Preferred co-fixatives (c-F) of class (V₁) are selected from those of Formula (II_{E1-1}) to (II_{E1-4}) herein below

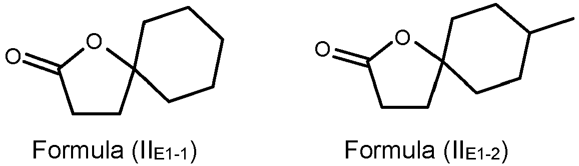

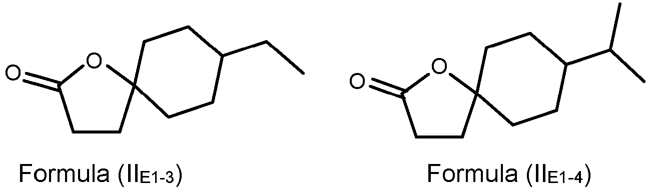

A preferred co-fixative (c-F) of class (V₂) is of Formula (II_{E2-1}) herein below

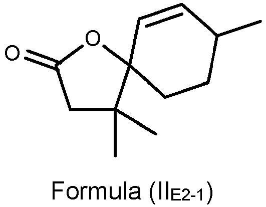

According to one embodiment of the present invention, the co-fixative (c-F) is a co-fixative (c-F) of Formula (II_{F}) [co-fixative (c-F) of class (VI), herein after], wherein said co-fixative (c-F) of Formula (II_{F}) is a co-fixative (c-F) chosen among those of Formula (II_{F1}) [co-fixative (c-F) of class (VI₁), herein after], Formula (II_{F2}) [co-fixative (c-F) of class (VI₂), herein after], or Formula (II_{F3}) [co-fixative (c-F) of class (VI₃), herein after] 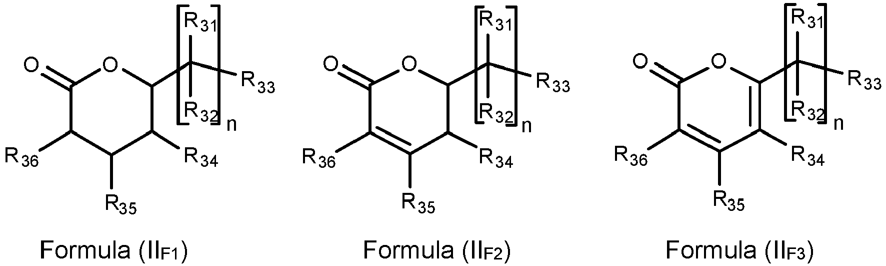 wherein n, R₃₁, R₃₂, R₃₃, R₃₄, R₃₅, and R₃₆ have the same meaning as defined above for Formula (II_{F}).

According to one embodiment of the present invention, the co-fixative (c-F) of class (VI₁) is a co-fixative (c-F) of Formula (II_{F1-1}) [co-fixative (c-F) of class (VI₁₋₁), herein after] 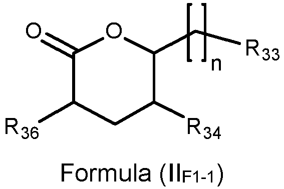 wherein
- each of n is an integer selected from 0, 1, 2, 3, 4, 5, 6, 7, 8, 9, 10, 11, or 12;
- each of R₃₃ is independently selected from hydrogen, or methyl;
- each of R₃₄ is independently selected from hydrogen, or C₁₋₈ alkyl;
- each of R₃₆ is independently selected from hydrogen, or methyl;

In a preferred embodiment of the composition (F) according to the present invention, each of n is an integer selected from 0, 1, 2, 3, 4, 5, 6, 7, 8, 9, or 10, or each of n is an integer selected from 0, 1, 2, 3, 4, 5, 6, 7, or 8, or each of n is an integer selected from 0, 1, 2, 3, 4, 5, or 6, or each of n is an integer selected from 1, 2, 3, 4, 5, or 6. Desirably, each of n is an integer selected from 2, 3, 4, 5, or 6, more desirably, each of n is an integer selected from 3, 4, 5, or 6.

In a preferred embodiment of the composition (F) according to the present invention, each of R₃₃ is methyl.

In a preferred embodiment of the composition (F) according to the present invention, each of R₃₄ is independently selected from hydrogen, or C₁₋₆ alkyl, or each of R₃₄ is independently selected from hydrogen, or C₁₋₄ alkyl, or each of R₃₄ is independently selected from hydrogen, methyl, ethyl, propyl, or isopropyl, or each of R₃₄ is independently selected from hydrogen, methyl, or ethyl. Desirably, each of R₃₄ is independently selected from hydrogen, or methyl, more desirably, each of R₃₄ is hydrogen.

In a preferred embodiment of the composition (F) according to the present invention, each of R₃₆ is hydrogen.

Preferred co-fixatives (c-F) of class (VI₁₋₁) are selected from those of Formula (II_{F1-1a}) to (II_{F1-1j}) herein below

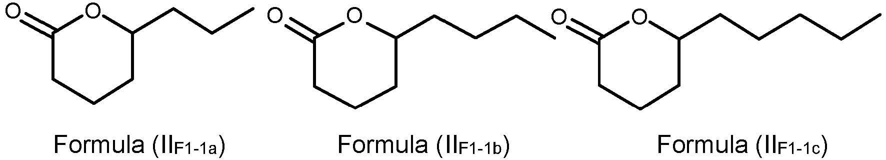

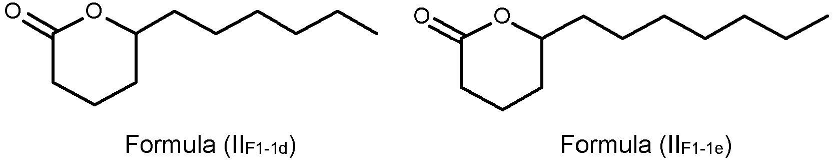

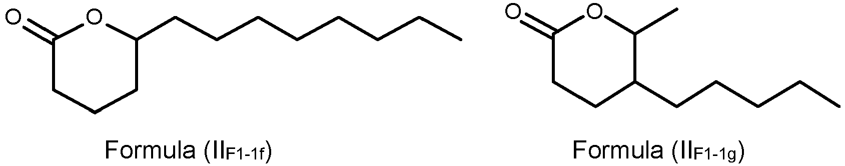

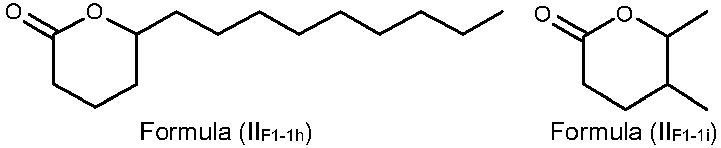

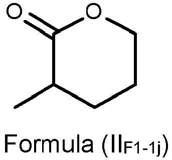

According to one embodiment of the present invention, the co-fixative (c-F) of class (VI₁) is a co-fixative (c-F) of Formula (II_{F1-2}) [co-fixative (c-F) of class (VI₁₋₂), herein after] 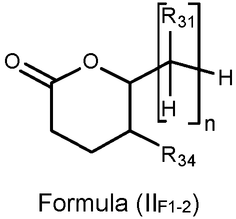 wherein
- each of n is an integer selected from 1, 2, 3, 4, 5, 6, 7, 8, 9, 10, 11, or 12;
- each of R₃₁ is independently selected from hydrogen, or C₂₋₈ alkenyl;
- each of R₃₄ is independently selected from hydrogen, or C₁₋₈ alkyl;

In a preferred embodiment of the composition (F) according to the present invention, each of n is an integer selected from 1, 2, 3, 4, 5, 6, 7, 8, 9, or 10, or each of n is an integer selected from 1, 2, 3, 4, 5, 6, 7, or 8, or each of n is an integer selected from 1, 2, 3, 4, 5, or 6, or each of n is an integer selected from 1, 2, 3, 4, or 5, or each of n is an integer selected from 1, 2, 3, or 4, or each of n is an integer selected from 1, 2, or 3. Desirably, each of n is an integer selected from 1, or 2.

In a preferred embodiment of the composition (F) according to the present invention, each of R₁₁ is independently selected from hydrogen, or C₂₋₇ alkenyl, or each of R₃₁ is independently selected from hydrogen, or C₂₋₆ alkenyl. Desirably, each of R₃₁ is independently selected from hydrogen, or C₂₋₄ alkenyl.

In a preferred embodiment of the composition (F) according to the present invention, each of R₃₄ is independently selected from hydrogen, or C₁₋₆ alkyl, or each of R₃₄ is independently selected from hydrogen, or C₁₋₄ alkyl, or each of R₃₄ is independently selected from hydrogen, methyl, ethyl, propyl, or isopropyl, or each of R₃₄ is independently selected from hydrogen, methyl, or ethyl. Desirably, each of R₃₄ is independently selected from hydrogen, or methyl, more desirably, each of R₃₄ is hydrogen.

Preferred co-fixatives (c-F) of class (VI₁₋₂) are selected from those of Formula (II_{F1-2a}) to (II_{F1-2b}) herein below

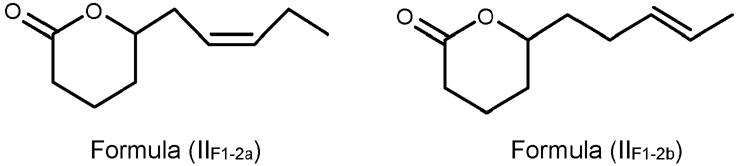

According to one embodiment of the present invention, the co-fixative (c-F) of class (VI₂) is a co-fixative (c-F) of Formula (II_{F2-1}) [co-fixative (c-F) of class (VI₂₋₁), herein after] 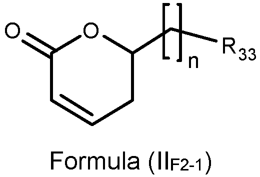 wherein
- each of n is an integer selected from 0, 1, 2, 3, 4, 5, 6, 7, 8, 9, 10, 11, or 12;
- each of R₃₃ is independently selected from hydrogen, or methyl;

In a preferred embodiment of the composition (F) according to the present invention, each of n is an integer selected from 0, 1, 2, 3, 4, 5, 6, 7, 8, 9, or 10, or each of n is an integer selected from 0, 1, 2, 3, 4, 5, 6, 7, or 8, or each of n is an integer selected from 0, 1, 2, 3, 4, 5, or 6, or each of n is an integer selected from 1, 2, 3, 4, 5, or 6. Desirably, each of n is an integer selected from 2, 3, 4, 5, or 6, more desirably, each of n is an integer selected from 3, 4, 5, or 6.

In a preferred embodiment of the composition (F) according to the present invention, each of R₃₃ is methyl.

A preferred co-fixative (c-F) of class (VI₂₋₁) is of Formula (II_{F2-1a}) herein below

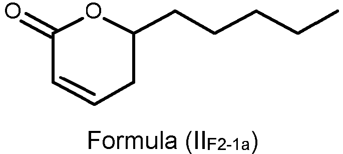

According to one embodiment of the present invention, the co-fixative (c-F) of class (VI₂) is a co-fixative (c-F) of Formula (II_{F2-2}) [co-fixative (c-F) of class (VI₂₋₂), herein after] 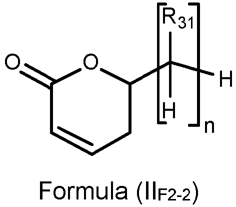 wherein
- each of n is an integer selected from 1, 2, 3, 4, 5, 6, 7, 8, 9, 10, 11, or 12;
- each of R₃₁ is independently selected from hydrogen, or C₂₋₈ alkenyl;

In a preferred embodiment of the composition (F) according to the present invention, each of n is an integer selected from 1, 2, 3, 4, 5, 6, 7, 8, 9, or 10, or each of n is an integer selected from 1, 2, 3, 4, 5, 6, 7, or 8, or each of n is an integer selected from 1, 2, 3, 4, 5, or 6, or each of n is an integer selected from 1, 2, 3, 4, or 5, or each of n is an integer selected from 1, 2, 3, or 4, or each of n is an integer selected from 1, 2, or 3. Desirably, each of n is an integer selected from 1, or 2.

In a preferred embodiment of the composition (F) according to the present invention, each of R₁₁ is independently selected from hydrogen, or C₂₋₇ alkenyl, or each of R₃₁ is independently selected from hydrogen, or C₂₋₆ alkenyl. Desirably, each of R₃₁ is independently selected from hydrogen, or C₂₋₄ alkenyl.

A preferred co-fixative (c-F) of class (VI₂₋₂) is of Formula (II_{F2-2a}) herein below

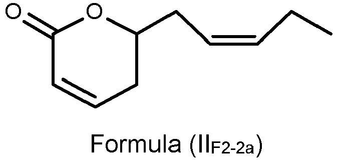

According to one embodiment of the present invention, the co-fixative (c-F) of class (VI₃) is a co-fixative (c-F) of Formula (II_{F3-1}) [co-fixative (c-F) of class (VI₃₋₁), herein after] 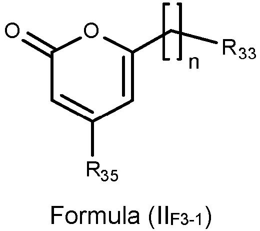 wherein
- each of n is an integer selected from 0, 1, 2, 3, 4, 5, 6, 7, 8, 9, 10, 11, or 12;
- each of R₃₃ is independently selected from hydrogen, or methyl;
- each of R₃₅ is independently selected from hydrogen, or methyl;

In a preferred embodiment of the composition (F) according to the present invention, each of n is an integer selected from 0, 1, 2, 3, 4, 5, 6, 7, 8, 9, or 10, or each of n is an integer selected from 0, 1, 2, 3, 4, 5, 6, 7, or 8, or each of n is an integer selected from 0, 1, 2, 3, 4, 5, or 6, or each of n is an integer selected from 0, 1, 2, 3, 4, or 5. Desirably, each of n is an integer selected from 0, 1, 2, 3, or 4.

In a preferred embodiment of the composition (F) according to the present invention, each of R₃₃ is methyl.

In a preferred embodiment of the composition (F) according to the present invention, each of R₃₅ is hydrogen.

Preferred co-fixatives (c-F) of class (VI₃₋₁) are selected from those of Formula (II_{F3-1a}) to (II_{F3-1b}) herein below

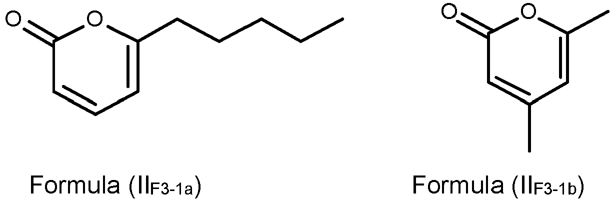

According to one embodiment of the present invention, the co-fixative (c-F) is a co-fixative (c-F) of Formula (II_{G}) [co-fixative (c-F) of class (VII), herein after]

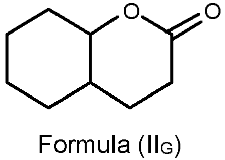

According to one embodiment of the present invention, the co-fixative (c-F) is a co-fixative (c-F) of Formula (II_{H}) [co-fixative (c-F) of class (VIII), herein after], wherein said co-fixative (c-F) of Formula (II_{H}) is a co-fixative (c-F) chosen among those of Formula (II_{H1}) [co-fixative (c-F) of class (VIII₁), herein after], or Formula (II_{H2}) [co-fixative (c-F) of class (VIII₂), herein after] 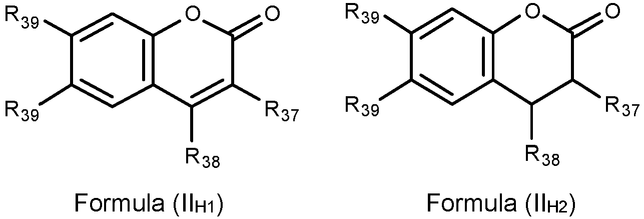 wherein R₃₇, R₃₈, and R₃₉ have the same meaning as defined above for Formula (II_{H}).

A most preferred co-fixative (c-F) of class (VIII₁) is of Formula (II_{H1-1}) herein below

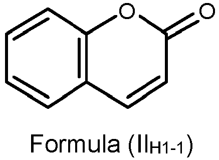

A preferred co-fixative (c-F) of class (VIII₂) is of Formula (II_{H2-1}) herein below

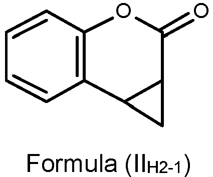

The co-fixative (c-F) chosen among those of Formula (II_{A}) to Formula (II_{H}), as specified herein, or a compound of any of the subgroups of co-fixatives (c-F) as detailed above, may have at least one center of chirality and may exist as stereochemically isomeric forms. The term "stereochemically isomeric forms" as used herein defines all the possible compounds made up of the same atoms bonded by the same sequence of bonds but having different three-dimensional structures. In this view, unless otherwise mentioned or indicated, each of the centers of chirality in the co-fixative (c-F) chosen among those of Formula (II_{A}) to Formula (II_{H}), as detailed above, or a compound of any of the subgroups of co-fixatives (c-F) as detailed above, may adopt an (*R*)- or (*S*)-configuration.

Unless otherwise mentioned or indicated, the chemical designation of the co-fixative (c-F) chosen among those of Formula (II_{A}) to Formula (II_{H}), as specified herein, or a compound of any of the subgroups of co-fixatives (c-F) as detailed above, encompasses the mixture of all possible stereochemically isomeric forms, which said compound may possess. Said mixture may contain all diastereomers and/or enantiomers of the basis molecular structure of said compound. All stereochemically isomeric forms of the compounds of the present invention both in pure form or mixed with each other are intended to be embraced within the scope of the present invention.

Pure stereoisomeric forms of the co-fixative (c-F) chosen among those of Formula (II_{A}) to Formula (II_{H}), as specified herein, or a compound of any of the subgroups of co-fixatives (c-F) as detailed above, and intermediates as mentioned herein are defined as isomers substantially free of other enantiomeric or diastereomeric forms of the same basic molecular structure of said compounds. In particular, the term "stereoisomerically pure" concerns compounds or intermediates having a stereoisomeric excess of at least 80% (in essence minimum 90% of one isomer and maximum 10% of the other possible isomers) up to a stereoisomeric excess of 100% (i.e. 100% of one isomer and none of the other), more in particular, compounds or intermediates having a stereoisomeric excess of 90% up to 100%, even more in particular having a stereoisomeric excess of 94% up to 100% and most in particular having a stereoisomeric excess of 97% up to 100%. The terms "enantiomerically pure" and "diastereomerically pure" should be understood in a similar way, but then having regard to the enantiomeric excess, and the diastereomeric excess, respectively, of the mixture in question.

In general, the co-fixative (c-F) chosen among those of Formula (II_{A}) to Formula (II_{H}) can be prepared by any methods known in the art for the manufacture of lactones. Non-limiting examples of such methods well-known in the art are intramolecular esterifications of hydroxycarboxylic acids, Yamaguchi esterifications, Baeyer-Villiger oxidations, metal-catalyzed cycloaddition reactions of alkenes with anhydrides, C-H functionalization of nonactivated C(sp³)-H bonds, radical addition of primary fatty alcohols to acrylic acid, and the like.

It is further understood that all definitions and preferences as described for the co-fixative (c-F) above equally apply for this embodiment and all further embodiments, as described below.

As said above, the amount of the co-fixative (c-F) chosen among those of Formula (II_{A}) to Formula (II_{H}), as detailed above, is from 2.50 to 50.00 wt. %, relative to the total weight of the composition (F).

Within the context of the present invention, the expression from 2.50 to 50.00 weight percentage [wt. %, herein after] of at least one fragrance co-fixative [co-fixative (c-F), herein after]" refers either to the amount of co-fixative (c-F), when the composition (F) contains only one co-fixative (c-F), or to the sum of the amounts of co-fixative (c-F), when the composition (F) contains more than one co-fixative (c-F). This being said, it means that it is necessary that, when more than one co-fixative (c-F) is present, then it is the sum of the amounts of each of said co-fixative (c-F) that ranges from 2.50 to 50.00 wt. %, relative to the total eight of the composition (F).

Advantageously, the amount of the co-fixative (c-F) chosen among those of Formula (II_{A}) to Formula (II_{H}), as detailed above, relative to the total weight of the composition (F), is equal to or greater than 5.00 wt. %, or equal to or greater than 7.50 wt. %, or equal to or greater than 10.00 wt. %, or equal to or greater than 12.50 wt. %.

It is further understood that the upper limit of the amount of the co-fixative (c-F) chosen among those of Formula (II_{A}) to Formula (II_{H}), as detailed above, relative to the total weight of the composition (F), is equal to or less than 45.00 wt. %, or equal to or less than 40.00 wt. %, or equal to or less than 35.00 wt. %, or equal to or less than 30.00 wt. %, or equal to or less than 25.00 wt. %, or equal to or less than 20.00 wt. %.

In a preferred embodiment of the composition (F) of the present invention, the co-fixative (c-F) chosen among those of Formula (II_{A}) to Formula (II_{H}), as detailed above, relative to the total weight of the composition (F), is present in an amount from 5.00 to 45.00 wt. %, or in an amount from 7.50 to 40.00 wt. %, or in an amount from 7.50 to 35.00 wt. %, or in an amount from 10.00 to 30.00 wt. %, or in an amount from 10.00 to 25.00 wt. %, or in an amount from 12.50 to 20.00 wt. %.

Advantageously, the composition (F), as detailed above, may further comprise at least one alcohol according to general Formula (III). It has been found that the at least one alcohol improves the miscibility of the fixative (Fix), as detailed above, with the other ingredients in the composition (F), in particular with the co-fixative (c-F), as detailed above, and the at least one fragrance component different to the co-fixative (c-F), as detailed above. When present, the at least one alcohol according to general Formula (III) is present in a weight ratio of the at least one alcohol to the fixative (Fix) from at least 1 to 20 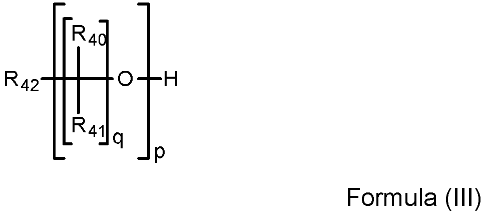 wherein
- each of p is an integer in the range from 1 to 400;
- each of q is an integer selected from 1, 2, or 3;
- each of R₄₀ is independently selected from hydrogen, methyl, ethyl, or OH;
- each of R₄₁ is independently selected from hydrogen, methyl, or ethyl;
each of R₄₂ is independently selected from methyl, or OH.

Within the context of the present invention, the expression "at least one alcohol" is intended to denote one or more than one alcohol. Mixtures of alcohols can also be used for the purpose of the invention.

In the rest of the text, the expression "alcohol" is understood, for the purposes of the present invention, both in the plural and the singular form, that is to say the composition (F) of the present invention may comprise one or more than alcohol.

In a preferred embodiment of the composition (F) according to the present invention, each of p is an integer in the range from 1 to 350, or each of p is an integer in the range from 1 to 300, or each of p is an integer in the range from 1 to 250, or each of p is an integer in the range from 1 to 200, or each of p is an integer in the range from 1 to 150, or each of p is an integer in the range from 1 to 100, or each of p is an integer in the range from 1 to 50, or each of p is an integer in the range from 1 to 40, or each of p is an integer in the range from 1 to 30, or each of p is an integer in the range from 1 to 20, or each of p is an integer in the range from 1 to 15, or each of p is an integer in the range from 1 to 10, or each of p is an integer in the range from 1 to 8, or each of p is an integer in the range from 1 to 6, or each of p is an integer in the range from 1 to 4, or each of p is an integer selected from 1, 2, or 3. Desirably, each of p is an integer selected from 1, or 2.

In a preferred embodiment of the composition (F) according to the present invention, each of R₄₀ is independently selected from hydrogen, methyl, or ethyl. Desirably, each of R₄₀ is independently selected from hydrogen, or methyl.

In a preferred embodiment of the composition (F) according to the present invention, each of R₄₁ is independently selected from hydrogen, or methyl. Desirably, each of R₄₁ is hydrogen.

Preferred alcohols of general Formula (III), as detailed above, may be chosen among ethanol, propanol, isopropanol, 1-butanol, ethylene glycol, diethylene glycol, propylene glycol, dipropylene glycol, tripropylene glycol, 1,3-butanediol, 1,2-butanediol, 2,3-butanediol, 2-methyl-1,2-propanediol, 2-methyl-1,3-propanediol, glycerol, 2-methyl-2,4-pentaandiol, polyethylene glycol monoalkyl ethers, polypropylene glycol monoalkyl ethers, or polyoxyalkylene copolymers.

As will be discussed in more detail below, the at least one alcohol ensures a good miscibility of the fixative (Fix), as detailed above, with the other ingredients in the composition (F), in particular with the co-fixative (c-F), as detailed above, and the at least one fragrance component different to the co-fixative (c-F), as detailed above.

The inventors have found that alcohols of Formula III₁), herein below, are especially suitable because of their capacity to completely or nearly completely solubilize the fixative (Fix), as detailed above, 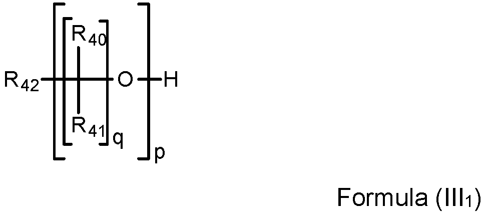 wherein
- each of p is an integer selected from 1, 2, or 3;
- each of q is an integer selected from 1, 2, or 3;
- each of R₄₀ is independently selected from hydrogen, methyl, ethyl, or OH;
- each of R₄₁ is independently selected from hydrogen, methyl, or ethyl;
- each of R₄₂ is independently selected from methyl, or OH.

In a preferred embodiment of the composition (F) according to the present invention, each of p is an integer selected from 1, or 2.

In a preferred embodiment of the composition (F) according to the present invention, each of q is an integer selected from 1, or 2.

In a preferred embodiment of the composition (F) according to the present invention, each of R₄₀ is independently selected from hydrogen, methyl, or ethyl. Desirably, each of R₄₀ is independently selected from hydrogen, or methyl.

In a preferred embodiment of the composition (F) according to the present invention, each of R₄₁ is independently selected from hydrogen, or methyl. Desirably, each of R₄₁ is hydrogen.

In a preferred embodiment of the composition (F) according to the present invention, R₄₂ is OH.

Preferred alcohols of Formula (III₁), as detailed above, to nearly solubilize or completely solubilize the fixative (Fix), as detaile above, may be chosen among ethanol, propanol, isopropanol, 1-butanol, ethylene glycol, diethylene glycol, propylene glycol, dipropylene glycol, tripropylene glycol, 1,3-butanediol, 1,2-butanediol, 2,3-butanediol, 2-methyl-1,2-propanediol, 2-methyl-1,3-propanediol, glycerol, or 2-methyl-2,4-pentaandiol.

It is further understood that all definitions and preferences as described for the at least one alcohol above equally apply for this embodiment and all further embodiments, as described below.

As said above, when present in the composition (F), as detailed above, the at least one alcohol according to general Formula (III), as detailed above, is present in a weight ratio of the at least one alcohol to the fixative (Fix) from at least 1 to 20.

Within the context of the present invention, the expression "the at least one alcohol is present in a weight ratio of the at least one alcohol to the fixative (Fix) from at least 1 to 20" refers either to the amount of alcohol, when the composition (F) contains only one alcohol according to general Formula (III), or to the sum of the amounts of alcohol, when the composition (F) contains more than one alcohol according to general Formula (III). This being said, it means that it is necessary that, when more than one alcohol according to general Formula (III) is present, then it is the sum of the amounts of each of said alcohol that fulfills the weight ratio of the at least one alcohol to the fixative (Fix) being from at least 1 to 20.

Advantageously, the weight ratio of the at least one alcohol according to general Formula (III), as detailed above, to the fixative (Fix), as detailed above, is from at least 1 to 17, or from at least 1 to 14, or from at least 1 to 12, or from at least 1 to 10, or from at least 1 to 7, or from at least 1 to 5.

It is further understood that the upper limit of the weight ratio of the at least one alcohol according to general Formula (III), as detailed above, to the fixative (Fix), as detailed above, is not critical. Desirably, the weight ratio of the at least one alcohol to the fixative (Fix) is from at most 20 to 1, or from at most 15 to 1, or from at most 10 to 1, or from at most 7 to 1, or from at most 4 to 1, or from at most 1 to 1, or from at most 1 to 2.

In a preferred embodiment of the composition (F) of the present invention, the weight ratio of the at least one alcohol according to general Formula (III), as detailed above, to the fixative (Fix), as detailed above, is from (1 to 17) to (20 to 1), or from (1 to 14) to (15 to 1), or from (1 to 12) to (10 to 1), or from (1 to 10) to (10 to 1), or from (1 to 7) to (7 to 1), or from (1 to 7) to (4 to 1), or from (1 to 5) to (1 to 1), or from (1 to 5) to (1 to 2).

According to the present invention, the composition (F) further comprises at least one fragrance component, wherein said fragrance component is different to the co-fixative (c-F), as detailed above.

It is understood that the composition (F) according to the present invention may contain all kinds of fragrance components of natural and/or synthetic origin, wherein said fragrance components are different to the co-fixative (c-F) chosen among those of Formula (II_{A}) to Formula (II_{H}), as detailed above. These fragrance components are well known to the person skilled in the art of perfuming and/or aromatizing consumer products and are described in detail in for example Perfume Flavors and Chemicals, Volume I and II by S. Arctander (1969) and Ullmann's Encyclopedia of Industrial Chemistry, Laundry Detergents by Smulders, E. et al. (2007).

The term "at least one fragrance component" as used herein shall be understood to refer to individual or single fragrance components or substances as well as mixtures thereof.

It is common for a plurality of fragrance components to be present in the composition (F). Preferably, the composition (F) of the present invention comprises two or more, preferably three or more, more preferably four or more, more preferably five or more, more preferably six or more or even seven or more different fragrance components.

Suitable fragrance components for use in the composition (F) according to the present invention include, but are not limited to: aldehyde fragrance components, ketone fragrance components, Schiff-Bases and other natural, synthetic or artificial fragrance components such as the perfume ingredients and fragrance components notably described in US 7,601,681 B2.

Non-limiting examples of suitable fragrance components for use in the composition (F), as detailed above, include anethole, benzaldehyde, decyl aldehyde, pentyl (amyl) acetate, benzyl acetate, benzyl alcohol, benzyl formate, benzyl propionate, isobornyl acetate, camphene, citral, *cis*-citral (neral), citronellal, citronellol, citronellyl acetate, *para*-cymene, decanal, dihydrolinalool, dihydromyrcenol, dimethyl phenyl carbinol, eucalyptol, *trans-*citral (geranial), geraniol, geranyl acetate, geranyl nitrile, helional, *cis-3-*hexenol, *cis*-3-hexenyl acetate, phenyl ethyl acetate, dihydrocitronellal, D-limonene, L-limonene, linalool oxide, tetrahydolinalool, linalyl acetate, linalyl propionate, ethyl anthranilate, methyl anthranilate, alpha-methyl ionone, methyl nonyl acetaldehyde, methyl benzyl carbinyl acetate, L-menthyl acetate, DL-isomenthone, myrcene, myrcenyl acetate, myrcenol, nerol, neryl acetate, nonyl acetate, prenyl acetate, 2-phenylethanol, phenyl acetaldehyde, alphapinene, beta-pinene, gamma-terpinene, alpha-terpineol, beta-terpineol, alphaterpinyl acetate, (4-tert-butylcyclohexyl) acetate, (E)-alpha-damascone, (E)-beta-damascone, undecylenic aldehyde, alpha-pentylcinnamaldehyde, isoamyl salicylate, beta-caryophyllene, alpha-cedrene, cinnamyl alcohol, cymal, dimethyl benzyl carbinyl acetate, dimethyl benzyl carbinol, ethyl vanillin, eugenol, iso-eugenol, dihydro-norcyclopentadienyl acetate, dihydro-norcyclopentadienyl propionate, heliotropine, cyclohexyl salicylate, hexyl salicylate, alpha-isomethylionone, nerolidol, patchouli alcohol, 6-phenyl-1-hexanol, beta-selinene, (2,2,2-trichloro-1-phenylethyl) acetate, trimethyl citrate, vanillin, 3,4-dimethoxybenzaldehyde, benzophenone, benzyl salicylate, ethylene brassylate, galaxolide (trade name for 1,3,4,6,7,8-hexahydro-4,6,6,7,8,8-hexamethylcyclopentagamma-2-benzopyran), hexyl cinnamic aldehyde, 4-(4-hydroxy-4-methylpentyl)cyclohex-3-ene-1-carbaldehyde (lyral), methyl cedryl ketone, dihydro isojasmonate, methyl dihydrojasmonate, betanaphthyl methyl ketone, musk indanone, musk ketone, musk tibetene, 2-phenylethyl 2-phenylacetate, methyl 2,4-dihydroxy-3,6-dimethylbenzoate, cyclopentane acetic acid 3-oxo-2-pentyl-methyl ester (hedione), prop-2-enyl 2-(3-methylbutoxy)acetate (allyl amyl glycolate), Iso E super (trade name for 1-(1,2,3,4,5,6,7,8-octahydro-2,3,8,8-tetramethyl-2-naphthalenyl)-ethanone), ethyl cinnamate, ethyl dihydro cinnamate, bangalol, 2,4,6-trimethylbenzaldehyde, 2,6-dimethyl-2-heptanol, phenethyl isobutyrate, ethyl hexyl ketone, butyl propyl ketone, propyl amyl ketone, ethyl amyl ketone, dibutyl ketone, heptyl methyl ketone, hexyl methyl ketone, 4,5-dihydrotoluene, caprylic aldehyde, isopropyl benzoate, cyclohexane propionic acid, L-carvone, campholene aldehyde, caprylic acid, caprylic alcohol, cuminaldehyde, 1-ethyl-4-nitrobenzene, heptyl formate, 4-isopropylphenol, 2-isopropylphenol, 3-isopropylphenol, 4-methyl-1-phenyl-2-pentanone, 2-propylfuran, allyl caproate, styrene, isoeugenyl methyl ether, eugenyl methyl ether, indonaphthene, diethyl suberate, L-menthone, *para*-cresyl isobutyrate, butyl butyrate, ethyl hexanoate, propyl valerate, *n*-pentyl propanoate, 2,6-xylenol, 2,4-xylenol, 2,5-xylenol, 3,5-xylenol, 2,3-xylenol, 3,4-xylenol, hexyl methyl ether, benzyl ethyl ether, methyl cinnamate, di-isobutylketone, ethyl benzoate, methyl 2-methylbenzoate, methyl 4-methylbenzoate, methyl 3-methylbenzoate, *sec*-butyl n-butyrate, ethyl *para*-anisate, 2-ethyl-1-hexanol, benzyl isobutyrate, 2-ethylbutyl acetate, isobutyl isobutanoate, propyl isovalerate, lepidine, 2-ethylbenzaldehyde, 4-ethylbenzaldehyde, *ortho-*ethylphenol, *para*-ethylphenol, *meta*-ethylphenol, para-isopropylbenzyl alcohol, 2-ethylhexanal, ethyl amyl carbinol, (+)-pulegone, coumarone, *trans-*3,3,5-trimethylcyclohexanol, and fenchyl alcohol.

In one embodiment of the composition (F) according to the present invention, in order to increase the fragrance deposition and longevity, highly water-soluble fragrance components are preferably to be avoided. The aqueous solubility of a fragrance component may be determined from the calculated octanol/water partition coefficient of the fragrance component which is expressed as cLogP. The cLogP value of a fragrance component is the calculated logarithm of the partition coefficient of said fragrance component between octanol and water. The higher the cLogP value the greater the hydrophobicity. The cLogP value of the fragrance component can be calculated from the molecular structure of the fragrance component by using commercially available software such as ChemDraw Professional 17 Suite available from PerkinElmer Informatics. In general, the higher the cLogP value, the greater the longevity of the fragrance component.

Non-limiting examples of suitable fragrance components having a cLogP value of equal to or greater than 2.0 notably include: Iso E super, citronellol, ethyl cinnamate, bangalol, 2,4,6-trimethylbenzaldehyde, hexyl cinnamic aldehyde, 2,6-dimethyl-2-heptanol, diisobutylcarbinol, para-cymene, dihydro myrcenol, ethyl salicylate, phenethyl isobutyrate, ethyl hexyl ketone, propyl amyl ketone, dibutyl ketone, heptyl methyl ketone, prop-2-enyl 2-(3-methylbutoxy)acetate (allyl amyl glycolate), 4,5-dihydrotoluene, caprylic aldehyde, citral, geranial, neral, isopropyl benzoate, cyclohexane propionic acid, laevo-carvone, campholene aldehyde, caprylic acid, caprylic alcohol, cuminaldehyde, 1-ethyl-4-nitrobenzene, heptyl formate, 4-isopropylphenol, 2-isopropylphenol, 3-isopropylphenol, allyl disulfide, 4-methyl-1-phenyl-2-pentanone, 2-propylfuran, allyl caproate, styrene, isoeugenyl methyl ether, indonaphthene, diethyl suberate, I-menthone, menthone racemic, p-cresyl isobutyrate, butyl butyrate, ethyl hexanoate, propyl valerate, n-pentyl propanoate, hexyl acetate, methyl heptanoate, trans-3,3,5-trimethylcyclohexanol, 3,3,5-trimethylcyclohexanol, ethyl p-anisate, 2-ethyl-1 - hexanol, benzyl isobutyrate, 2,5-dimethylthiophene, isobutyl 2-butenoate, caprylnitrile, iso-bornyl acetate, carvacrol, nerol, geraniol, cis-jasmone, indole, methyl dihydrojasmonate, 1-vinylheptanol, eucalyptol, 4-terpinenol, dihydrocarveol, ethyl 2-methoxybenzoate, ethyl cyclohexanecarboxylate, 2-ethylhexanal, galaxolide (trade name for 1,3,4,6,7,8-hexahydro-4,6,6,7,8,8-hexamethylcyclopentagamma-2-benzopyran), ethyl amyl carbinol, 2-octanol, ethyl methylphenylglycidate, diisobutyl ketone, coumarone, propyl isovalerate, isobutyl butanoate, isopentyl propanoate, 2-ethylbutyl acetate, 6-methyl-tetrahydroquinoline, eugenyl methyl ether, ethyl dihydrocinnamate, 3,5-dimethoxytoluene, toluene, ethyl benzoate, n-butyrophenone, alpha-terpineol, geranyl acetate, linalyl acetate, Vertenex, d-limonene, methyl 2-methylbenzoate, methyl 4- methylbenzoate, methyl 3, methylbenzoate, sec. butyl n-butyrate, 1 ,4-cineole, fenchyl alcohol, pinanol, cis-2-pinanol, 2,4, dimethylacetophenone, eugenol, isoeugenol, safrole, methyl 2-octynoate, o-methylanisole, p-cresyl methyl ether, methyl-n-methyl anthranilate, cyclopentane acetic acid 3-oxo-2-pentyl-methyl ester (hedione), ethyl anthranilate, linalool, phenyl butyrate, ethylene glycol dibutyrate, diethyl phthalate, phenyl mercaptan, cumic alcohol, m-toluquinoline, 6-methylquinoline, lepidine, 2-ethylbenzaldehyde, 4-ethylbenzaldehyde, o-ethylphenol, p-ethylphenol, m-ethylphenol, (+)-pulegone, 2,4-dimethylbenzaldehyde, isoxylaldehyde, ethyl sorbate, benzyl propionate, 1 ,3-dimethylbutyl acetate, isobutyl isobutanoate, 2,6-xylenol, 2,4-xylenol, 2,5-xylenol, 3,5-xylenol, methyl cinnamate, hexyl methyl ether, benzyl ethyl ether, methyl salicylate, butyl propyl ketone, ethyl amyl ketone, hexyl methyl ketone, 2,3-xylenol, 3,4, xylenol, cyclopentadenanolide, and phenylethyl 2-phenylacetate.

The vapor pressure and the boiling point of a fragrance component, which are related, are also important parameters to ensure that the fragrance component does not evaporate rapidly. High-boiling fragrance components will evaporate more slowly, giving a longer lasting fragrance perception. Vapor pressure and boiling point may be calculated using one of the commercially available software programs, such as ACD Software ACD/Boiling Point calculator version 4.0. Alternatively, the boiling point and vapor pressure may be measured by thermal analysis, for example according to ASTM E1782. In addition to calculating or measuring vapor pressures and boiling points, both can be found in various reference books such as CRC Handbook of Chemicals and Physics, various editions; and Chemical Properties Handbook, Yaw, Carl L., editor; McGraw-Hill Publishing Company, 1999.

Non-limiting examples of suitable fragrance components having a cLogP value of equal to or greater than 2.0 and a boiling point equal to or greater than 250°C notably include: allyl cyclohexane propionate, amyl benzoate, amyl cinnamate, amyl cinnamic aldehyde, amyl cinnamic aldehyde dimethyl acetal, iso-amyl salicylate, Aurantiol (trade name for hydroxy-citronellal-methylanthranilate), benzophenone, cyclopentane acetic acid 3-oxo-2-pentyl-methyl ester (hedione), benzyl salicylate, para-tert-butyl cyclohexyl acetate, iso-butyl quinolone, beta-caryophyllene, cadinene, cedrol, cedryl acetate, cedryl formate, cinnamyl cinnamate, cyclohexyl salicylate, cyclamen aldehyde, dihydro isojasmonate, diphenyl methane, diphenyl oxide, Iso E super (trade name for 1-(1,2,3,4,5,6,7,8-octahydro-2,3,8,8-tetramethyl-2-naphthalenyl)-ethanone), ethylene brassylate, ethyl methyl phenyl glycidate, ethyl undecylenate, galaxolide (trade name for 1,3,4,6,7,8-hexahydro-4,6,6,7,8,8-hexamethylcyclopenta-gamma-2-benzopyran), eugenol, iso-eugenol, indole, methyl cinnamate, methyl dihydrojasmonate, methyl-n-methyl anthranilate, beta-methyl naphthyl ketone, geranyl anthranilate, geranyl phenyl acetate, hexenyl salicylate, hexyl cinnamic aldehyde, hexyl salicylate, alpha-irone, Lilial (trade name for para-tertiary-butyl-alpha-methyl hydrocinnamic aldehyde), linalyl benzoate, 2-methoxy naphthalene, methyl dihydrojasmone, gamma-n-methyl ionone, musk indanone, musk ketone, musk tibetine, myristicin, patchouli alcohol, Phantolide (trade name for 5-acetyl-1,1,2,3,3,6-hexamethyl indan), phenyl ethyl benzoate, phenylethylphenylacetate, phenyl heptanol, phenyl hexanol, alpha-santalol, vetiveryl acetate, yara-yara, ylangene.

In an alternative embodiment of the composition (F) according to the present invention, the average cLogP value of all the fragrance components in the composition (F) is equal to or greater than 2.0, or equal to greater than 2.5, or equal to greater than 2.75, or equal to greater than 3.0.

The average cLogP value is determined by adding up the cLogP values of all the fragrance components in the composition (F) and dividing by the number of fragrances.

It is further understood that all definitions and preferences as described for the at least one fragrance component above equally apply for this embodiment and all further embodiments, as described below.

As said above, the amount of the at least one fragrance component, wherein said fragrance component is different to the co-fixative (c-F), as detailed above, is at least 1.00 wt. %, relative to the total weight of the composition (F).

Within the context of the present invention, the expression at least 1.00 weight percentage [wt. %, herein after] of at least one fragrance component" refers either to the amount of fragrance component, when the composition (F) contains only one fragrance component, or to the sum of the amounts of fragrance components, when the composition (F) contains more than one fragrance component. This being said, it means that it is necessary that, when more than one fragrance component is present, then it is the sum of the amounts of each of said fragrance component that is at least 1.00 wt. %, relative to the total weight of the composition (F).

Advantageously, the amount of the at least one fragrance component, wherein said fragrance component is different to the co-fixative (c-F), as detailed above, relative to the total weight of the composition (F), is at least 5.00 wt. %, or at least 10.00 wt. %, or at least 12.50 wt. %, or at least 15.00 wt. %.

It is further understood that the upper limit of the amount of the at least one fragrance component, wherein said fragrance component is different to the co-fixative (c-F), as detailed above, is not critical. Desirably, the amount of the at least one fragrance component, wherein said fragrance component is different to the co-fixative (c-F), as detailed above, relative to the total weight of the composition (F), is less than 70.00 wt. %, or less than 55.00 wt. %, or less than 40.00 wt. %, or less than 30.00 wt. %.

In a preferred embodiment of the composition (F) of the present invention, the at least one fragrance component, wherein said fragrance component is different to the co-fixative (c-F), as detailed above, relative to the total weight of the composition (F), is present in an amount from 5.00 to 70.00 wt. %, or in an amount from 10.00 to 55.00 wt. %, or in an amount from 12.50 to 40.00 wt. %, or in an amount from 15.00 to 30.00 wt. %.

Depending on the final end use of the composition (F), as detailed above, the composition (F) may further comprise water. Preferably, when the composition (F) comprises water, demineralized water is used.

Typically, the amount of water, when present, in the composition (F), as detailed above, relative to the total weight of the composition (F), is from 0.00 wt. % to 25.00 wt. %, or from 0.00 wt. % to 20.00 wt. %, or from 0.00 wt. % to 15.00 wt. %, or from 0.00 wt. % to 10.00 wt. %, or from 0.00 wt. % to 5.00 wt. %.

Another aspect of the present invention is a method for the manufacturing of the composition (F), as detailed above.

It is further understood that all definitions and preferences, as described above, equally apply for all further embodiments, as described below.

The composition (F) of the present invention can be prepared by a variety of methods known in the art. For manufacturing composition (F) of the present invention, several methods known in the art may adequately be used.

In one embodiment of the present invention, the method for the manufacturing of the composition (F), as detailed above, comprises intimate admixing of the at least one fixative (Fix) according to general Formula (IF), as detailed above, the at least one co-fixative (c-F) chosen among those of Formula (IIA) to Formula (IIH), as detailed above, optionally at least one alcohol according to general Formula (III), as detailed above, at least one fragrance component, wherein said at least one fragrance component is different to the co-fixative (c-F), as detailed above, and optionally water.

In a preferred embodiment, the method for the manufacturing of the composition (F), as detailed above, comprises the steps of intimate admixing:
- from 12.00 to 65.00 wt. %, or from 14.00 to 60.00 wt. %, or from 16.00 to 55.00 wt. %, or from 16.00 to 50.00, or from 18.00 to 45.00, or from 18.00 to 40.00, or from 20.00 to 35.00, or from 20.00 to 30.00 of the fixative (Fix), as detailed above;
- from 5.00 to 45.00 wt. %, or from 7.50 to 40.00 wt. %, or from 7.50 to 35.00 wt. %, or from 10.00 to 30.00 wt. %, or from 10.00 to 25.00 wt. %, or from 12.50 to 20.00 wt. % of the co-fixative (c-F), as detailed above;
- optionally at least one alcohol according to general Formula (III), as detailed above, wherein the at least one alcohol is present in a weight ratio of the at least one alcohol to the fixative (Fix), as detailed above, from at least 1 to 20, or from at least 1 to 17, or from at least 1 to 14, or from at least 1 to 12, or from at least 1 to 10, or from at least 1 to 7, or from at least 1 to 5;
- at least 5.00 wt. %, or at least 10.00 wt. %, or at least 12.50 wt. %, or at least 15.00 wt. % of the at least one fragrance component different to the co-fixative (c-F), as detailed above;
- optionally water; and
wherein all wt. % are relative to the total weight of the composition (F).

Typically said intimate admixing, as detailed above, may be carried out by using traditional mixers and blenders, high intensity mixers and electric stirrers.

It is understood that the skilled person in the art will carry out said intimate admixing according to general practice such as notably using optimal times, speeds, weights, volumes and batch quantities.

In a preferred embodiment of the method for the manufacturing of the composition (F), as detailed above, the fixative (Fix) is first completely or nearly completely dissolved in at least part of the alcohol having Formula (III₁), as detailed above, prior to be mixed with the co-fixative (c-F), as detailed above, the at least one fragrance component different to the co-fixative (c-F), as detailed above, and optionally water, so that the fixative (Fix) exhibits a good miscibility with the other ingredients.

Preferred alcohols of Formula (III₁), as detailed above, to nearly solubilize or completely solubilize the fixative (Fix), as detaile above, may be chosen among ethanol, propanol, isopropanol, 1-butanol, ethylene glycol, diethylene glycol, propylene glycol, dipropylene glycol, tripropylene glycol, 1,3-butanediol, 1,2-butanediol, 2,3-butanediol, 2-methyl-1,2-propanediol, 2-methyl-1,3-propanediol, glycerol, or 2-methyl-2,4-pentaandiol.

In general, the fixative (Fix) and the at least one alcohol having Formula (III₁), as detailed above, form a solution in which the weight ratio of the fixative (Fix) to the at least one alcohol having Formula (III₁), as detailed above, ranges from (20 to 1) to (1 to 20), or from (15 to 1) to (1 to 15), or from (10 to 1) to (1 to 10), or from (5 to 1) to (1 to 5).

It is further understood that all definitions and preferences, as described above, equally apply for all further embodiments, as described below.

In another aspect, the present invention also relates to a diluted composition (F) [composition (F_{D}), herein after], said composition (F_{D}) comprising the composition (F), as detailed above, and a diluent [diluent (dil), herein after].

Among diluents (dil) suitable for use in the present invention mention may be notably made of water and/or organic solvents.

When the diluent is an organic solvent, said organic solvent may be equal or different to the at least one alcohol according to general Formula (III), as detailed above. Non-limiting examples of organic solvents which are different to the at least one alcohol according to general Formula (III) notably include esters such as methyl acetate, ethyl acetate, and n-butyl acetate, acetone, paraffins, and medium chain triglycerides (MCT).

The composition (F_{D}) of the present invention can be prepared by a variety of methods known to the skilled in the art. For example, the composition (F_{D}) can be obtained by diluting the composition (F), as detailed above, with the diluent, as detailed above.

Preferably, for the composition (F_{D}), as detailed above, the weight ratio of the composition (F), as detailed above, to the diluent (dil), as detailed above, is equal to or at least 1 to 10, or equal to or at least 1 to 100, or equal to or at least 1 to 1000, or equal to or at least 1 to 10000.

It is further understood that all definitions and preferences, as described above, equally apply for all further embodiments, as described below.

A further aspect of the present invention is personal care and home care formulations comprising the composition (F), as detailed above, or the composition (F_{D}), as detailed above.

Non-limiting examples of personal care formulations notably include perfumes, deodorants, antiperspirants, antiperspirant/deodorants, body splash, shaving products, skin creams, skin cleansing formulations, skin lotions, moisturizers, toners, liquid soap, bath products, shower gel, cleansing products, shampoos, conditioners, mousses, styling gels, hair sprays, hair dyes, hair color products, hair bleaches, waving products, hair straighteners, nail polish, nail polish remover, nail creams and lotions, cuticle softeners, sunscreen, insect repellent and anti-aging products, lipsticks, foundations, face powders, eye liners, eye shadows, blushes, makeup, pet grooming products, mascaras.

Non-limiting examples of home formulations notably include laundry detergent, fabric softener, scent boosters, fabric fresheners, dishwashing liquids, wood and furniture polish, floor polish, tub and tile cleaners, toilet bowl cleaners, hard surface cleaners such as synthetic polymeric surfaces, natural stone, wood, tiles, or porcelain, kitchen cleaners, bathroom cleaners, floor cleaners, oven cleaners, window cleaners, anti-fog agents, drain cleaners, auto-dishwashing detergents and sheeting agents, carpet cleaners, prewash spotters, rust cleaners, automotive care products, leather cleaners, leather conditioners, room air fresheners, odor maskers, and scale removers.

Within the scope of the present invention, the amount of the composition (F), as detailed above, or the amount of the composition (F_{D}), as detailed above, comprised in said personal and home care formulations can vary widely depending on the specific personal and home care formulations targeted and the desired properties of their resultant personal and home care application.

In general, the amount of the composition (F) of the present invention, as detailed above, or the amount of the composition (F_{D}), as detailed above, in said personal and home care formulations, relative to the total weight of the personal and home care formulation, may vary from 0.01 wt. % to 100.00 wt. %, or from 0.10 wt. % to 80.00 wt. %, or from 0.20 wt. % to 60.00 wt. %.

According to certain embodiments of the present invention, the personal and home care formulations, as detailed above, may further comprise at least one surfactant.

Within the context of the present invention, the expression "at least one surfactant" is intended to denote one or more than one surfactant. Mixtures of surfactants can also be used for the purpose of the invention. In the remainder of the text, the term "surfactant" is understood, for the purposes of the invention both in the plural and the singular form.

When present in the personal and home care formulations, as detailed above, the surfactant can desirably be used to stabilize these formulations so that the mixture maintains one single liquid phase.

Suitable surfactants are well known to a person skilled in the art of personal and home care formulations.

Suitable surfactants include, but are not limited to: non-ionic surfactants, anionic surfactants, cationic surfactants, amphoteric surfactants, and combinations thereof.

Non-limiting examples of cationic surfactants notably include cetrimonium bromide, cetylpyridinium chloride, benzalkonium chloride, benzethonium chloride, dimethyldioctadecylammonium chloride, dioctadecylmethylammonium bromide, ditallowdimethyl ammonium methylsulfate, di(hydrogenated tallow)dimethyl ammonium methylsulfate, di(hydrogenated tallow)dimethyl ammonium chloride, distearyldimethyl ammonium methyl-sulfate, dicocodimethyl ammonium methylsulfate, di(hydrogenated tallow)dimethyl imidazolinium methylsulfate, 1-ethylene-bis(2-tallow-1-methyl) imidazolinium methylsulfate, methyl-bis(hydrogenated tallow amidoethyl)-2-hydroxethyl ammonium methylsulfate, methyl bi(tallowamidoethyl)-2-hydroxypropyl ammonium methylsulfate, N,N-di(tallowoyl-oxy-ethyl)-N,N-dimethyl ammonium methyl sulfate, N,N-di(tallowoyl-oxy-propyl)-N,N-dimethyl ammonium methyl sulfate, octyltrimethylammonium hydroxide, dodecyltrimethylammonium hydroxide, hexadecyltrimethylammonium hydroxide, octyldimethylbenzylammonium hydroxide, decyldimethylbenzylammonium hydroxide, dioctadecyldimethylammonium hydroxide, beef tallow trimethylammonium hydroxide, and coconut oil trimethylammonium hydroxide.

Non-limiting examples of anionic surfactants notably include ammonium lauryl sulfate, sodium lauryl sulfate, sodium dodecyl sulfate, sodium laureth sulfate, sodium myreth sulfate, dioctyl sodium sulfosuccinate, perfluorooctanesulfonate, perfluorobutanesulfonate, alkyl-aryl ether phosphates, alkyl ether phosphates, hexylbenzene sulphonic acid, octylbenzene sulphonic acid, decylbenzene sulfonic acid, dodecylbenzene sulphonic acid, and myristyl benzene sulphonic acid.

Non-limiting examples of amphoteric surfactants notably include alkyl betaine, alkyl dimethyl betaine, alkylamido betaine, alkyl amide betaine, alkylamidopropyl betaine, alkyl dimethylammonium betaine, alkyl amidopropyl betaine, alkyl sulfobetaine; alkyl, alkylampho glycinate, alkylamphocarboxy glycinate, alkyl or alkyl substituted imidazoline monocarboxylate, alkyl or alkyl substituted imidazoline dicarboxylate, sodium salts of alkyl monocarboxylates, sodium salts of alkyl monocarboxylates, alkyl beta amino acids, alkyl amidopropyl hydroxysultaine, alkyl ether hydroxysultaine, alkyl amidopropyl dimethyl ammonia acetate, alkyl ampho monoacetate, alkyl ampho diacetate, alkyl dipropionate, alkyl ampho dipropionate, alkyl imino dipropionate, alkyl amphopropionate, alkyl beta amino propionic acid, alkyl dipropionate, alkyl beta iminodipropionate, branched or n-alkyl dimethylamidopropionate, alkyl carboxylated propionate, alkyl imidazoline, methyl alkyl imidazoline, and fluorinated alkyl amphoteric mixtures.

Non-limiting examples of non-ionic surfactants generally include polyethoxylated fatty acids, vegetable oils, fatty alcohols, alcohol alkoxylates, alkoxylated alkyl alcohols, polyoxyethylene alkyl alcohols, polyol esters of fatty acids, polyoxyethylene esters of fatty acids, fatty acid amides, polyoxyethylene fatty acid amides, polyalkylene oxide block copolymers, and ethoxylated alkyl mercaptans.

Preferred non-ionic surfactants may be chosen among octaethylene glycol monododecyl ether, pentaethylene glycol monododecyl ether, nonoxynols, polyoxyethylene octyl phenyl ether, polyethoxylated tallow amine, cocoamide monoethanolamine, cocoamide dietholamine, poloxamers, glycerol monostearate, glycerol monolaurate, sorbitan monolaurate, sorbitan monosetearate, sorbitan tristearate, polyethylene glycol (20) sorbitan monolaurate, (i.e. PEG(20)sorbitan monolaurate or Tween 20), polyethylene glycol (20) sorbitan monostearate, polyoxyethylene (20) sorbitan monooleate, polyoxyethylene (20) sorbitan monopalmitate, decyl glucoside, lauryl glucoside, octyl glucoside, lauryldimethylamine oxide, BrijTM, glycerol, glyceryl polyethylene glycol ricinoleate (PEG); wherein the number of ethylene oxide units varies from 2 to 200, polyglyceryl ester, polyglyceryl monooleate, decaglyceryl monocaprylate, propylene glycol dicaprilate, triglycerol monostearate, and hydrogenated and ethoxylated castor oil.

As to the amount of the surfactant, it is understood that the skilled person in the art will practise said surfactant in a suitable amount according to standard and general practice known by said skilled person in the art.

Typically, the amount of the surfactant, when present, in said personal and home care formulations, relative to the total weight of the personal and home care formulation, is from 0.01 wt. % to 50.00 wt. %, or from 0.20 wt. % to 40.00 wt. %, or from 1.00 wt. % to 30.00 wt. %, or from 2.00 wt. % to 20.00 wt. %.

According to certain embodiments of the present invention, the personal and home care formulations, as detailed above, may further comprise at least one additional ingredient [ingredient (Ic), herein after] to enhance the appearance, storage, transport, handling and/or performance.

Within the context of the present invention, the expression "at least one additional ingredients [ingredient (Ic), herein after]" is intended to denote one or more than one ingredient (Ic). Mixtures of ingredients (I_{C}) can also be used for the purpose of the invention. In the remainder of the text, the expression "ingredient (Ic)" is understood, for the purposes of the present invention, both in the plural and the singular form.

Said ingredients (Ic) are known to those skilled in the art of personal and home care formulations. Non-limiting examples of ingredients (Ic) notably include pH modifiers such as hydrochloric acid, lactic acid, citric acid, acetic acid, tartaric acid, succinic acid, or malic acid, preservatives such as bactericides, germicides, thickeners, pH buffering agents, inorganic or organic salts such as water-soluble potassium, sodium, calcium, or magnesium salts, optical brighteners, fabric crisping agents, viscosity modifiers, viscosity donating agents, antioxidant agents, vitamins, spotting agents, colorants, dyes such as shading dyes, direct dyes, acid dyes, hydrophobic dyes, reactive dyes or dye conjugates, dye fixing agents or dye transfer inhibitors, fatty complexing agents, co-softeners, perfume carriers, retinoids, silicones, hydrotropes, antiredeposition agents, soil-release agents, polyelectrolytes, anti-shrinking agents, anti-wrinkle agents, anti-oxidants, anti-foaming agents, enzymes, reductive agents, herbal extracts, viscosity control agents, pigments, sunscreen or sunblock compounds, anti-corrosion agents, drape imparting agents, antistatic agents, ironing aids, pearlisers and/or opacifiers, wet soiling reduction agents such as polyorganosiloxanes, natural oils/extracts, processing aids such as electrolytes, hygiene agents such as anti-bacterials and antifungals, skin benefit agents, skin conditioners, moisturizers, and fillers such as fumed silica, precipitated silica, clay, carbon black, silicone resins, or calcium carbonates.

As to the amount of the additional ingredients (I_{C}), it is understood that the skilled person in the art will practise said additional ingredients (I_{C}) in a suitable amount according to standard and general practice known by said skilled person in the art.

Typically, the amount of the ingredient (Ic), when present, in said personal and home care formulations, relative to the total weight of the personal and home care formulation, is from 0.05 wt. % to 20.00 wt. %, or from 0.10 wt. % to 10.00 wt. %, or from 0.10 wt. % to 5.00 wt. %.

It is further understood that all definitions and preferences, as described above, equally apply for all further embodiments, as described below.

A further aspect of the present invention is a use of the composition (F), as detailed above, or the composition (F_{D}), as detailed above, for the manufacturing of personal and home care formulations.

### EXAMPLES

The invention will be now described in more details with reference to the following examples, whose purpose is merely illustrative and not intended to limit the scope of the invention.

### General procedure for manufacturing composition (F) for GC-MS experiments:

Example 1 (Ex1) was prepared by admixing and homogenizing in a clean container all the various components as described and listed in Table 1 until complete dissolution was obtained. The admixing of the various components was carried out using an electric stirrer.

Then, for enabling measurement experiments to be carried out, Example 1 (Ex1) was subsequently diluted with ethanol and water to give Example 1_{D} (Ex1_{D}) as listed in Table 1.

Examples 2_{D} - 3_{D} (Ex2_{D} to Ex3_{D}) and Comparative Examples 4_{D} - 9_{D} (CEx4_{D} to CEx9_{D}), as listed in Table 1, were prepared in the same way as for Example 1 (Ex1) and Example 1_{D} (Ex1_{D}), i.e. Examples 2 - 3 (Ex2 to Ex3) and Comparative Examples 4 - 9 (CEx4 to CEx9) (*not shown*) were subsequently diluted using ethanol and water to give Examples 2_{D} - 3_{D} (Ex2_{D} to Ex3_{D}) and Comparative Examples 4_{D} - 9_{D} (CEx4_{D} to CEx9_{D}).

With regards to Examples 1_{D} - 3_{D} (Ex1_{D} to Ex3_{D}) and Comparative Examples 4_{D} - 7_{D} (CEx4_{D} to CEx7_{D}), the fixative (Fix) according to Formula (I_{F1}) was sucrose acetate isobutyrate available as SAIB-100 from Eastman Chemical Company. The approximate ratio of acetate:isobutyrate ester groups in SAIB-100 is 2:6.

With regards to Comparative Example 8_{D} (CEx8_{D}), no use is made of any fixative (Fix) according to Formula (I_{F1}).

With regards to Comparative Example 9_{D} (CEx9_{D}), instead of a fixative (Fix) according to Formula (I_{F1}), sucrose octaacetate was used commercially available from Merck (Sigma Aldrich) Article code 252603-250G.

In Example 1_{D} (Ex1_{D}) and Comparative Examples 8_{D} - 9_{D} (CEx8_{D} to CEx9_{D}), use is made of 0.625 wt. % of the co-fixative (c-F) according to Formula (II_{A1-1f}) (CAS 706-14-9), commercially available as a mixture of (R)- and (S)-enantiomers, and 0.625 wt. % of the co-fixative (c-F) according to Formula (II_{H1**-**1}) (CAS 91-64-5).

In Example 2_{D} (Ex2_{D}), use is made of 1.52 wt. % of the co-fixative (c-F) according to Formula (II_{A1-1f}) (CAS 706-14-9), commercially available as a mixture of (R)- and (S)-enantiomers, and 1.52 wt. % of the co-fixative (c-F) according to Formula (II_{H1-1}) (CAS 91-64-5).

In Example 3_{D} (CEx3_{D}), use is made of 0.092 wt. % of the co-fixative (c-F) according to Formula (II_{A1-1f}) (CAS 706-14-9), commercially available as a mixture of (R)- and (S)-enantiomers, and 0.092 wt. % of the co-fixative (c-F) according to Formula (II_{H1-1}) (CAS 91-64-5).

In Comparative Example 4_{D} (CEx4_{D}), use is made of 1.25 wt. % of citral (CAS 5392-40-5), commercially available as a mixture of E- and Z-isomers, in essence geranial and neral, respectively.

In Comparative Example 5_{D} (CEx5_{D}), use is made of 1.25 wt. % of hedione (CAS 24851-98-7), commercially available as a mixture of cis- and trans-stereoisomers, respectively.

In Comparative Example 6_{D} (CEx6_{D}), use is made of 1.25 wt. % of galaxolide (CAS 1222-05-5), commercially available as a mixture of cis- and trans-stereoisomers in diethyl phthalate (DEP), respectively.

All contents in Table 1 are given in wt. %, relative to the total weight of the respective compositions, unless stated otherwise.

Table 1 further summarizes the total loss percentage (%) of the amounts for each of the three fragrance components benzyl alcohol (CAS 100-51-6), 2-phenylethanol (CAS 60-12-8), and prop-2-enyl 2-(3-methylbutoxy)acetate (CAS 67634-00-8), respectively, as measured according to the GC test method as described below. The results are summarized in Table 1.

### Analytical measurement method

### Sample preparation and sample storage

Experimental samples were respectively prepared by applying, i.e., blotting, 75 µL of the compositions of Examples 1_{D} - 3_{D} (Ex1_{D} to Ex3_{D}) and Comparative Examples 4_{D} - 9_{D} (CEx4_{D} to CEx9_{D}) on separate odour-free blotter strips using a micropipette. 75 µL of the compositions of Examples 1_{D} - 3_{D} (Ex1_{D} to Ex3_{D}) and Comparative Examples 4_{D} - 9_{D} (CEx4_{D} to CEx9_{D}) was evenly distributed over a distance of 5 cm of the respective blotter strips. Each blotter strip was coded in advance with the following appropriate time tags: T8h and T16h. After blotting, each blotter strip was placed vertically into an open vial without any contact of said blotter strip with the inside walls of said open vial, while each blotter strip was attached to a piece of rope above the respective vials with a clothespin. During storage of the respective blotters, sufficient air renewal was ensured to avoid any odour cross-contamination between the blotters. Each blotter strip was removed from the rope at the specified times, i.e., after 8 hours or after 16 hours. After removal, the above-mentioned 5 cm of each blotter strip was placed into a separate vial and subsequently extracted with 3 mL of ethanol. After extraction, each ethanol extract was vortexed at maximum speed during a time interval of 2 minutes. Next, a GC vial was filled for each extract and a GC-analysis was started for each extract following the GC test method as described below. The peak area of the different fragrances was monitored in function of time (i.e., 8 hours versus 16 hours).

### GC test method

GC analysis was performed using an Agilent GC 7890B instrument equipped with OpenLab 2.1 software and a HP-1 (60 m x 0.32 mm x 1 µm) column. No calibration was performed. After analysis, the peak area for each compound of interest is determined. The integration of the peaks is done automatically by the software and was manually checked. Between separate measurements, ethanol was injected as a blank injection to prevent pollution on the column.

Specific GC method parameters were the following:
- inlet:
- injector temperature: 300 °C;
- injection volume: 1.0 µL;
- split ratio: 1/5;

- column:
- HP-1: 60 m x 0.32 mm x 1 µm (Agilent #19091Z-216);

- carrier gas:
- He;
- flow: 3.0 mL/min;
- constant pressure;

- oven:
- 60°C - 1 min;
- 15°C/min - 325 °C - 8 min;

- detector:
- type: FID;
- detector temperature: 330 °C;
- H₂ flow: 30 mL/min;
- air flow: 300 mL/min;
- make up (He) + column flow: 20 mL/min

**Table 1: Composition (F): Example 1 (Ex1), Examples 1_{D} - 3_{D} (Ex1_{D} to Ex3_{D}) and Comparative Examples 4_{D} - 9_{D} (CEx4_{D} to CEx9_{D})**

| **Components** | **Ex1** | **Ex1_{D}** | **Ex2_{D}** | **Ex3_{D}** | **CEx4_{D}** | **CEx5_{D}** | **CEx6_{D}** | **CEx7_{D}** | **CEx8_{D}** | **CEx9_{D}** |
|---|---|---|---|---|---|---|---|---|---|---|
| fixative (Fix) according to Formula (I_{F1}) | 25.51 | 2.00 | 0.21 | 3.066 | 2.00 | 2.00 | 2.00 | 2.00 | - | - |
| sucrose octaacetate | - | - | - | - | - | - | - | - | - | 2.00 |
| co-fixative (c-F) according to Formula (II_{A1-1f}) | 7.97 | 0.625 | 1.52 | 0.092 | - | - | - | - | 0.625 | 0.625 |
| co-fixative (c-F) according to Formula (II_{H1-1}) | 7.97 | 0.625 | 1.52 | 0.092 | - | - | - | - | 0.625 | 0.625 |
| citral (a mixture of E- and Z-isomers, geranial and neral, respectively) | - | - | - | - | 1.25 | - | - | - | - | - |
| hedione (a mixture of cis- and trans stereoisomers, respectively) | - | - | - | - | - | 1.25 | - | - | - | - |
| galaxolide (a mixture of cis- and trans stereoisomers in diethyl phthalate (DEP), respectively) | - | - | - | - | - | - | 1.25 | - | - | - |
| benzyl alcohol | 3.19 | 0.25 | 0.25 | 0.25 | 0.25 | 0.25 | 0.25 | 0.25 | 0.25 | 0.25 |
| 2-phenylethanol | 3.19 | 0.25 | 0.25 | 0.25 | 0.25 | 0.25 | 0.25 | 0.25 | 0.25 | 0.25 |
| prop-2-enyl 2-(3-methylbutoxy)acetate (allyl amyl glycolate) | 3.19 | 0.25 | 0.25 | 0.25 | 0.25 | 0.25 | 0.25 | 0.25 | 0.25 | 0.25 |
| ethanol | 48.98 | 85.00 | 85.00 | 85.00 | 85.00 | 85.00 | 85.00 | 85.00 | 85.00 | 85.00 |
| demineralized water | - | 11.00 | 11.00 | 11.00 | 11.00 | 11.00 | 11.00 | 12.25 | 13.00 | 11.00 |
| **% loss absolute peak area: 8 hours versus 16 hours** | | | | | | | | | | |
| benzyl alcohol | - | no loss detected* | no loss detected* | no loss detected* | 12.7 | 17.0 | 23.3 | 5.5 | no loss detected* | 5.1 |
| 2-phenylethanol | - | no loss detected* | no loss detected* | no loss detected* | 11.1 | 14.0 | 19.7 | 1.5 | no loss detected* | 1.9 |
| prop-2-enyl 2-(3-methylbutoxy)acetate (allyl amyl glycolate) | - | no loss detected* | no loss detected* | no loss detected* | 10.9 | 14.4 | 30.9 | 8.4 | no loss detected* | 1.4 |

| | | | | | | | | | | |
|---|---|---|---|---|---|---|---|---|---|---|
| * within the limits of detection there was no measurement of a loss. | | | | | | | | | | |

The experimental results in Table 1 demonstrate that when the co-fixatives (c-F) according to Formula (II_{A1-1f}) and to Formula (II_{H1-1}) as contained in Comparative Example 8_{D} (CEx8_{D}) are now partially replaced by the fixative (Fix) according to Formula (I_{F}) in Example 3_{D} (Ex3_{D}), no loss in the remaining amounts was detected for each of the fragrance components benzyl alcohol, 2-phenylethanol, and prop-2-enyl 2-(3-methylbutoxy)acetate (allyl amyl glycolate), respectively.

The experimental results in Table 1 further demonstrate that in Comparative Example 7_{D} (CEx7_{D}) the sole presence of the fixative (Fix) according to Formula (I_{F}), i.e., in absence of any co-fixative (c-F), results in a loss in the remaining amounts for each of the fragrance components benzyl alcohol, 2-phenylethanol, and prop-2-enyl 2-(3-methylbutoxy)acetate (allyl amyl glycolate), respectively. The same applies mutatis mutandis for Comparative Example 9_{D} (CEx9_{D}) containing sucrose octaacetate in presence of the co-fixatives (c-F) according to Formula (II_{A1-1f}) and to Formula (II_{H1-1}). Whereas on the other hand Example 1_{D} (Ex1_{D}) demonstrates that upon replacement of sucrose octaacetate as contained in Comparative Example 9_{D} (CEx9_{D}) by the fixative (Fix) according to Formula (I_{F}), a complete retention of the amounts of benzyl alcohol, 2-phenylethanol, and prop-2-enyl 2-(3-methylbutoxy)acetate (allyl amyl glycolate) was again observed and thus thereby maintaining fragrance longevity and keeping the overall performance of the composition (F).

### General procedure for manufacturing composition (F) for longevity testing (sensory analysis of fragrance intensity):

Examples 10_{D} - 11_{D} (Ex10_{D} to Ex11_{D}) and Comparative Example 12_{D} (CEx12_{D}), as listed in Table 2, were prepared in the same way as for Example 1 (Ex1) and Example 1_{D} (Ex1_{D}), i.e. Examples 10 - 11 (Ex10 to Ex11) and Comparative Example 12 (CEx12) (*not shown*) were subsequently diluted using ethanol and water to give Examples 10_{D} - 11_{D} (Ex10_{D} to Ex11_{D}) and Comparative Example 12_{D} (CEx12_{D}).

With regards to Examples 10_{D} - 11_{D} (Ex10_{D} to Ex11_{D}), the fixative (Fix) according to Formula (I_{F1}) was sucrose acetate isobutyrate available as SAIB-100 from Eastman Chemical Company. The approximate ratio of acetate:isobutyrate ester groups in SAIB-100 is 2:6.

With regards to Comparative Example 12_{D} (CEx12_{D}), no use is made of any fixative (Fix) according to Formula (I_{F1}).

In Examples 10_{D} - 11_{D} (Ex10_{D} to Ex11_{D}) and Comparative Example 12_{D} (CEx12_{D}), use is made of 2.0 wt. % of the multi-component fragrance mixture "Perfume Honeysuckle & Sandalwood", which is commercially available from Holland Aromatics (product code 1-28569). The multi-component fragrance mixture "Perfume Honeysuckle & Sandalwood" contains 6.0 wt. % of the co-fixative (c-F) according to Formula (II_{H1-1}) (CAS 91-64-5).

All contents in Table 2 are given in wt. %, relative to the total weight of the respective compositions, unless stated otherwise.

Table 2 further summarizes the total fragrance intensity evolution as a function of time corresponding to each of the compositions of Examples 10_{D} - 11_{D} (Ex10_{D} to Ex11_{D}) and Comparative Example 12_{D} (CEx12_{D}), respectively, as measured according to the olfactive evaluation methodology as described below. The corresponding results are summarized in Table 2.

### Measurement method

### Sample preparation and sample storage

The fragrance intensity of the compositions of Examples 10_{D} - 11_{D} (Ex10_{D} to Ex11_{D}) and Comparative Example 12_{D} (CEx12_{D}) was evaluated via olfactive evaluation measurements in accordance with the standard VDI 3882 part 1, said olfactive evaluation measurements being conducted at different time points at room temperature by six selected and trained panelists. These six selected and trained panelists were selected according to standard EN 13725:2019 and trained on a n-butanol reference scale.

Samples have been prepared by applying 50 µL of the respective compositions on separate odour-free blotters using a micropipette. For each of the compositions of Examples 10_{D} - 11_{D} (Ex10_{D} to Ex11_{D}) and Comparative Example 12_{D} (CEx12_{D}), six blotters have been prepared (one for each panelist per composition to be tested). 50 µL of the compositions of Examples 10_{D} - 11_{D} (Ex10_{D} to Ex11_{D}) and Comparative Example 12_{D} (CEx12_{D}) was evenly distributed over a distance of 5 cm of the respective blotter strips. After blotting, each blotter strip was kept at room temperature and fixed per six on a blotter's holder. When fixed on a blotter's holder, sufficient attention was paid to avoid the blotter strips to overlap with each other. The blotter holders were placed in opened glass tubes each having a 45 mm diameter. The glass tubes were then fixed and stored on a board, said board being located under two aeration grids to ensure proper and sufficient air renewal. In this way, any odour cross-contamination between the blotter strips of the various examples was avoided.

The blotter strips were presented every 24 hours to six selected and trained panelists as described above. For each sample and each time point, the panelists described the corresponding olfactory profile (fragrance intensity) of said sample, respectively. For this purpose, the panelists were seated in sensory analysis cabins. The blotter strips were distributed one after the other to the panelists. Additionally, small jars with coffee beans were freely accessible to the panelists to "reset" their nose. Regarding the particular sensory measurements, each member of the panelist smelled the sampled composition on the respective blotter strip and evaluated the corresponding intensity on a scale ranging from 0 to 5 (5 = odour intensity is very high; 4 = odour intensity is high; 3 = odour intensity is medium; 2 = odour intensity is weak; 1 = odour intensity is very weak; 0 = odour is not perceptible). The average fragrance intensity of the respective composition is calculated according to the arithmetic mean, of each individual measurement result.

**Table 2: Composition (F): Examples 10_{D} - 11_{D} (Ex10_{D} to Ex11_{D}) and Comparative Example 12_{D} (CEx12_{D})**

| **Components** | **Ex10_{D}** | **Ex11_{D}** | **CEx12_{D}** |
|---|---|---|---|
| fixative (Fix) according to Formula (I_{F1}) | 0.50 | 2.00 | 0.00 |
| multi-component fragrance mixture "Perfume Honeysuckle & Sandalwood" [1] | 2.00 | 2.00 | 2.00 |
| ethanol | 84.00 | 84.00 | 84.00 |
| demineralized water | 13.50 | 12.00 | 14.00 |

| **Average intensity** [2] | | | |
|---|---|---|---|
| day 0 | 4.2 (0.8) | 4.0 (0.9) | 4.2 (0.4) |
| day 1 | 3.3 (0.5) | 3.0 (0.6) | 2.7 (0.5) |
| day 2 | 3.0 (0.6) | 3.3 (0.8) | 3.2 (0.8) |
| day 4 | 2.3 (0.8) | 2.8 (0.4) | 2.7 (0.5) |
| day 7 | 3.2 (1.0) | 3.0 (0.6) | 3.2 (1.1) |
| day 8 | 2.5 (0.5) | 2.5 (0.5) | 2.8 (0.4) |
| day 9 | 2.3 (0.8) | 2.7 (0.5) | 2.5 (0.5) |
| day 10 | 2.3 (0.8) | 2.7 (0.5) | 2.8 (0.4) |
| day 11 | 2.3 (0.5) | 2.3 (0.5) | 2.3 (0.5) |

| | | | |
|---|---|---|---|
| [1] The multi-component fragrance mixture "Perfume Honeysuckle & Sandalwood" contains 6.00 wt. % of the co-fixative (c-F) according to Formula (II_{H1-1}) (CAS 91-64-5). [2] The standard deviation is described between brackets following the averaged fragrance intensity values. | | | |

The experimental results in Table 2 demonstrate that when the fixative (Fix) according to Formula (I_{F1}) is added to the compositions in Examples 10_{D} - 11_{D} (Ex10_{D} to Ex11_{D}) containing the co-fixative (c-F) according to Formula (II_{H1-1}), the fragrance intensity of said compositions was maintained when compared to the composition of Comparative Example 12_{D} (CEx12_{D}) in absence of the fixative (Fix) according to Formula (I_{F1}), respectively.

## Claims

1. A fragrance delivery composition [composition (F), herein after] comprising, relative to the total weight of the composition (F):
a) from 10.00 to 70.00 weight percentage [wt. %, herein after] of at least one fragrance fixative [fixative (Fix), herein after], wherein said fixative (Fix) has a general Formula (I_{F}) 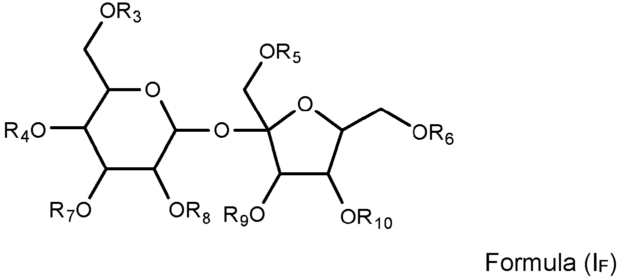 wherein
- each of R₃, R₄, R₅, R₆, R₇, R₈, R₉, and R₁₀, equal to or different from each other and at each occurrence, are COR₁, wherein each of R₁ is independently selected from methyl, or isopropyl;
with the proviso that at least one R₁ is isopropyl
b) from 2.50 to 50.00 wt. % of at least one fragrance co-fixative [co-fixative (c-F), herein after], wherein said co-fixative (c-F) is a co-fixative chosen among those of Formula (II_{A}) [co-fixative (c-F) of class (I), herein after], Formula (II_{B}) [co-fixative (c-F) of class (II), herein after], Formula (II_{C}) [co-fixative (c-F) of class (III), herein after], Formula (II_{D}) [co-fixative (c-F) of class (IV), herein after], Formula (II_{E}) [co-fixative (c-F) of class (V), herein after], Formula (II_{F}) [co-fixative (c-F) of class (VI), herein after], Formula (II_{G}) [co-fixative (c-F) of class (VII), herein after], or Formula (II_{H}) [co-fixative (c-F) of class (VIII), herein after] 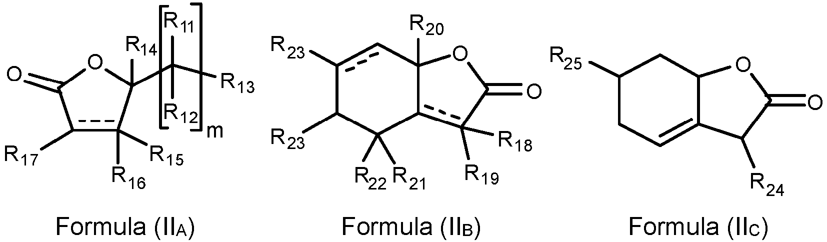 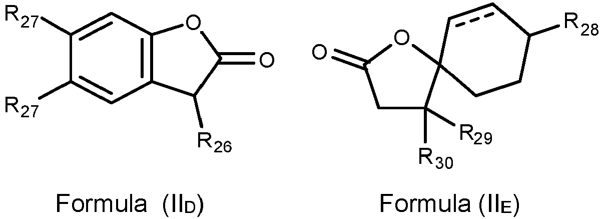 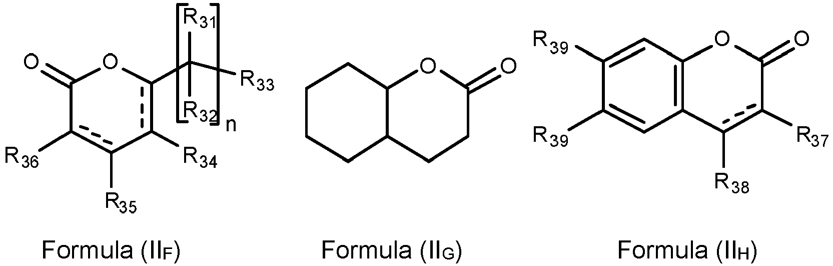 wherein the dash bond represents an optional double bound; provided that when there is a double bond in Formula (II_{A}), R₁₆ is not present; provided that when there is a double bond in the fused 5-membered lactone ring in Formula (II_{B}), R₁₉ is not present;
and wherein
- each of m, and n is an integer selected from 0, 1, 2, 3, 4, 5, 6, 7, 8, 9, 10, 11, or 12;
- each of R₁₁, R₁₂, R₃₁, and R₃₂, equal to or different from each other and at each occurrence, is independently selected from hydrogen, C₁₋₈ alkyl, C₂₋₈ alkenyl, or OH; wherein R₁₁ and R₁₂ can together form =O;
- each of R₁₃, R₁₅, R₁₆, R₁₈, R₁₉, R₂₀, R₂₁, R₂₂, R₂₄, R₂₆, R₂₇, R₂₉, R₃₀, R₃₃, R₃₅, R₃₆, R₃₇, R₃₈, and R₃₉, equal to or different from each other and at each occurrence, is independently selected from hydrogen, or methyl; wherein R₁₈ and R₁₉ can together form =CH₂ provided that there is no double bond in the fused 5-membered lactone ring of Formula (II_{B}); wherein R₃₇ and R₃₈ can together form a C₃ cycloalkyl provided that there is no double bond in the fused 6-membered lactone ring of Formula (II_{H});
- each of R₁₄, and R₃₄, equal to or different from each other and at each occurrence, is independently selected from hydrogen, C₁₋₈ alkyl, or C₂₋₈ alkenyl;
- each of R₁₇ is independently selected from hydrogen, methyl, or OH;
- each of R₂₃, and R₂₅, equal to or different from each other and at each occurrence, is independently selected from hydrogen, C₁₋₄ alkyl, or C₂₋₄ alkenyl;
- each of R₂₈ is independently selected from hydrogen, or C₁₋₄ alkyl;
c) at least 1.00 wt. % of at least one fragrance component, wherein said fragrance component is different to the co-fixative (c-F).

2. The composition (F) according to claim 1, wherein the fixative (Fix) has a Formula (I_{F1}) herein below 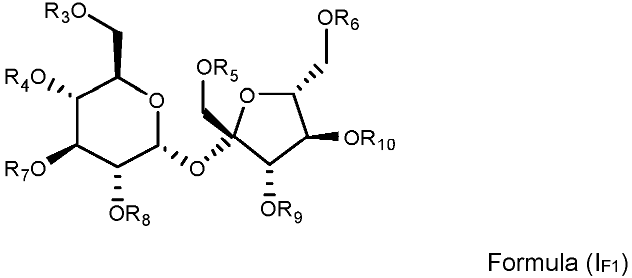 wherein
- each of R₃, R₄, R₅, R₆, R₇, R₈, R₉, and R₁₀, equal to or different from each other and at each occurrence, are COR₁, wherein each of R₁ is independently selected from methyl, or isopropyl;
with the proviso that at least one R₁ is isopropyl.

3. The composition (F) according to any one of claims 1 or 2, wherein the fixative (Fix), relative to the total weight of the composition (F), is present in an amount from 12.00 to 65.00 wt. %, or in an amount from 14.00 to 60.00 wt. %, or in an amount from 16.00 to 55.00 wt. %, or in an amount from 16.00 to 50.00 wt. %, or in an amount from 18.00 to 45.00 wt. %, or in an amount from 18.00 to 40.00 wt. %, or in an amount from 20.00 to 35.00 wt. %, or in an amount from 20.00 to 30.00 wt. %.

4. The composition (F) according to any one of claims 1 to 3, wherein the co-fixative (c-F) is selected from those of Formulae (II_{A1-1}) to (II_{A1-3}), or (II_{A2-1}), or (II_{F1-1}) to (II_{F1-2}), or (II_{F2-1}) to (II_{F2-2}), or (II_{F3-1}), or (II_{H1}) to (II_{H2}) herein below 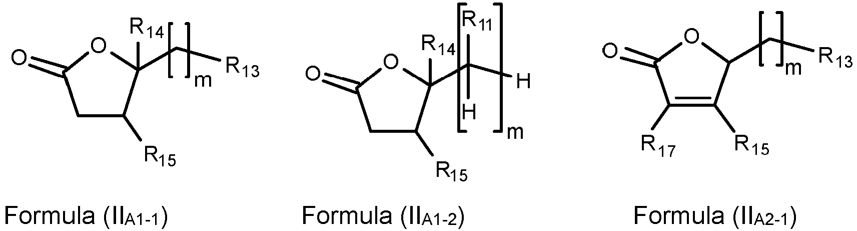 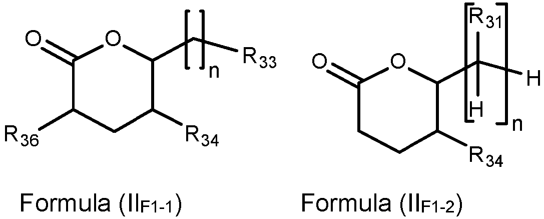 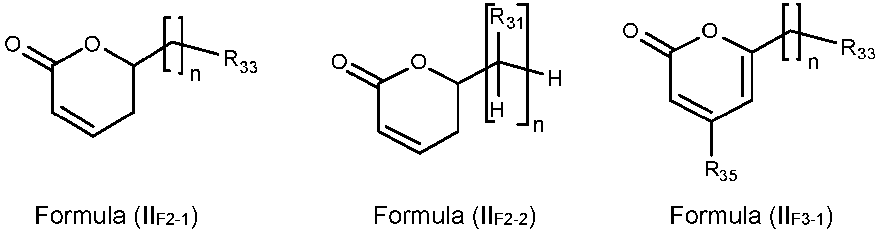 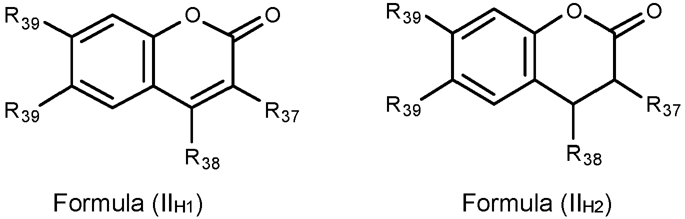 wherein
- each of m, and n is an integer selected from 0, 1, 2, 3, 4, 5, 6, 7, 8, 9, 10, 11, or 12;
- each of R₁₁, and R₃₁, equal to or different from each other and at each occurrence, is independently selected from hydrogen, or C₂₋₈ alkenyl;
- each of R₁₃, R₁₅, R₁₇, R₃₃, R₃₅, R₃₆, R₃₇, R₃₈, and R₃₉, equal to or different from each other and at each occurrence, is independently selected from hydrogen, or methyl; wherein R₃₇ and R₃₈ can together form a C₃ cycloalkyl provided that there is no double bond in the fused 6-membered lactone ring of Formula (II_{H});
- each of R₁₄, and R₃₄, equal to or different from each other and at each occurrence, is independently selected from hydrogen, or C₁₋₈ alkyl.

5. The composition (F) according to any one of claims 1 to 4, wherein the co-fixative (c-F) is selected from those of Formulae (II_{A1-1a}) to (II_{A1-1p}), or (II_{A1-2a}) to (II_{A1-2e}), or (II_{A2-1a}) to (II_{A2-1b}), or (II_{F1-1a}) to (II_{F1-1j}), or (II_{F1-2a}) to (II_{F1-2b}), or (II_{F2-1a}), or (II_{F2-2a}), or (II_{F3-1a}) to (II_{F3-1b}), or (II_{H1-1}), or (II_{H2-1}) herein below

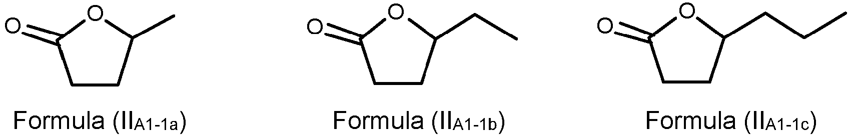

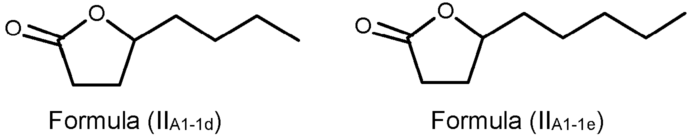

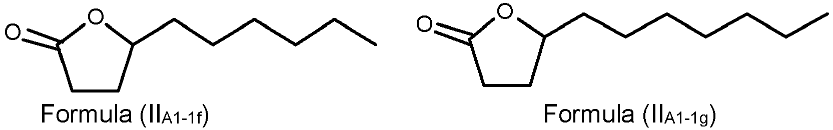

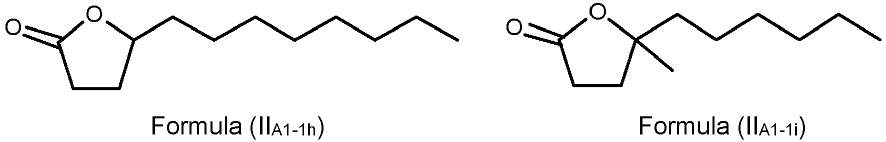

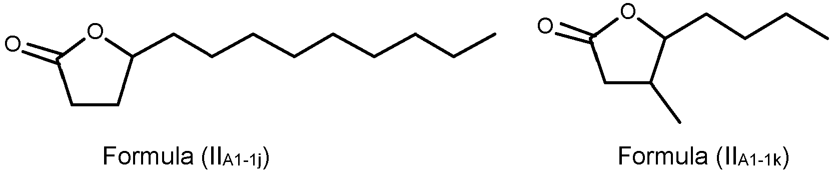

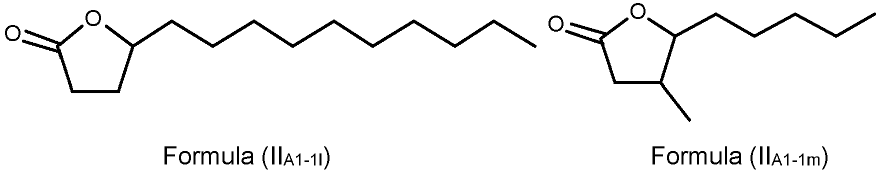

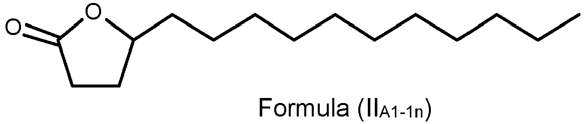

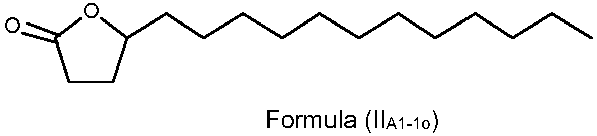

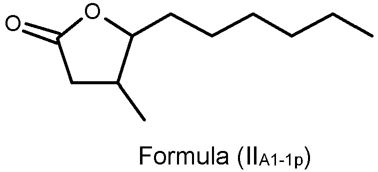

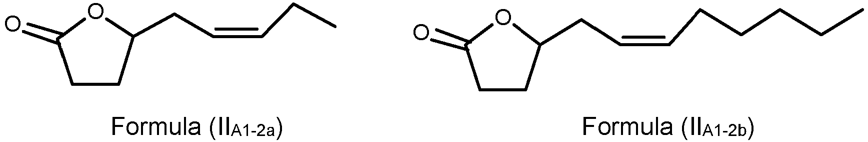

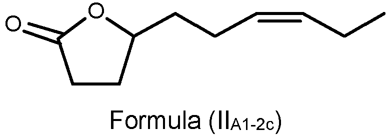

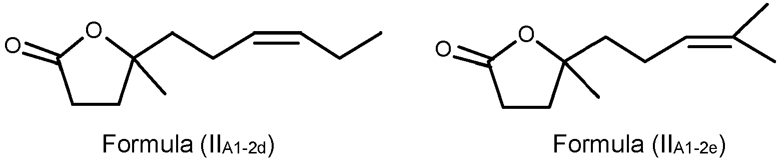

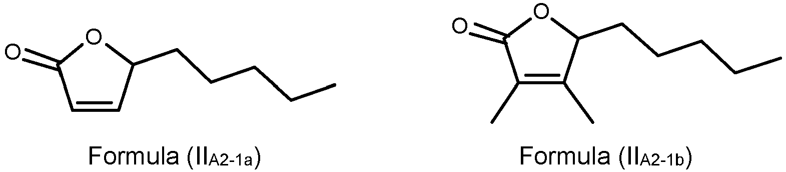

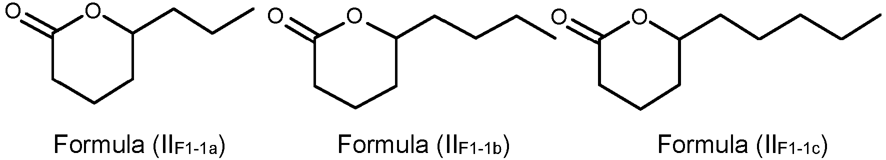

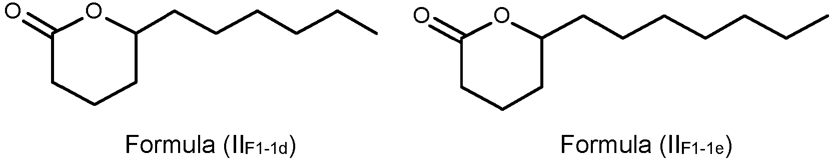

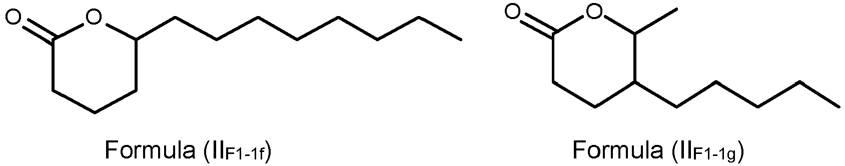

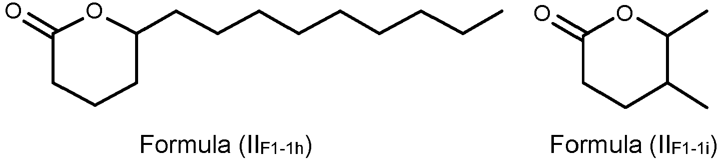

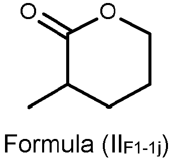

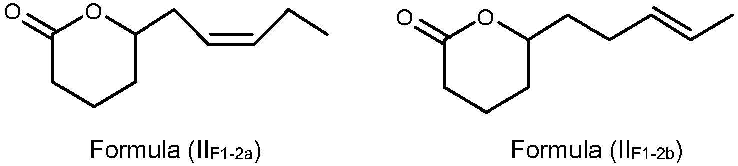

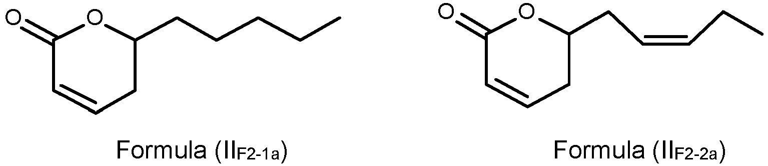

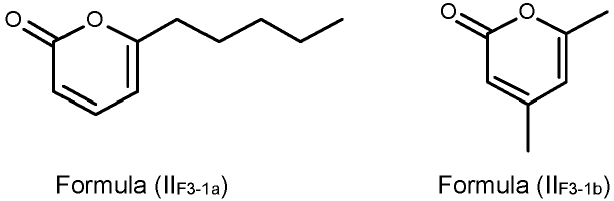

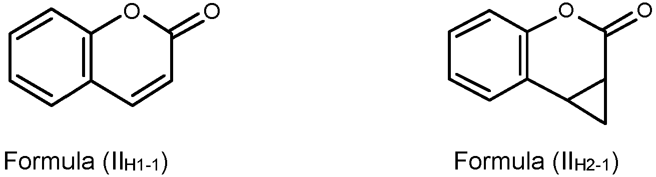

6. The composition (F) according to any one of claims 1 to 5, wherein the co-fixative (c-F) is selected from those of Formulae (II_{A1-1f}), or (II_{H1-1}) herein below

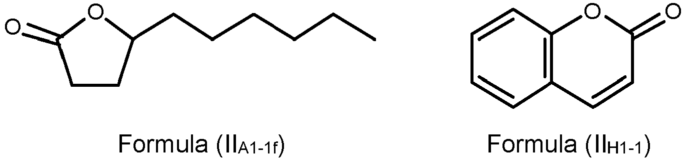

7. The composition (F) according to any one of claims 1 to 6, wherein the co-fixative (c-F), relative to the total weight of the composition (F), is present in an amount from 5.00 to 45.00 wt. %, or in an amount from 7.50 to 40.00 wt. %, or in an amount from 7.50 to 35.00 wt. %, or in an amount from 10.00 to 30.00 wt. %, or in an amount from 10.00 to 25.00 wt. %, or in an amount from 12.50 to 20.00 wt. %.

8. The composition (F) according to any one of claims 1 to 7, wherein the composition (F) comprises at least one alcohol according to general Formula (III), wherein the at least one alcohol is present in a weight ratio of the at least one alcohol to the fixative (Fix) from at least 1 to 20 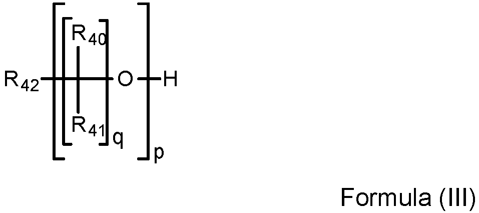 wherein
- each of p is an integer in the range from 1 to 400;
- each of q is an integer selected from 1, 2, or 3;
- each of R₄₀ is independently selected from hydrogen, methyl, ethyl, or OH;
- each of R₄₁ is independently selected from hydrogen, methyl, or ethyl; and
- each of R₄₂ is independently selected from methyl, or OH.

9. The composition (F) according to claim 8, wherein the weight ratio of the at least one alcohol according to general Formula (III) to the fixative (Fix) is from at least 1 to 17, or from at least 1 to 14, or from at least 1 to 12, or from at least 1 to 10, or from at least 1 to 7, or from at least 1 to 5.

10. The composition (F) according to any one of claims 8 to 9, wherein the at least one alcohol according to general Formula (III) is selected from ethanol, propanol, isopropanol, 1-butanol, ethylene glycol, diethylene glycol, propylene glycol, dipropylene glycol, tripropylene glycol, 1,3-butanediol, 1,2-butanediol, 2,3-butanediol, 2-methyl-1,2-propanediol, 2-methyl-1,3-propanediol, glycerol, 2-methyl-2,4-pentaandiol, polyethylene glycol monoalkyl ethers, polypropylene glycol monoalkyl ethers, or polyoxyalkylene copolymers.

11. A method for the manufacturing of the composition (F) according to any one of claims 1 to 10, wherein the method comprises intimate admixing:
- from 10.00 to 70.00 wt. %, or from 12.00 to 65.00 wt. %, or from 14.00 to 60.00 wt. %, or from 16.00 to 55.00 wt. %, or from 16.00 to 50.00 wt. %, or from 18.00 to 45.00 wt. %, or from 18.00 to 40.00 wt. %, or from 20.00 to 35.00 wt. %, or from 20.00 to 30.00 wt. % of the fixative (Fix), as defined in claims 1 to 2;
- from 2.50 to 50.00 wt. %, or from 5.00 to 45.00 wt. %, or from 7.50 to 40.00 wt. %, or from 7.50 to 35.00 wt. %, or from 10.00 to 30.00 wt. %, or from 10.00 to 25.00 wt. %, or from 12.50 to 20.00 wt. % of the co-fixative (c-F), as defined in claim 1 and claims 4 to 6;
- optionally at least one alcohol according to general Formula (III), as defined in claim 8, wherein the at least one alcohol is present in a weight ratio of the at least one alcohol to the fixative (Fix), as detailed above, from at least 1 to 20, or from at least 1 to 17, or from at least 1 to 14, or from at least 1 to 12, or from at least 1 to 10, or from at least 1 to 7, or from at least 1 to 5;
- at least 1.00 wt. %, or at least 5.00 wt. %, or at least 10.00 wt. %, or at least 12.50 wt. %, or at least 15.00 wt. % of the at least one fragrance component different to the co-fixative (c-F), as defined in claim 1 and claims 4 to 6;
- optionally water; and
wherein all wt. % are relative to the total weight of the composition (F).

12. The method according to claim 11, wherein the fixative (Fix) is first completely or nearly completely dissolved in at least part of the alcohol according to Formula (III₁) herein below 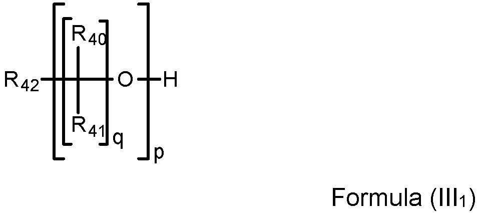 wherein
- each of p is an integer selected from 1, 2, or 3;
- each of q is an integer selected from 1, 2, or 3;
- each of R₄₀ is independently selected from hydrogen, methyl, ethyl, or OH;
- each of R₄₁ is independently selected from hydrogen, methyl, or ethyl; and
- each of R₄₂ is independently selected from methyl, or OH
prior to be mixed with the co-fixative (c-F), the at least one fragrance component different to the co-fixative (c-F), and optionally water.

13. A diluted composition (F) [composition (F_{D}), herein after] comprising the composition (F), according to any one of claims 1 to 10, and a diluent [diluent (dil), herein after].

14. The composition (F_{D}) according to claim 13, wherein the weight ratio of the composition (F) to the diluent (dil) is equal to or at least 1 to 10, or equal to or at least 1 to 100, or equal to or at least 1 to 1000, or equal to or at least 1 to 10000.

15. Personal and home care formulations comprising the composition (F), according to any one of claims 1 to 10, or the composition (F_{D}), according to any one of claims 13 to 14, in an amount from 0.01 wt. % to 100.00 wt. %, or from 0.10 wt. % to 80.00 wt. %, or from 0.20 wt. % to 60.00 wt. %, relative to the total weight of said personal and home care formulations.

16. Use of the composition (F), according to any one of claims 1 to 10, or the composition (F_{D}), according to any one of claims 13 to 14, for the manufacturing of personal and home care formulations.

## Patentansprüche

1. Eine Duftstoffabgabezusammensetzung [nachstehend Zusammensetzung (F)], welche, in Bezug auf das Gesamtgewicht der Zusammensetzung (F), Folgendes umfasst:
a) von 10,00 bis 70,00 Gewichtsprozent [nachstehend Gew.-%] von mindestens einem Duftstofffixateur [nachstehend Fixateur (Fix)], wobei der erwähnte Fixateur (Fix) folgende allgemeine Formel (I_{F}) hat: 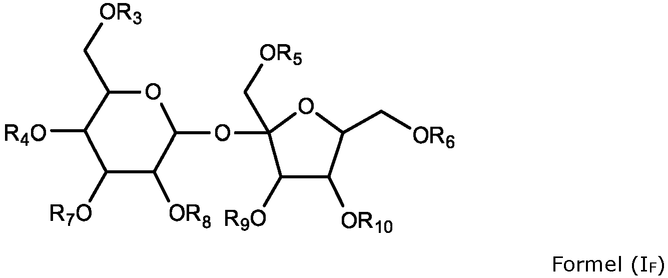 wobei
- jedes von R₃, R₄, R₅, R₆, R₇, R₈, R₉, und R₁₀, gleich wie oder unterschiedlich voneinander und bei jedem Vorkommen, COR₁ sind, wobei jedes R₁ unabhängig ausgewählt ist aus Methyl, oder Isopropyl;
mit der Bedingung, dass mindestens ein R₁ Isopropyl ist
b) von 2,50 bis 50,00 Gew.-% von mindestens einem Duftstoff-Co-Fixateur [nachstehend Co-Fixateur (c-F)], wobei der erwähnte Co-Fixateur (c-F) ein Co-Fixateur ist ausgewählt aus jenen der Formel (II_{A}) [nachstehend Co-Fixateur (c-F) der Klasse (I)], Formel (II_{B}) [nachstehend Co-Fixateur (c-F) der Klasse (II)], Formel (II_{C}) [nachstehend Co-Fixateur (c-F) der Klasse (III)], Formel (II_{D}) [nachstehend Co-Fixateur (c-F) der Klasse (IV)], Formel (II_{E}) [nachstehend Co-Fixateur (c-F) der Klasse (V)], Formel (II_{F}) [nachstehend Co-Fixateur (c-F) der Klasse (VI)], Formel (II_{G}) [nachstehend Co-Fixateur (c-F) der Klasse (VII)], oder Formel (II_{H}) [nachstehend Co-Fixateur (c-F) der Klasse (VIII)] 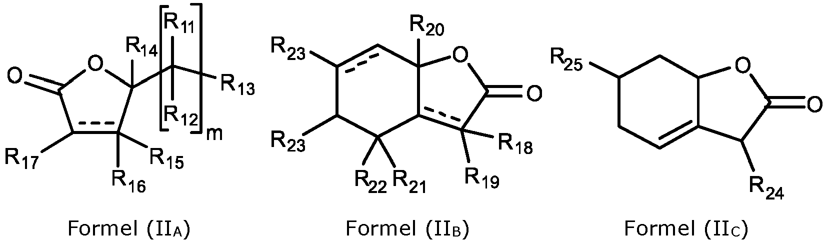 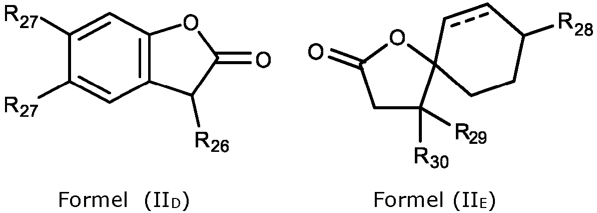 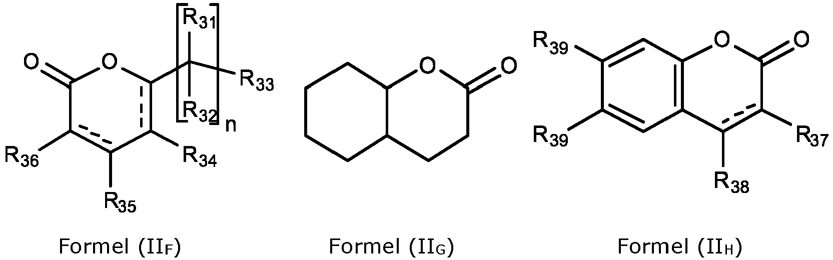 wobei die Strich-Bindung eine optionale Doppelbindung repräsentiert; unter der Bedingung, dass, wenn es eine Doppelbindung in Formel (II_{A}) gibt, R₁₆ nicht vorhanden ist; unter der Bedingung, dass, wenn es eine Doppelbindung im verschmolzenen 5-gliedrigen Lactonring in Formel (II_{B}) gibt, R₁₉ nicht vorhanden ist;
und wobei
- jedes von m, und n eine Ganzzahl ausgewählt aus 0, 1, 2, 3, 4, 5, 6, 7, 8, 9, 10, 11, oder 12 ist;
- jedes von R₁₁, R₁₂, R₃₁, und R₃₂, gleich wie oder unterschiedlich voneinander und bei jedem Vorkommen, unabhängig ausgewählt ist aus Wasserstoff, C₁₋₈-Alkyl, C₂₋₈-Alkenyl, oder OH; wobei R₁₁ und R₁₂ zusammen =O bilden können;
- jedes von R₁₃, R₁₅, R₁₆, R₁₈, R₁₉, R₂₀, R₂₁, R₂₂, R₂₄, R₂₆, R₂₇, R₂₉, R₃₀, R₃₃, R₃₅, R₃₆, R₃₇, R₃₈, und R₃₉, gleich wie oder unterschiedlich voneinander und bei jedem Vorkommen, unabhängig ausgewählt ist aus Wasserstoff, oder Methyl; wobei R₁₈ und R₁₉ unter der Bedingung zusammen =CH₂ bilden können, dass es keine Doppelbindung im verschmolzenen 5-gliedrigen Lactonring der Formel (II_{B}) gibt; wobei R₃₇ und R₃₈ unter der Bedingung zusammen ein C₃-Cycloalkyl bilden können, dass es keine Doppelbindung im verschmolzenen 6-gliedrigen Lactonring der Formel (II_{H}) gibt;
- jedes von R₁₄, und R₃₄, gleich wie oder unterschiedlich voneinander und bei jedem Vorkommen, unabhängig ausgewählt ist aus Wasserstoff, C₁₋₈-Alkyl, oder C₂₋₈-Alkenyl;
- jedes R₁₇ unabhängig ausgewählt ist aus Wasserstoff, Methyl, oder OH;
- jedes von R₂₃, und R₂₅, gleich wie oder unterschiedlich voneinander und bei jedem Vorkommen, unabhängig ausgewählt ist aus Wasserstoff, C₁₋₄-Alkyl, oder C₂₋₄-Alkenyl;
- jedes R₂₈ unabhängig ausgewählt ist aus Wasserstoff, oder C₁₋₄-Alkyl;
c)mindestens 1,00 Gew.-% von zumindest einer Duftstoffkomponente, wobei sich die erwähnte Duftstoffkomponente vom Co-Fixateur (c-F) unterscheidet.

2. Die Zusammensetzung (F) nach Anspruch 1, wobei der Fixateur (Fix) eine Formel (I_{F1}) wie hier unten hat 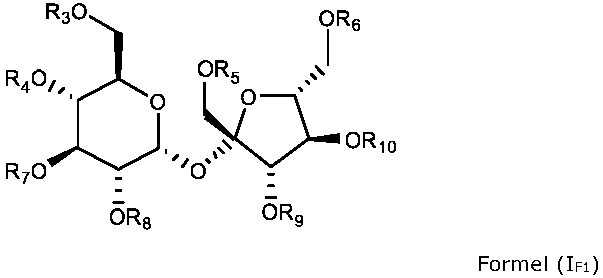 wobei
- jedes von R₃, R₄, R₅, R₆, R₇, R₈, R₉, und R₁₀, gleich wie oder unterschiedlich voneinander und bei jedem Vorkommen, COR₁ sind, wobei jedes R₁ unabhängig ausgewählt ist aus Methyl, oder Isopropyl;
mit der Bedingung, dass mindestens ein R₁ Isopropyl ist.

3. Die Zusammensetzung (F) nach irgendeinem der Ansprüche 1 oder 2, wobei der Fixateur (Fix), in Bezug zum Gesamtgewicht der Zusammensetzung (F), in einer Menge von 12,00 bis 65,00 Gew.-%, oder in einer Menge von 14,00 bis 60,00 Gew.-%, oder in einer Menge von 16,00 bis 55,00 Gew.-%, oder in einer Menge von 16,00 bis 50,00 Gew.-%, oder in einer Menge von 18,00 bis 45,00 Gew.-%, oder in einer Menge von 18,00 bis 40,00 Gew.-%, oder in einer Menge von 20,00 bis 35,00 Gew.-%, oder in einer Menge von 20,00 bis 30,00 Gew.-% anwesend ist.

4. Die Zusammensetzung (F) nach irgendeinem der Ansprüche 1 bis 3, wobei der Co-Fixateur (c-F) ausgewählt ist aus jenen der Formeln (II_{A1-1}) bis (II_{A1-3}), oder (II_{A2-1}), oder (II_{F1-1}) bis (II_{F1-2}), oder (II_{F2-1}) bis (II_{F2-2}), oder (II_{F3-1}), oder (II_{H1}) bis (II_{H2}) wie hier unten 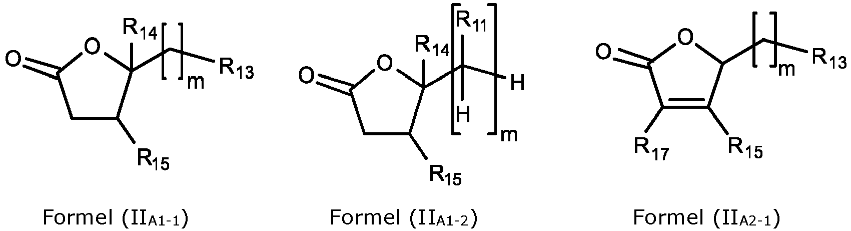 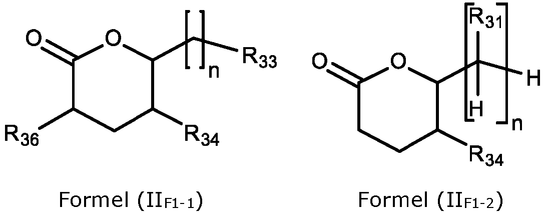 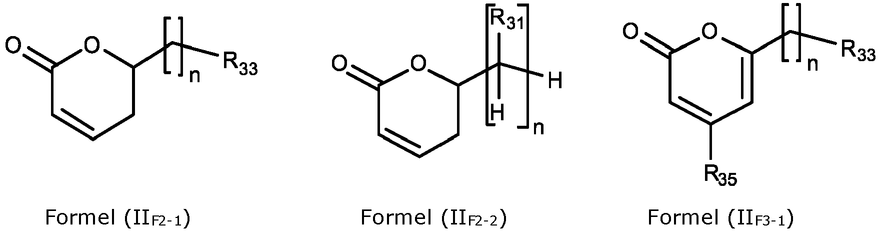 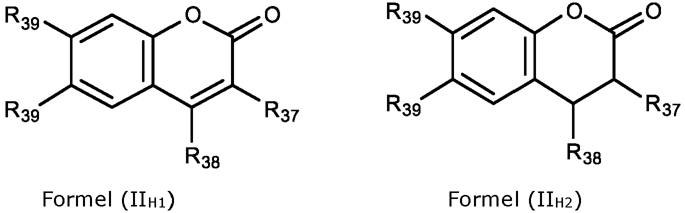 wobei
- jedes von m, und n eine Ganzzahl ausgewählt aus 0, 1, 2, 3, 4, 5, 6, 7, 8, 9, 10, 11, oder 12 ist;
- jedes von R₁₁, und R₃₁, gleich wie oder unterschiedlich voneinander und bei jedem Vorkommen, unabhängig ausgewählt ist aus Wasserstoff, oder C₂₋₈-Alkenyl;
- jedes von R₁₃, R₁₅, R₁₇, R₃₃, R₃₅, R₃₆, R₃₇, R₃₈, und R₃₉, gleich wie oder unterschiedlich voneinander und bei jedem Vorkommen, unabhängig ausgewählt ist aus Wasserstoff, oder Methyl; wobei R₃₇ und R₃₈ unter der Bedingung ein C₃-Cycloalkyl bilden können, dass es keine Doppelbindung im verschmolzenen 6-gliedrigen Lactonring der Formel (II_{H}) gibt;
- jedes von R₁₄, und R₃₄, gleich wie oder unterschiedlich voneinander und bei jedem Vorkommen, unabhängig ausgewählt ist aus Wasserstoff, oder C₁₋₈-Alkyl.

5. Die Zusammensetzung (F) nach irgendeinem der Ansprüche 1 bis 4, wobei der Co-Fixateur (c-F) ausgewählt ist aus jenen der Formeln (II_{A1-1a}) bis (II_{A1-1p}), oder (II_{A1-2a}) bis (II_{A1-2e}), oder (II_{A2-1a}) bis (II_{A2-1b}), oder (II_{F1-1a}) bis (II_{F1-1j}), oder (II_{F1-2a}) bis (II_{F1-2b}), oder (II_{F2-1a}), oder (II_{F2-2a}), oder (II_{F3-1a}) bis (II_{F3-1b}), oder (II_{H1-1}), oder (II_{H2-1}) wie hier unten

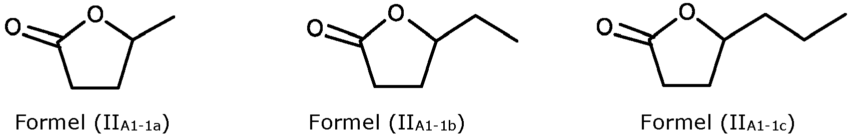

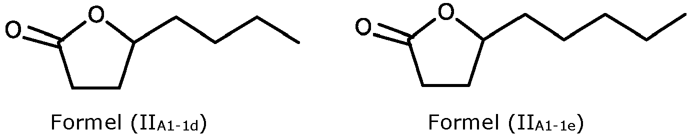

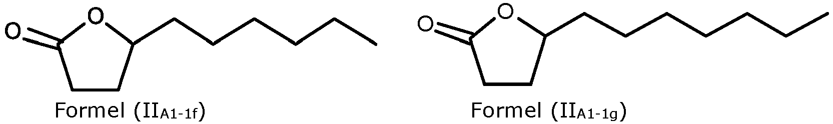

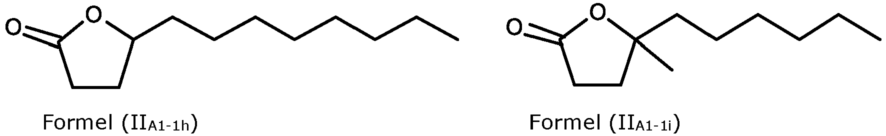

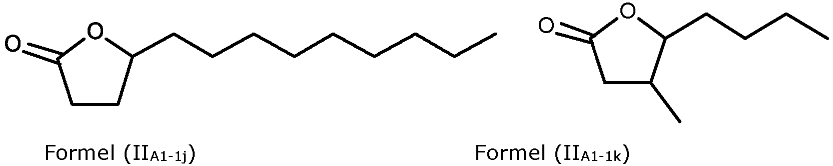

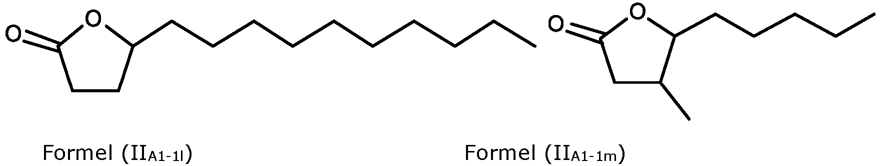

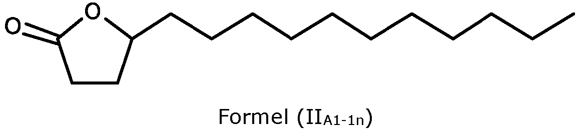

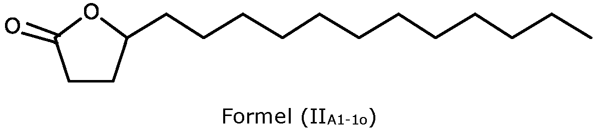

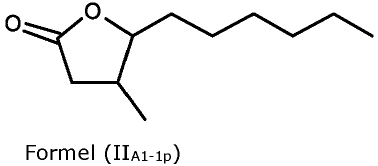

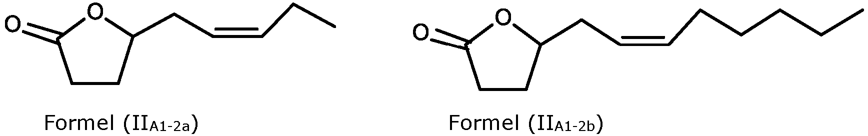

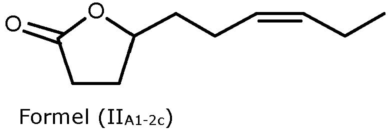

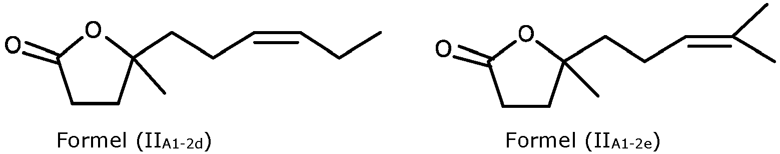

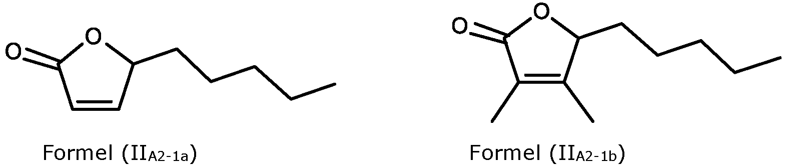

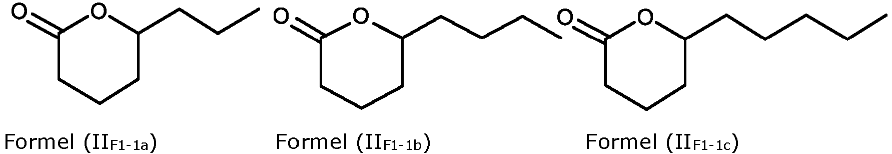

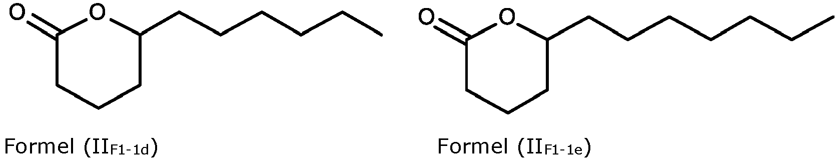

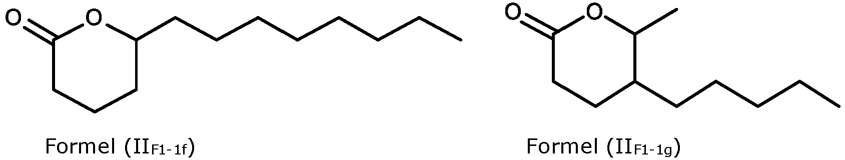

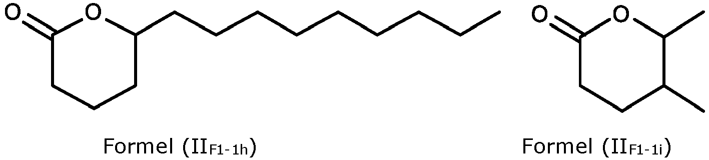

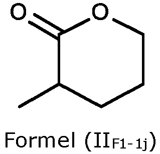

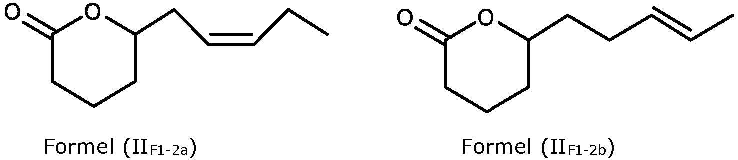

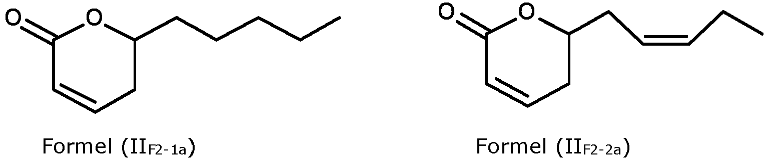

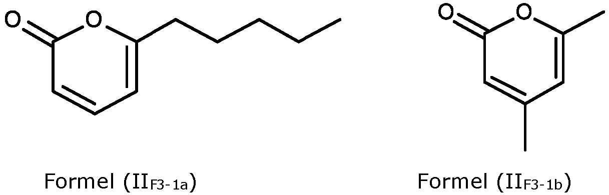

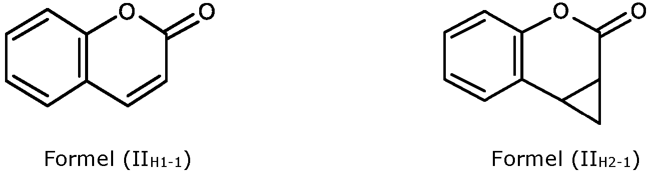

6. Die Zusammensetzung (F) nach irgendeinem der Ansprüche 1 bis 5, wobei der Co-Fixateur (c-F) ausgewählt ist aus jenen der Formeln (II_{A1-1f}), oder (II_{H1-1}) wie hier unten

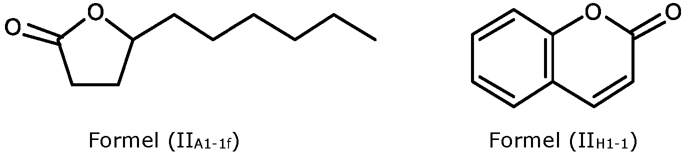

7. Die Zusammensetzung (F) nach irgendeinem der Ansprüche 1 oder 6, wobei der Co-Fixateur (c-F), in Bezug zum Gesamtgewicht der Zusammensetzung (F), in einer Menge von 5,00 bis 45,00 Gew.-%, oder in einer Menge von 7,50 bis 40,00 Gew.-%, oder in einer Menge von 7,50 bis 35,00 Gew.-%, oder in einer Menge von 10,00 bis 30,00 Gew.-%, oder in einer Menge von 10,00 bis 25,00 Gew.-%, oder in einer Menge von 12,50 bis 20,00 Gew.-% anwesend ist.

8. Die Zusammensetzung (F) nach irgendeinem der Ansprüche 1 bis 7, wobei die Zusammensetzung (F) zumindest einen Alkohol nach der allgemeinen Formel (III) umfasst, wobei der zumindest eine Alkohol in einem Gewichtsverhältnis des zumindest einen Alkohols zum Fixateur (Fix) von mindestens 1 zu 20 anwesend ist. 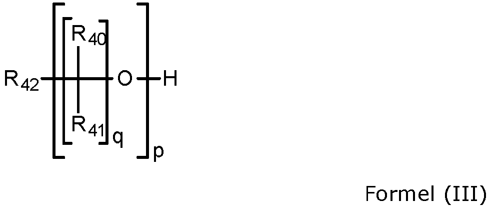 wobei
- jedes p eine Ganzzahl im Bereich von 1 bis 400 ist;
- jedes q eine Ganzzahl ausgewählt aus 1, 2 oder 3 ist;
- jedes R₄₀ unabhängig ausgewählt ist aus Wasserstoff, Methyl, Ethyl oder OH;
- jedes R₄₁ unabhängig ausgewählt ist aus Wasserstoff, Methyl, oder Ethyl; und
- jedes R₄₂ unabhängig ausgewählt ist aus Methyl, oder OH.

9. Die Zusammensetzung (F) nach Anspruch 8, wobei das Gewichtsverhältnis des zumindest einen Alkohols nach der allgemeinen Formel (III) zum Fixateur (Fix) von mindestens 1 zu 17 beträgt, oder von mindestens 1 zu 14, oder von mindestens 1 zu 12, oder von mindestens 1 zu 10, oder von mindestens 1 zu 7, oder von mindestens 1 zu 5.

10. Die Zusammensetzung (F) nach irgendeinem der Ansprüche 8 bis 9, wobei der zumindest eine Alkohol nach der allgemeinen Formel (III) ausgewählt ist aus Ethanol, Propanol, Isopropanol, 1-Butanol, Ethylenglykol, Diethylenglykol, Propylenglykol, Dipropylenglykol, Tripropylenglykol, 1,3-Butandiol, 1,2-Butandiol, 2,3-Butandiol, 2-Methyl-1,2-propandiol, 2-Methyl-1,3-propandiol, Glycerol, 2-Methyl-2,4-pentandiol, Polyethylenglykol-Monoalkylethern, Polypropylenglykol-Monoalkylethern, oder Polyoxyalkylen-Copolymeren.

11. Ein Verfahren zur Herstellung der Zusammensetzung (F), nach irgendeinem der Ansprüche 1 bis 10, wobei das Verfahren inniges Vermischen der folgenden Komponenten umfasst:
- von 10,00 bis 70,00 Gew.-%, oder von 12,00 bis 65,00 Gew.-%, oder von 14,00 bis 60,00 Gew.-%, oder von 16,00 bis 55,00 Gew.-%, oder von 16,00 bis 50,00 Gew.-%, oder von 18,00 bis 45,00 Gew.-%, oder von 18,00 bis 40,00 Gew.-%, oder von 20,00 bis 35,00 Gew.-%, oder von 20,00 bis 30,00 Gew.-% des Fixateurs (Fix), wie definiert in den Ansprüchen 1 bis 2;
- von 2,50 bis 50,00 Gew.-%, oder von 5,00 bis 45,00 Gew.-%, oder von 7,50 bis 40,00 Gew.-%, oder von 7,50 bis 35,00 Gew.-%, oder von 10,00 bis 30,00 Gew.-%, oder von 10,00 bis 25,00 Gew.-%, oder von 12,50 bis 20,00 Gew.-% des Co-Fixateurs (c-F), wie definiert in Anspruch 1 und Ansprüchen 4 bis 6;
- optional zumindest eines Alkohols nach der allgemeinen Formel (III), wie definiert in Anspruch 8, wobei der zumindest eine Alkohol in einem Gewichtsverhältnis des zumindest einen Alkohols zum Fixateur (Fix), wie oben detailliert angegeben, von mindestens 1 zu 20, oder von mindestens 1 zu 17, oder von mindestens 1 zu 14, oder von mindestens 1 zu 12, oder von mindestens 1 zu 10, oder von mindestens 1 zu 7, oder von mindestens 1 zu 5 anwesend ist;
- mindestens 1,00 Gew.-%, oder mindestens 5,00 Gew.-%, oder mindestens 10,00 Gew.-%, oder mindestens 12,50 Gew.-%, oder mindestens 15,00 Gew.-% der zumindest einen Duftstoffkomponente, die sich vom Co-Fixateur (c-F), wie definiert in Anspruch 1 und Ansprüchen 4 bis 6, unterscheidet;
- optional Wasser; und
wobei sich alle Gew.-% auf das Gesamtgewicht der Zusammensetzung (F) beziehen.

12. Das Verfahren nach Anspruch 11, wobei der Fixateur (Fix) zuerst vollständig oder nahezu vollständig aufgelöst wird in zumindest einem Teil des Alkohols nach der Formel (III₁) wie hier unten 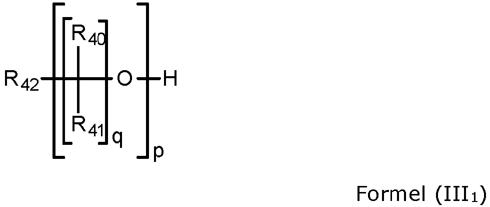 wobei
- jedes p eine Ganzzahl ausgewählt aus 1, 2 oder 3 ist;
- jedes q eine Ganzzahl ausgewählt aus 1, 2 oder 3 ist;
- jedes R₄₀ unabhängig ausgewählt ist aus Wasserstoff, Methyl, Ethyl oder OH;
- jedes R₄₁ unabhängig ausgewählt ist aus Wasserstoff, Methyl, oder Ethyl; und
- jedes R₄₂ unabhängig ausgewählt ist aus Methyl, oder OH
bevor er mit dem Co-Fixateur (c-F), der zumindest einen Duftstoffkomponente, die sich vom Co-Fixateur (c-F) unterscheidet, und optional Wasser vermischt wird.

13. Eine verdünnte Zusammensetzung (F) [nachstehend Zusammensetzung (F_{D})], welche die Zusammensetzung (F), nach irgendeinem der Ansprüche 1 bis 10, und ein Verdünnungsmittel [nachstehend Verdünnungsmittel (dil)] umfasst.

14. Die Zusammensetzung (F_{D}) nach Anspruch 13, wobei das Gewichtsverhältnis der Zusammensetzung (F) zum Verdünnungsmittel (dil) gleich wie oder mindestens 1 zu 10, oder gleich wie oder mindestens 1 zu 100, oder gleich wie oder mindestens 1 zu 1.000, oder gleich wie oder mindestens 1 zu 10.000 ist.

15. Rezepturen für die Körper- und Haushaltspflege, welche die Zusammensetzung (F), nach irgendeinem der Ansprüche 1 bis 10, oder die Zusammensetzung (F_{D}), nach irgendeinem der Ansprüche 13 bis 14, in einer Menge von 0,01 Gew.-% bis 100,00 Gew.-%, oder von 0,10 Gew.-% bis 80,00 Gew.-%, oder von 0,20 Gew.-% bis 60,00 Gew.-%, in Bezug zum Gesamtgewicht der erwähnten Rezepturen für die Körper- und Haushaltspflege umfassen.

16. Verwendung der Zusammensetzung (F), nach irgendeinem der Ansprüche 1 bis 10, oder der Zusammensetzung (F_{D}), nach irgendeinem der Ansprüche 13 bis 14, zur Herstellung von Rezepturen für die Körper- und Haushaltspflege.

## Revendications

1. Composition de diffusion de parfum [composition (F), ci-après] comprenant, par rapport au poids total de la composition (F) :
a) de 10,00 à 70,00 pourcentage en poids [% en poids, ci-après] d'au moins un fixateur de parfum [fixateur (Fix), ci-après], dans laquelle ledit fixateur (Fix) a une formule générale (I_{F}) 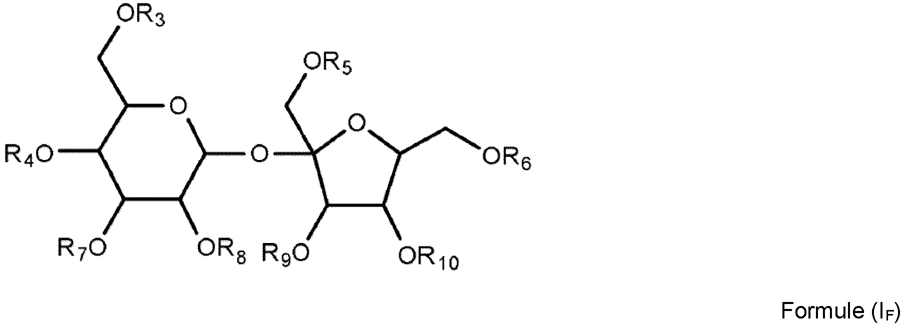 dans laquelle
- chacun des R₃, R₄, R₅, R₆, R₇, R₈, R₉, et R₁₀, égaux ou différents l'un de l'autre et à chaque occurrence, sont COR₁, dans laquelle chacun de R₁ est indépendamment choisi parmi méthyle ou isopropyle ;
à condition qu'au moins un R1 soit isopropyle
b) de 2,50 à 50,00 % en poids d'au moins un co-fixateur de parfum [co-fixateur (c-F), ci-après], dans laquelle ledit co-fixateur (c-F) est un co-fixateur choisi parmi ceux de la formule (II_{A}) [co-fixateur (c-F) de la classe (I), ci-après], de la formule (II_{B}) [co-fixateur (c-F) de la classe (II), ci-après], de la formule (II_{C}) [co-fixateur (c-F) de la classe (III), ci-après], Formule (II_{D}) [co-fixateur (c-F) de la classe (IV), ci-après], Formule (II_{E}) [co-fixateur (c-F) de la classe (V), ci-après], Formule (II_{F}) [co-fixateur (c-F) de la classe (VI), ci-après], Formule (II_{G}) [co-fixateur (c-F) de la classe (VII), ci-après], ou Formule (II_{H}) [co-fixateur (c-F) de la classe (VIII), ci-après] 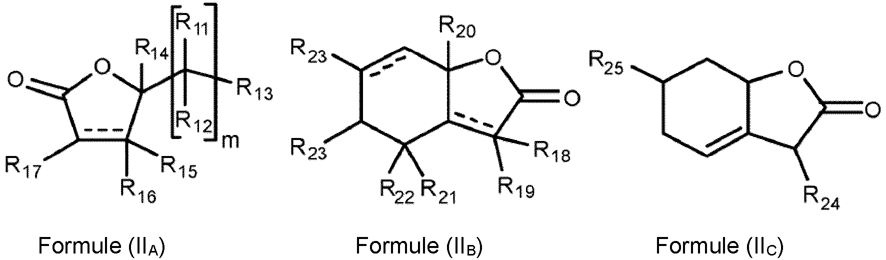 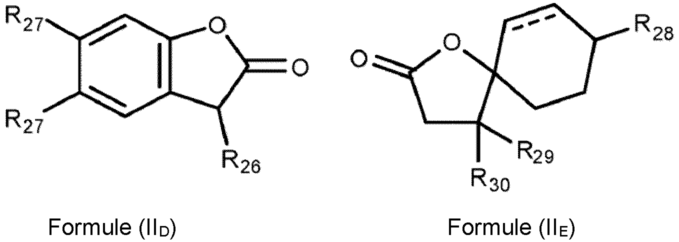 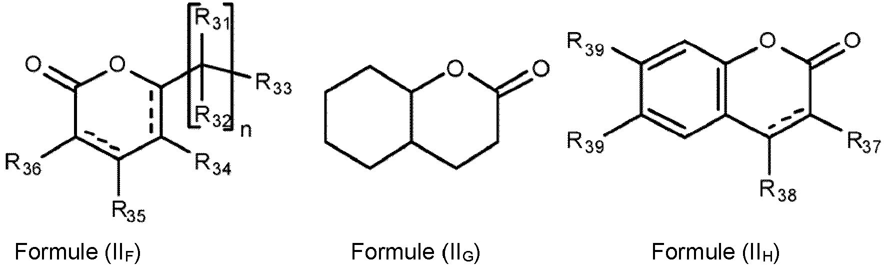
où la liaison en tirets représente une double liaison facultative ; à condition que R₁₆ ne soit pas présent en cas de double liaison dans la formule (II_{A}) ; à condition que R₁₉ ne soit pas présent en cas de double liaison dans l'anneau lactone fusionné à 5 chaînons de la formule (II_{B}) ;
et dans laquelle
- chacun de m et n est un nombre entier choisi parmi 0, 1, 2, 3, 4, 5, 6, 7, 8, 9, 10, 11 ou 12 ;
- chacun de R₁₁, R₁₂, R₃₁, et R₃₂, égal ou différent l'un de l'autre et à chaque occurrence, est indépendamment choisi parmi l'hydrogène, l'alkyle en C₁₋₈, l'alcényle en C₂₋₈ ou OH ; dans laquelle R₁₁ et R₁₂ peuvent former ensemble =O ;
- chacun de R₁₃, R₁₅, R₁₆, R₁₈, R₁₉, R₂₀, R₂₁, R₂₂, R₂₄, R₂₆, R₂₇, R₂₉, R₃₀, R₃₃, R₃₅, R₃₆, R₃₇, R₃₈, et R₃₉, égal ou différent l'un de l'autre et à chaque occurrence, est indépendamment choisi parmi l'hydrogène ou le méthyle ; dans laquelle R₁₈ et R₁₉ peuvent former ensemble =CH₂ à condition qu'il n'y ait pas de double liaison dans le cycle lactone fusionné à 5 chaînons de la formule (II_{B}) ; dans laquelle R₃₇ et R₃₈ peuvent former ensemble un cycloalkyle en C₃ à condition qu'il n'y ait pas de double liaison dans le cycle lactone fusionné à 6 chaînons de la formule (II_{H}) ;
- chacun de R₁₄ et R₃₄, égal ou différent l'un de l'autre et à chaque occurrence, est indépendamment choisi parmi l'hydrogène, l'alkyle en C₁₋₈ ou l'alcényle en C_{2-8 ;}
- chacun des R₁₇ est indépendamment choisi parmi l'hydrogène, le méthyle ou OH ;
- chacun de R₂₃ et R₂₅, égal ou différent l'un de l'autre et à chaque occurrence, est indépendamment choisi parmi l'hydrogène, l'alkyle en C₁₋₄ ou l'alcényle en C_{2-4 ;}
- chacun de R₂₈ est indépendamment choisi parmi l'hydrogène ou l'alkyle en C_{1-4 ;}
c) au moins 1,00 % en poids d'au moins un composant de parfum, où ledit composant de parfum est différent du co-fixateur (c-F).

2. Composition (F) selon la revendication 1, dans laquelle le fixateur (Fix) a la formule (I_{F1}) suivante 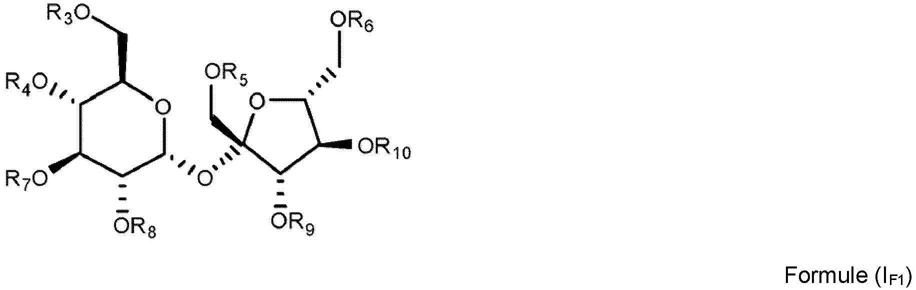 dans laquelle
- chacun des R₃, R₄, R₅, R₆, R₇, R₈, R₉, et R₁₀, égaux ou différents l'un de l'autre et à chaque occurrence, sont COR₁, dans laquelle chacun de R₁ est indépendamment choisi parmi méthyle ou isopropyle ;
à condition qu'au moins un R₁ soit un isopropyle.

3. Composition (F) selon l'une quelconque des revendications 1 ou 2, dans laquelle le fixateur (Fix), par rapport au poids total de la composition (F), est présent en une quantité allant de 12,00 à 65,00 % en poids, ou en une quantité allant de 14,00 à 60,00 % en poids, ou en une quantité allant de 16.00 à 55,00 % en poids, ou dans une quantité de 16,00 à 50,00 % en poids, ou dans une quantité de 18,00 à 45,00 % en poids, ou dans une quantité de 18,00 à 40,00 % en poids, ou dans une quantité de 20,00 à 35,00 % en poids, ou dans une quantité de 20,00 à 30,00 % en poids.

4. Composition (F) selon l'une quelconque des revendications 1 à 3, dans laquelle le co-fixateur (c-F) est choisi parmi ceux des formules (II_{A1-1}) à (II_{A1-3}), ou (II_{A2-1}), ou (II_{F1-1}) à (II_{F1-2}), ou (II_{F2-1}) à (II_{F2-2}), ou (II_{F3-1}), ou (II_{H1}) à (II_{H2}) ci-après 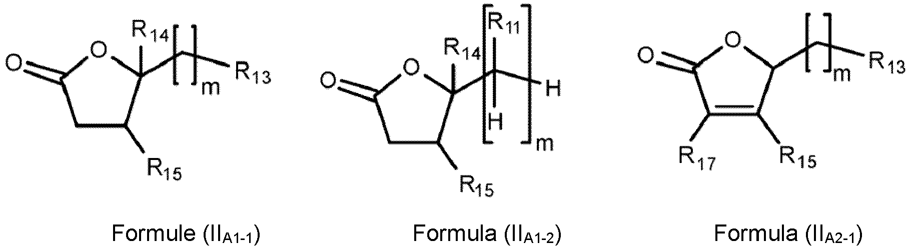 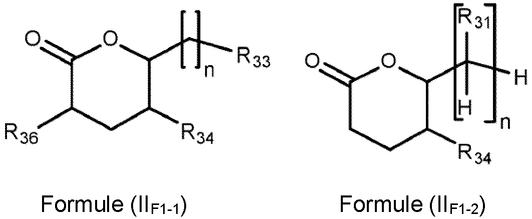 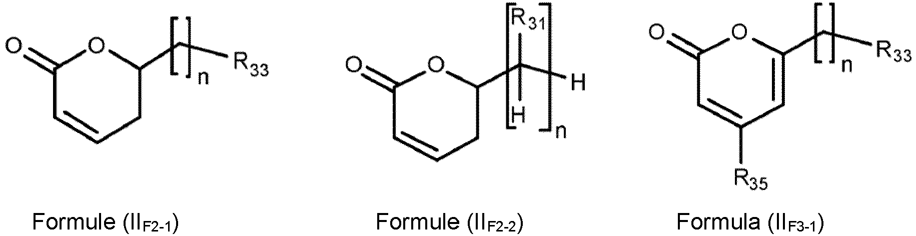 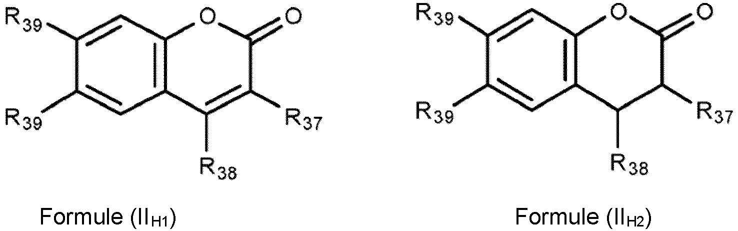 dans laquelle
- chacun de m et n est un nombre entier choisi parmi 0, 1, 2, 3, 4, 5, 6, 7, 8, 9, 10, 11 ou 12 ;
- chacun de R₁₁ et R₃₁, égal ou différent l'un de l'autre et à chaque occurrence, est indépendamment choisi parmi l'hydrogène ou l'alcényle C_{2-8 ;}
- chacun de R₁₃, R₁₅, R₁₇, R₃₃, R₃₅, R₃₆, R₃₇, R₃₈, et R₃₉, égal ou différent l'un de l'autre et à chaque occurrence, est indépendamment choisi parmi l'hydrogène ou le méthyle ; dans laquelle R₃₇ et R₃₈ peuvent former ensemble un cycloalkyle en C₃ à condition qu'il n'y ait pas de double liaison dans l'anneau lactone fusionné à 6 chaînons de la formule (II_{H}) ;
- chacun de R₁₄ et R₃₄, égal ou différent l'un de l'autre et à chaque occurrence, est indépendamment choisi parmi l'hydrogène ou l'alkyle en C₁₋₈ .

5. Composition (F) selon l'une quelconque des revendications 1 à 4, dans laquelle le co-fixateur (c-F) est choisi parmi ceux des formules (II_{A1-1a}) à (II_{A1-1p}), ou (II_{A1-2a}) à (II_{A1-2e}), ou (II_{A2-1a}) à (II_{A2-1b}), ou (II_{F1-1a}) à (II_{F1-1j}), ou (II_{F1-2a}) à (II_{F1-2b}), ou (II_{F2-1a}), ou (II_{F2-2a}), ou (II_{F3-1a}) à (II_{F3-1b}), ou (II_{H1-1}), ou (II_{H2-1}) ci-dessous

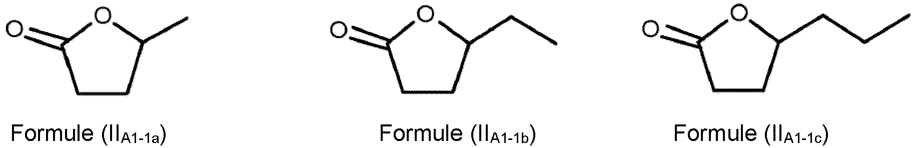

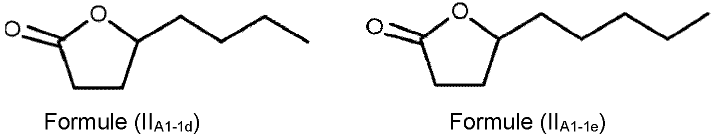

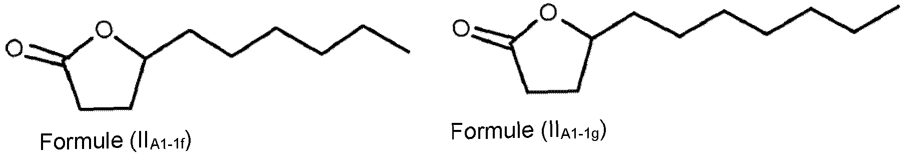

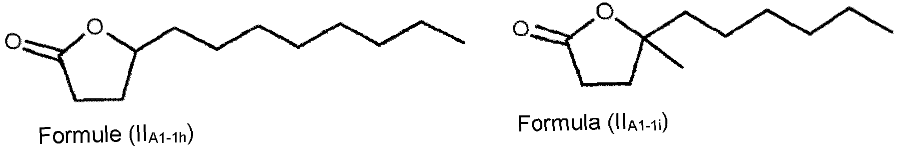

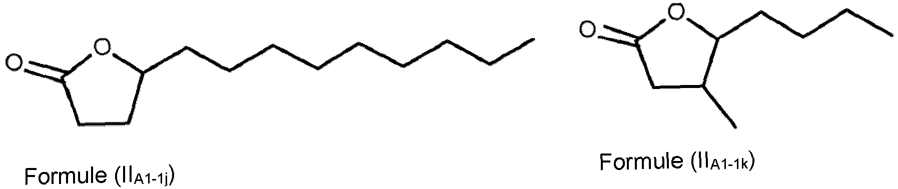

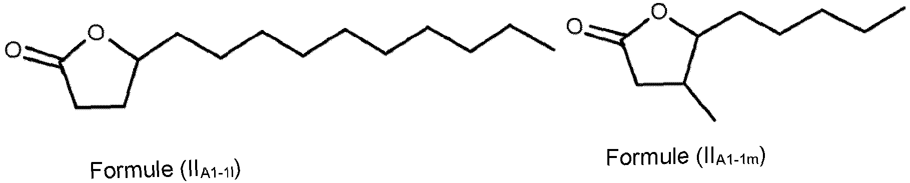

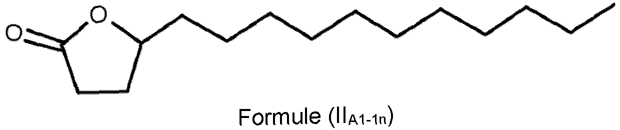

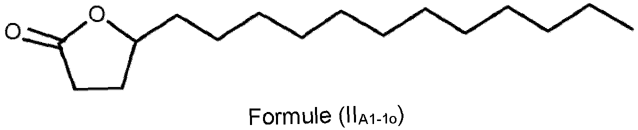

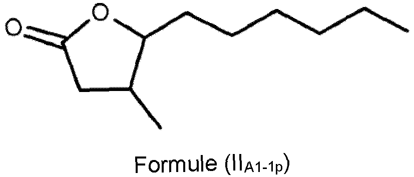

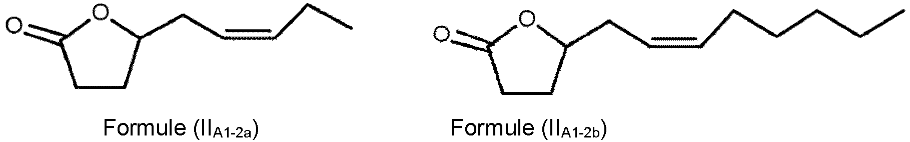

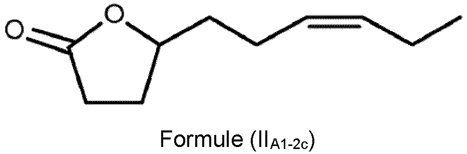

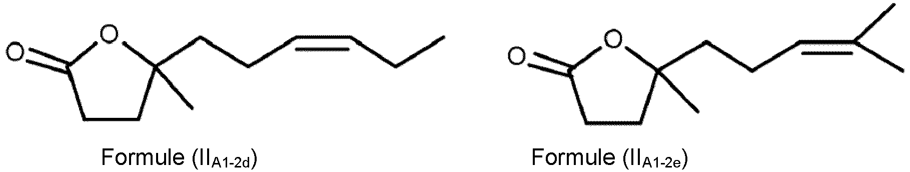

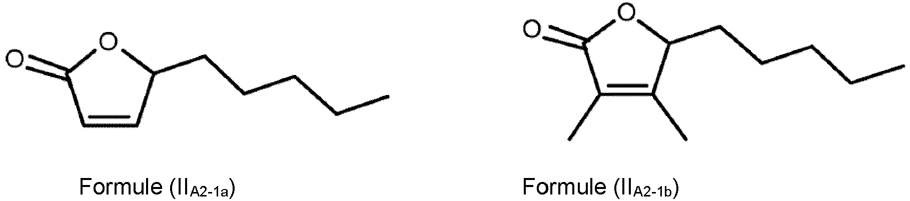

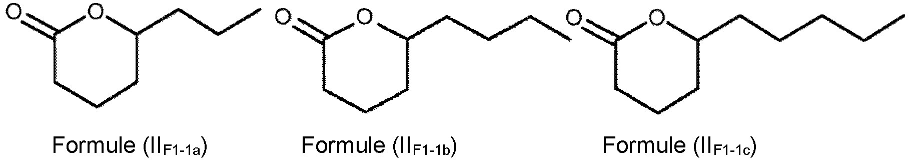

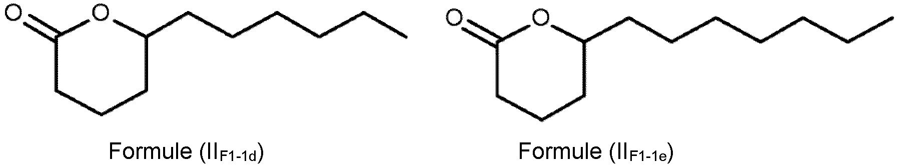

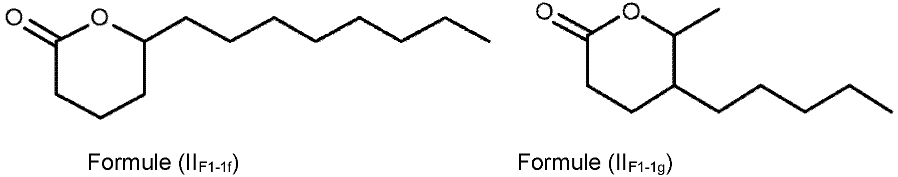

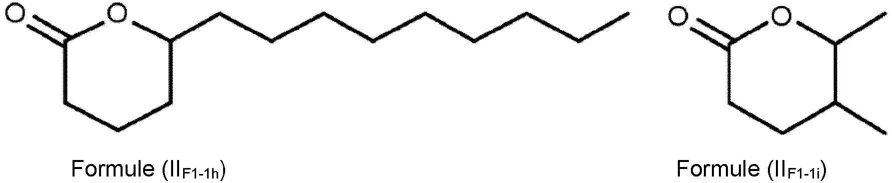

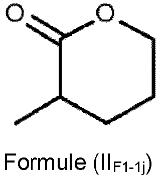

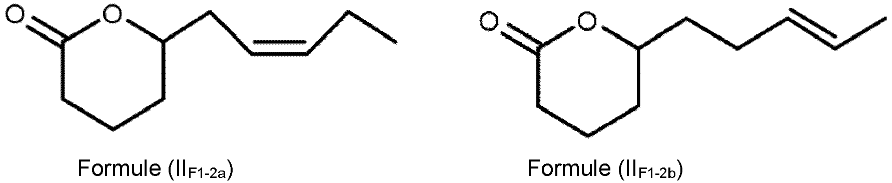

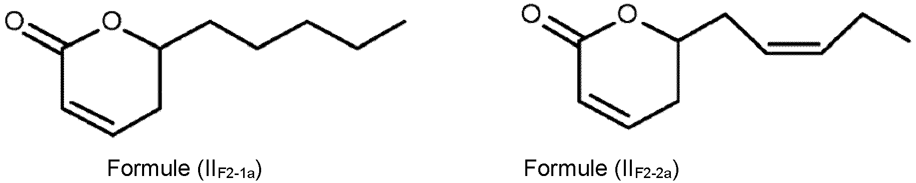

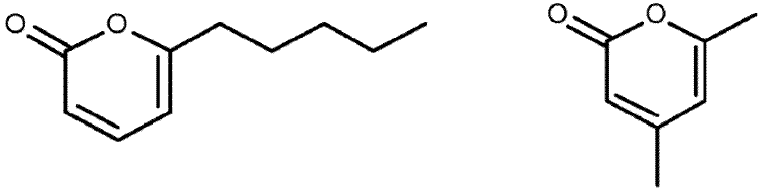

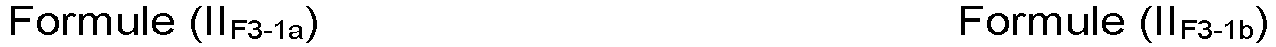

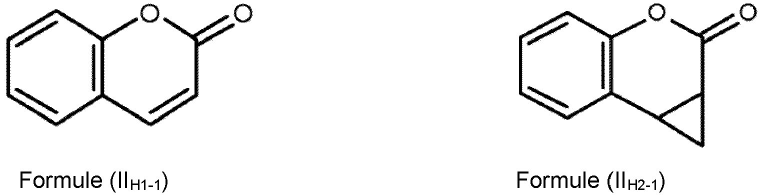

6. Composition (F) selon l'une quelconque des revendications 1 à 5, dans laquelle le co-fixateur (c-F) est choisi parmi ceux des formules (II_{A1-1f}), ou (II_{H1-1}) ci-dessous

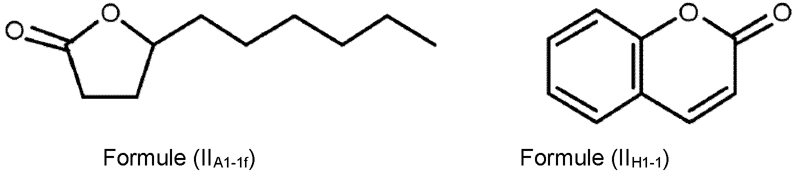

7. Composition (F) selon l'une quelconque des revendications 1 à 6, dans laquelle le co-fixateur (c-F), par rapport au poids total de la composition (F), est présent en une quantité allant de 5,00 à 45,00 wt. %, ou en une quantité de 7,50 à 40,00 % en poids, ou en une quantité de 7,50 à 35,00 % en poids, ou en une quantité de 10,00 à 30,00 % en poids, ou en une quantité de 10,00 à 25,00 % en poids, ou en une quantité de 12,50 à 20,00 % en poids.

8. Composition (F) selon l'une quelconque des revendications 1 à 7, dans laquelle la composition (F) comprend au moins un alcool selon la formule générale (III), dans laquelle ledit au moins un alcool est présent dans un rapport en poids entre ledit au moins un alcool et le fixateur (Fix) d'au moins 1 à 20 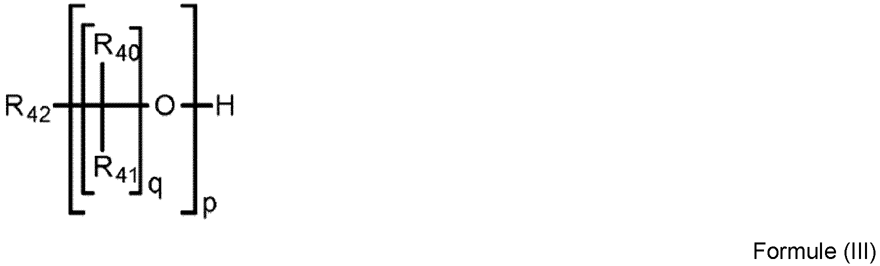 dans laquelle
- chacun de p est un nombre entier compris entre 1 et 400 ;
- chacun de q est un nombre entier choisi parmi 1, 2 ou 3 ;
- chacun des R₄₀ est indépendamment choisi parmi l'hydrogène, le méthyle, l'éthyle ou OH;
- chacun de R₄₁ est indépendamment choisi parmi l'hydrogène, le méthyle ou l'éthyle ; et
- chacun des R₄₂ est indépendamment choisi parmi les méthyles ou les OH.

9. Composition (F) selon la revendication 8, dans laquelle le rapport pondéral dudit au moins un alcool selon la formule générale (III) et le fixateur (Fix) est d'au moins 1 à 17, ou d'au moins 1 à 14, ou d'au moins 1 à 12, ou d'au moins 1 à 10, ou d'au moins 1 à 7, ou d'au moins 1 à 5.

10. Composition (F) selon l'une quelconque des revendications 8 à 9, dans laquelle ledit au moins un alcool selon la formule générale (III) est choisi parmi l'éthanol, le propanol, l'isopropanol, le 1-butanol, l'éthylène glycol, le diéthylène glycol, le propylène glycol, le dipropylène glycol, le tripropylène glycol, le 1,3-butanediol, le 1,2-butanediol, le 2,3-butanediol, le 2-méthyl-1,2-propanediol, le 2-méthyl-1,3-propanediol, le glycérol, le 2-méthyl-2,4-pentaandiol, les éthers monoalkyles de polyéthylène glycol, les éthers monoalkyles de polypropylène glycol ou les copolymères polyoxyalkylènes.

11. Procédé de fabrication de la composition (F) selon l'une quelconque des revendications 1 à 10, dans lequel le procédé comprend un mélange intime :
- de 10,00 à 70,00 % en poids, ou de 12,00 à 65,00 % en poids, ou de 14,00 à 60,00 % en poids, ou de 16,00 à 55,00 % en poids, ou de 16,00 à 50,00 % en poids, ou de 18,00 à 45,00 % en poids, ou de 18,00 à 40,00 % en poids, ou de 20,00 à 35,00 % en poids, ou de 20,00 à 30,00 % en poids du fixateur (Fix), tel que défini dans les revendications 1 à 2 ;
- de 2,50 à 50,00 % en poids, ou de 5,00 à 45,00 % en poids, ou de 7,50 à 40,00 % en poids, ou de 7,50 à 35,00 % en poids, ou de 10,00 à 30,00 % en poids, ou de 10,00 à 25,00 % en poids, ou de 12,50 à 20,00 % en poids du co-fixateur (c-F), tel que défini dans la revendication 1 et les revendications 4 à 6 ;
- éventuellement au moins un alcool selon la formule générale (III), tel que défini dans la revendication 8, dans lequel ledit au moins un alcool est présent dans un rapport pondéral entre ledit au moins un alcool et le fixateur (Fix), tel que détaillé ci-dessus, d'au moins 1 à 20, ou d'au moins 1 à 17, ou d'au moins 1 à 14, ou d'au moins 1 à 12, ou d'au moins 1 à 10, ou d'au moins 1 à 7, ou d'au moins 1 à 5 ;
- au moins 1,00 % en poids, ou au moins 5,00 % en poids, ou au moins 10,00 % en poids, ou au moins 12,50 % en poids, ou au moins 15,00 % en poids d'au moins un composant de parfum différent du co-fixateur (c-F), tel que défini dans la revendication 1 et les revendications 4 à 6 ;
- éventuellement de l'eau ; et
où tous les % en poids sont relatifs au poids total de la composition (F).

12. Procédé selon la revendication 11, dans lequel le fixateur (Fix) est d'abord complètement ou presque complètement dissous dans au moins une partie de l'alcool selon la formule (III₁) ci-dessous 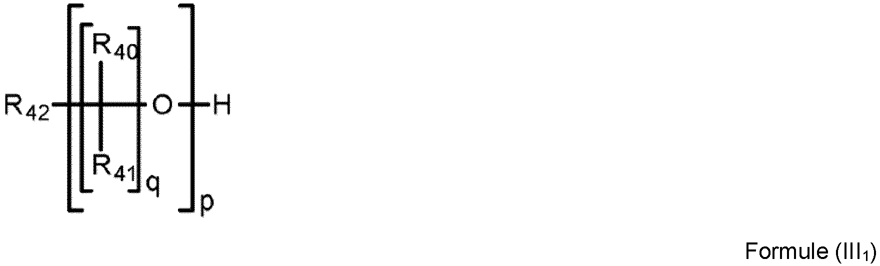 dans lequel
- chacun de p est un nombre entier choisi parmi 1, 2 ou 3 ;
- chacun de q est un nombre entier choisi parmi 1, 2 ou 3 ;
- chacun des R₄₀ est indépendamment choisi parmi l'hydrogène, le méthyle, l'éthyle ou OH;
- chacun de R₄₁ est indépendamment choisi parmi l'hydrogène, le méthyle ou l'éthyle ; et
- chacun des R₄₂ est indépendamment choisi parmi les méthyles ou les OH
avant d'être mélangés au co-fixateur (c-F), à au moins un composant de parfum différent du co-fixateur (c-F) et, éventuellement, à de l'eau.

13. Composition diluée (F) [composition (F_{D}), ci-après] comprenant la composition (F), selon l'une quelconque des revendications 1 à 10, et un diluant [diluant (dil), ci-après].

14. Composition (F_{D}) selon la revendication 13, dans laquelle le rapport pondéral entre la composition (F) et le diluant (dil) est égal ou au moins égal à 1 pour 10, ou égal ou au moins égal à 1 pour 100, ou égal ou au moins égal à 1 pour 1000, ou égal ou au moins égal à 1 pour 10000.

15. Formulations de soins personnels et domestiques comprenant la composition (F), selon l'une quelconque des revendications 1 à 10, ou la composition (F_{D}), selon l'une quelconque des revendications 13 à 14, en une quantité allant de 0,01 % en poids à 100,00 % en poids, ou de 0,10 % en poids à 80,00 % en poids, ou de 0,20 % en poids à 60,00 % en poids, par rapport au poids total desdites formulations de soins personnels et domestiques.

16. Utilisation de la composition (F), selon l'une quelconque des revendications 1 à 10, ou de la composition (F_{D}), selon l'une quelconque des revendications 13 à 14, pour la fabrication de formulations de soins personnels et domestiques.
